# EUROPEAN PATENT APPLICATION

(11) **EP 4 653 419 A1**
(43) Date of publication of application: **26.11.2025**
(21) Application number: 24744684.2
(22) Date of filing: 17.01.2024
(51) Int. Cl.: C07C 243/14, A61K 39/395, A61K 47/68, A61P 35/00, C07C 309/13, C07D 255/02, C07K 7/06, C07K 16/00

(54) **LINKER FOR ANTIBODY-DRUG CONJUGATES, AND ANTIBODY-DRUG CONJUGATE**

(30) Priority: 17.01.2023 JP 2023005221
(71) Applicant: Otsuka Pharmaceutical Co., Ltd., Tokyo 101-8535 (JP)
(72) Inventor: MAKI, Keisuke, Osaka-shi, Osaka 540-0021 (JP); MIYAJIMA, Rin, Osaka-shi, Osaka 540-0021 (JP); SHU, Zhaoma, Osaka-shi, Osaka 540-0021 (JP); OHDACHI, Kazuhiro, Osaka-shi, Osaka 540-0021 (JP); YAMASHITA, Yu, Osaka-shi, Osaka 540-0021 (JP)
(74) Representative: Hoffmann Eitle
(86) International application number: PCT/JP2024/001169
(87) International publication number: WO 2024/154763

(57) **Abstract**

The present invention provides a compound or a salt thereof for use in preparing a linker that facilitates the acquisition of an antibody-drug conjugate that has a uniform structure and that is stable in blood. Disclosed as the compound or a salt thereof is a compound represented by formula [ML-I]: 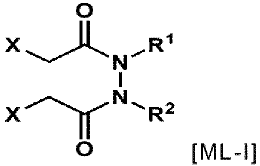 wherein X is a leaving group; R¹ and R² are each independently hydrogen, C₁₋₆ alkyl optionally substituted with one or more substituents, or C₁₋₆ alkylene-R¹¹ optionally substituted with one or more substituents, with the proviso that R¹ and R² are not hydrogen at the same time; or R¹ and R² together with nitrogen atoms adjacent thereto form a seven-membered ring that further contains a nitrogen atom as a ring-constituting atom, the ring further having, as a substituent, -C(=O)-C₁₋₆ alkylene-R¹¹ optionally substituted with one or more substituents; and R¹¹ is COOH, SO₃H, or PO₃H₂ or an ester group of COOH, SO₃H, or PO₃H₂; and a salt thereof.

## Description

### Technical Field

The present invention relates to a linker for antibody-drug conjugates, an antibody-drug conjugate, and the like.

### Background Art

Antibody-drug conjugates (ADC) are compounds in which a drug having cytotoxic or other potent pharmacological effects, such as an anticancer drug, is bonded via a linker to an antibody against an antigen that is highly expressed at a disease site. Therefore, antibody-drug conjugates can reach the target site with a high probability. When a cleavage site incorporated into the linker is cleaved by a metabolic enzyme that is present at the target site, a drug is released and exhibits activity corresponding to the drug, such as a cytotoxic effect or antiinflammatory immune response. The drug loaded onto the antibody-drug conjugate is less active while it exists as an antibody-drug conjugate. Therefore, an antibody-drug conjugate is an ideal method for efficiently delivering a drug to the site of a disease (Patent Literature (PTL) 1 to 3 and Non-patent Literature (NPL) 1 to 4) .

In many cases, a drug is bonded to a cysteine residue or lysine residue in an antibody via a linker. However, it is not easy to control this reaction. Attempts have been made to develop a method for producing an antibody-drug conjugate with a uniform structure.

One example of a method for preparing an antibody-drug conjugate is a method comprising cleaving disulfide bonds between heavy chains or between light and heavy chains to create thiol groups of cysteine, and allowing the thiol groups to bind to maleimide groups (Patent Literature (PTL) 1).

### Citation List

### Patent Literature

PTL 1: WO2017/002776
PTL 2: WO2013/132268
PTL 3: WO2018/094414

### Non-patent Literature

NPL 1: Bioconjugate Chem. 2021, 32, 1525
NPL 2: Nature Commun. 2015, 6, 6645
NPL 3: ACS Omega 2020, 5, 1557
NPL 4: Bioconjugate Chem. 2021, 32, 595

### Summary of Invention

### Technical Problem

An object of the present invention is to provide a linker for antibody-drug conjugates that facilitates the acquisition of an antibody-drug conjugate that has a uniform structure and that is stable in blood; a compound or a salt thereof for use in forming the linker; an antibody-drug conjugate using the linker; and methods for production thereof.

### Solution to Problem

The present inventors conducted extensive research to solve the above problem. As a result, the present inventors found a compound represented by the following formula or a salt thereof. The present inventors further found an antibody-drug conjugate linker using the compound or a salt thereof, an antibody-drug conjugate using the compound or a salt thereof, and methods for production thereof.

The present invention includes the following embodiments.
[1-1] A compound represented by formula [ML-I]: 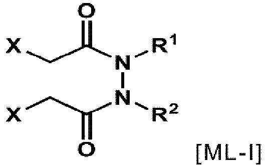 (wherein
   X is a leaving group;
   R¹ and R² are each independently hydrogen, C₁₋₆ alkyl optionally substituted with one or more substituents, or C₁₋₆ alkylene-R¹¹ optionally substituted with one or more substituents (with the proviso that R¹ and R² are not hydrogen at the same time); or R¹ and R² together with nitrogen atoms adjacent thereto form a seven-membered ring that further contains a nitrogen atom as a ring-constituting atom, the ring further having, as a substituent, -C(=O)-C₁₋₆ alkylene-R¹¹ optionally substituted with one or more substituents; and
   R¹¹ is COOH, SO₃H, PO₃H₂, or an ester group of COOH, SO₃H, or PO₃H₂); or
      a salt thereof.
[1-2] The compound or a salt thereof according to [1-1], wherein
   X is halogen, alkanesulfonyloxy, or arylsulfonyloxy;
   R¹ and R² are each independently hydrogen, C₁₋₆ alkyl, or C₁₋₆ alkylene-R¹¹ (with the proviso that R¹ and R² are not hydrogen at the same time); or
   R¹ and R² together with nitrogen atoms adjacent thereto form a seven-membered ring that further contains a nitrogen atom as a ring-constituting atom, the ring further having -C(=O)-C₁₋₆ alkylene-R¹¹ as a substituent.
[1-3] The compound or a salt thereof according to [1-1] or [1-2], which is
   a compound represented by formula [ML-Ia] or formula [ML-Ib]: 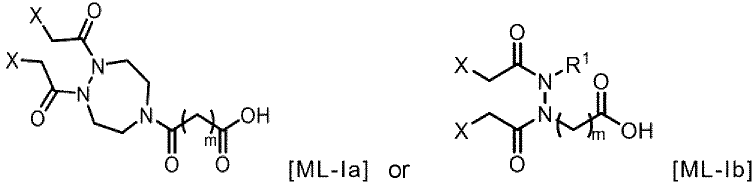 (wherein m is an integer of 1 to 6, and
      X and R¹ are as defined above), or
   a salt thereof.
[1-4] The compound or a salt thereof according to any one of [1-1] to [1-3], which is selected from the following compounds or salts thereof: 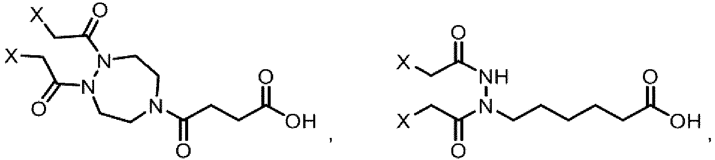 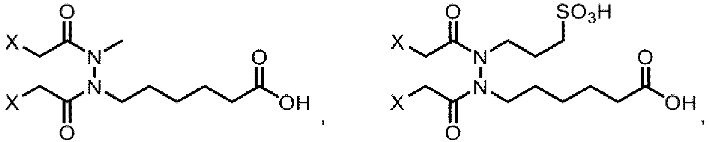 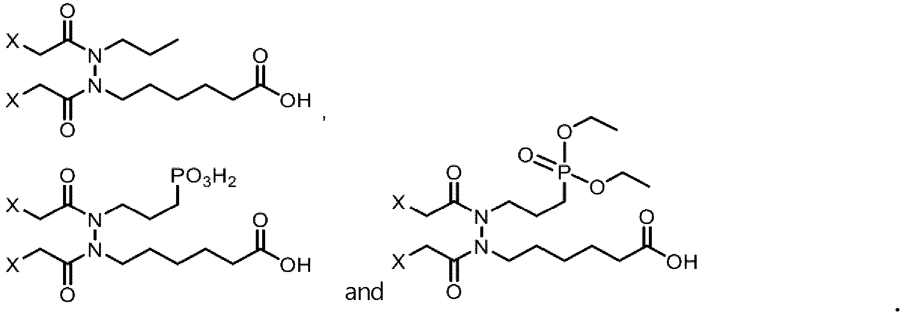
[1-5] The compound or a salt thereof according to any one of [1-1] to [1-4], wherein X is halogen.
[1-6] The compound or a salt thereof according to any one of [1-1] to [1-4], wherein X is chlorine, bromine, or iodine.
[2-1] A drug-linker intermediate, which is a compound or a salt thereof, the compound being represented by formula [MCL-Iα] or formula [MCL-IIα]: 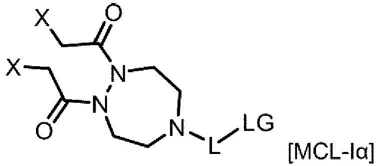 or 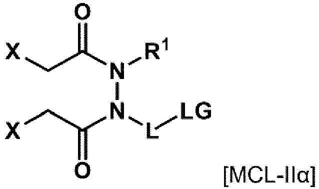 (wherein
   X is a leaving group;
   R¹ is hydrogen, C₁₋₆ alkyl optionally substituted with one or more substituents, or C₁₋₆ alkylene-R¹¹ optionally substituted with one or more substituents;
   R¹¹ is SO₃H, PO₃H₂, COOH, or an ester group of SO₃H, PO₃H₂, or COOH; L is a linker comprising L¹, L², and L³, wherein
   L¹ is 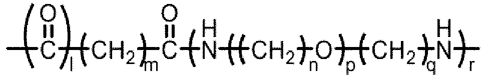 wherein each methylene is optionally substituted with one or more substituents (e.g., polyethylene glycol, saccharides (e.g., oligosaccharide), crown ether, polysarcosine, sulfonic acid-containing groups, phosphonic acid-containing groups, or amine- or ammonium salt-containing groups;
   L² is a bond or a cleavable linker; and
   L³ is a bond, amino(aryl or heteroaryl)methyloxycarbonyl represented by 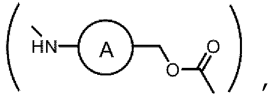
   amino(aryl or heteroaryl)methyl represented by 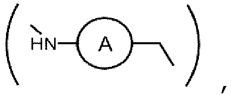
   aminomethyl represented by 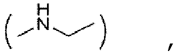
   or oxymethyl represented by 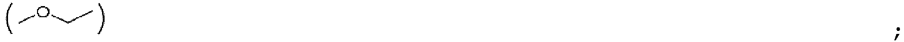 and
   A is aryl or heteroaryl each optionally substituted with one or more substituents;
   with the proviso that L² and L³ are not a bond at the same time; LG is a halogen or an O-electron-withdrawing group;
   l and r are each independently 0 or 1; and
   m, n, p, and q are each independently an integer of 1 to 6).
[2-2] The drug-linker intermediate according to [2-1], wherein L is L¹-L²-L³.
[2-3] The drug-linker intermediate according to [2-1] or [2-2], wherein
   L¹ is 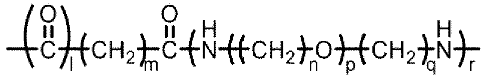 wherein each methylene is optionally substituted with polyethylene glycol or a saccharide (e.g., oligosaccharide); L² is a bond or a cleavable linker; and
   L³ is a bond, p-aminobenzyloxycarbonyl represented by 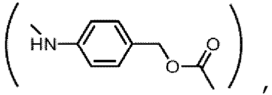
   p-aminobenzyl represented by 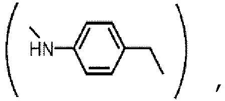
   or aminomethyl represented by 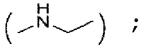 wherein the benzene rings in the p-aminobenzyloxycarbonyl and the p-aminobenzyl are each independently optionally substituted with one or more substituents.
[2-4] The drug-linker intermediate according to any one of [2-1] to [2-3], which is a compound or a salt thereof, the compound being represented by formula [MCL-I] or formula [MCL-II]: 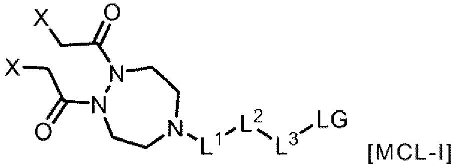 or 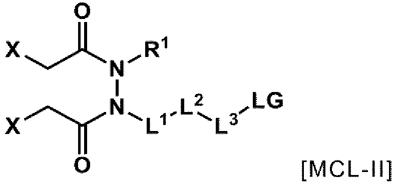 (wherein X is halogen, alkanesulfonyloxy, or arylsulfonyloxy;
   R¹ is hydrogen, C₁₋₆ alkyl, or C₁₋₆ alkylene-R¹¹;
   R¹¹ is SO₃H, PO₃H₂, COOH, or an ester group of SO₃H, PO₃H₂, or COOH; L¹ is 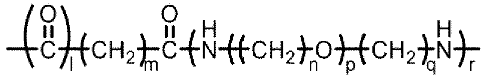 wherein each methylene is optionally substituted with polyethylene glycol or a saccharide (e.g., oligosaccharide); L² is a bond or a cleavable linker; and
   L³ is a bond, p-aminobenzyloxycarbonyl represented by 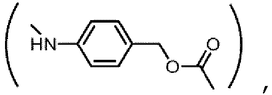
   p-aminobenzyl represented by 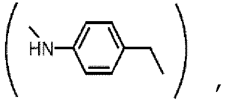
   or aminomethyl represented by 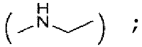 wherein the benzene rings in the p-aminobenzyloxycarbonyl and the p-aminobenzyl are each independently optionally substituted with one or more substituents;
   with the proviso that L² and L³ are not a bond at the same time; LG is a halogen or an O-electron-withdrawing group;
   l and r are each independently 0 or 1; and
   m, n, p, and q are each independently an integer of 1 to 6).
[2-5] The drug-linker intermediate according to any one of [2-1] to [2-4], wherein the cleavable linker is a cathepsin-cleavable linker, a pyrophosphatase/phosphoesterase-cleavable linker, a glycosidase-cleavable linker (β-glucuronidase-cleavable linker), a carboxylesterase-cleavable linker, a legumain-cleavable linker, a neutrophil elastase-cleavable linker, a matrix metalloproteinase-cleavable linker, or a fibroblast activation protein-cleavable linker.
[2-6] The drug-linker intermediate according to any one of [2-1] to [2-4], wherein the cleavable linker is a hydroxymethylbenzoic acid residue substituted with glucuronic acid, a peptide residue, a pyrophosphate (diphosphate) residue, or disulfide.
[2-7] The drug-linker intermediate according to [2-6], wherein the hydroxymethylbenzoic acid substituted with glucuronic acid is 2-(1-D-glucuronyl)-5-hydroxymethylbenzoic acid.
[2-8] The drug-linker intermediate according to [2-6], wherein the peptide is a dipeptide consisting of valine and citrulline, a dipeptide consisting of two alanines, a tripeptide consisting of glutamic acid, D-alanine, and alanine, a tripeptide consisting of glutamic acid, D-alanine, and alanine with polyethylene glycol or oligosaccharide added to the terminal carboxylic acid of the glutamic acid, or a tetrapeptide consisting of phenylalanine and three glycines.
[2-9] The drug-linker intermediate according to any one of [2-1] to [2-8], wherein X is halogen.
[2-10] The drug-linker intermediate according to any one of [2-1] to [2-9], wherein X is chlorine, bromine, or iodine.
[2-11] The drug-linker intermediate according to any one of [2-1] to [2-10], wherein L¹ is 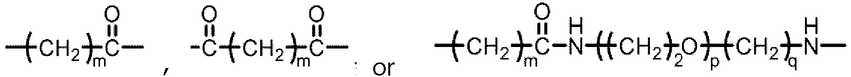 (wherein m, p, and q are as defined above).
[2-12] The drug-linker intermediate according to any one of [2-1] to [2-11], wherein L¹ is 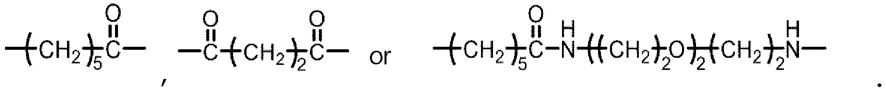
[2-13] The drug-linker intermediate according to any one of [2-1] to [2-12], wherein L is L¹-L²-L³, the L¹-L²-L³ being 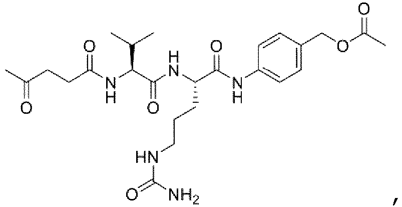 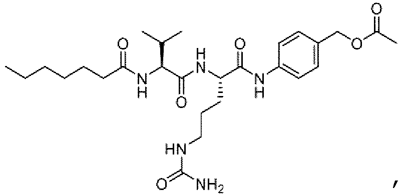 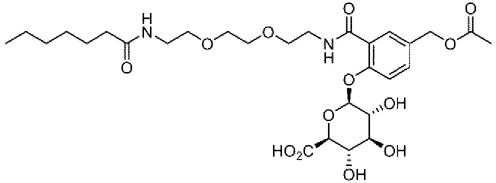 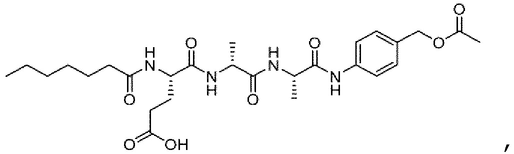 or 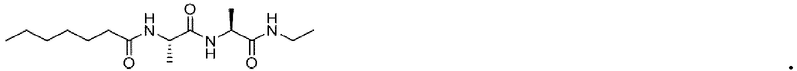
[2-14] The drug-linker intermediate according to any one of [2-1] to [2-13], which is a compound or a salt thereof, the compound being represented by formula [MCL-Ia], formula [MCL-IIa], formula [MCL-IIb], formula [MCL-IIc], or formula [MCL-IId] 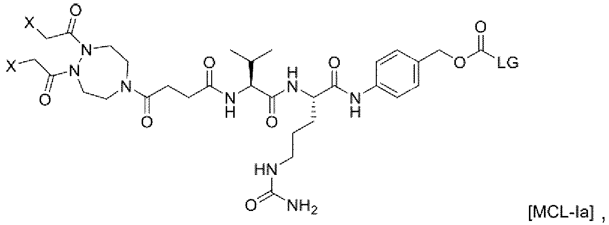 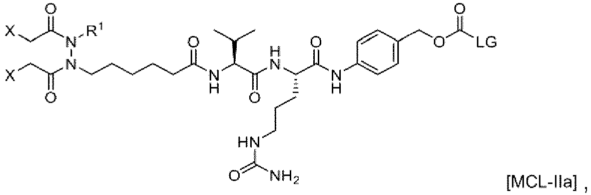 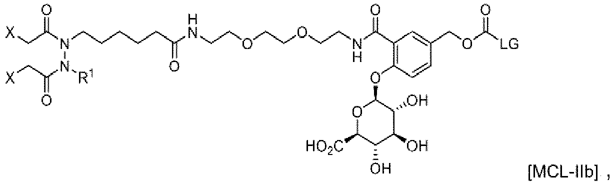 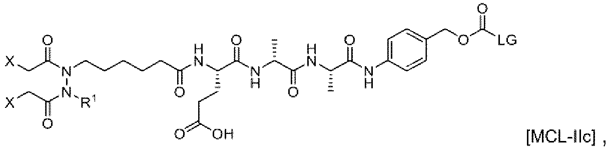 or 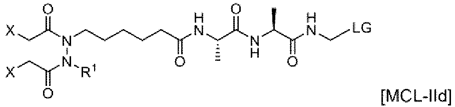 (wherein X, R¹, LG are as defined above).
[2-15] The drug-linker intermediate according to any one of [2-1] to [2-14], wherein the O-electron-withdrawing group is acyloxy optionally substituted with one or more substituents or aryloxy optionally substituted with one or more substituents.
[2-16] The drug-linker intermediate according to [2-15], wherein the acyloxy optionally substituted with one or more substituents is acetoxy.
[2-17] The drug-linker intermediate according to [2-15], wherein the aryloxy optionally substituted with one or more substituent is p-nitrophenyloxy.
[3-1] A drug-linker, which is a compound or a salt thereof,
   the compound being represented by formula [ADL-Iα] or formula [ADL-IIα]: 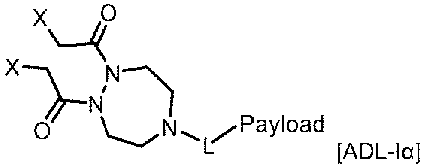 or 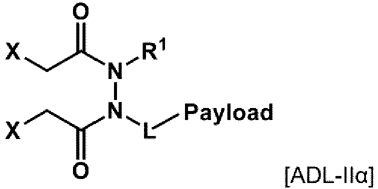 (wherein Payload is a drug residue;
   X is a leaving group;
   R¹ is hydrogen, C₁₋₆ alkyl optionally substituted with one or more substituents, or C₁₋₆ alkylene-R¹¹ optionally substituted with one or more substituents;
   R¹¹ is SO₃H, PO₃H₂, COOH, or an ester group of SO₃H, PO₃H₂, or COOH;
   L is a linker comprising L¹, L², and L³;
   L¹ is 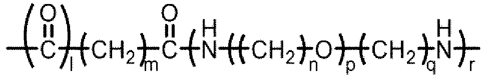 wherein each methylene is optionally substituted with one or more substituents (e.g., polyethylene glycol, saccharides (e.g., oligosaccharide), crown ether, polysarcosine, sulfonic acid-containing groups, phosphonic acid-containing groups, or amine- or ammonium salt-containing groups);
   L² is a bond or a cleavable linker;
   L³ is a bond, amino(aryl or heteroaryl)methyloxycarbonyl represented by 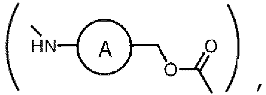
   amino(aryl or heteroaryl)methyl represented by 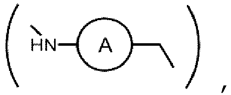
   aminomethyl represented by 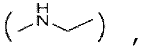
   or oxymethyl represented by 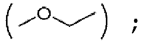 and
   A is an aryl or heteroaryl optionally substituted with one or more substituents;
   with the proviso that L² and L³ are not a bond at the same time; l and r are each independently 0 or 1; and
   m, n, p, and q are each independently an integer of 1 to 6).
[3-2] The drug-linker according to [3-1], wherein L is L¹-L²-L³.
[3-3] The drug-linker according to [3-1], wherein L¹ is 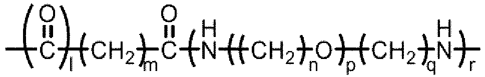 wherein each methylene is optionally substituted with polyethylene glycol or a saccharide (e.g., oligosaccharide);
   L² is a bond or a cleavable linker;
   L³ is a bond, p-aminobenzyloxycarbonyl represented by 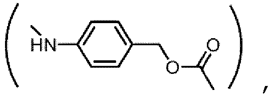
   p-aminobenzyl represented by 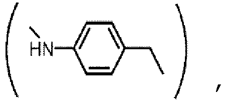
   or aminomethyl represented by 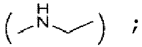 wherein the benzene rings in the p-aminobenzyloxycarbonyl and the p-aminobenzyl are each independently optionally substituted with one or more substituents.
[3-4] The drug-linker according to any one of [3-1] to [3-3], which is a compound or a salt thereof, the compound being represented by formula [ADL-I] or formula [ADL-II]: 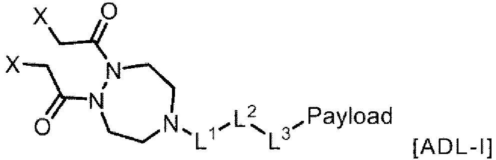 or 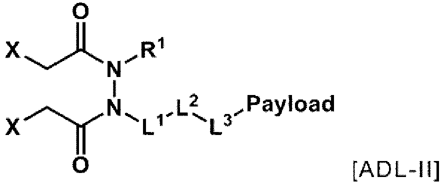 (wherein Payload is a drug residue;
   X is halogen, alkanesulfonyloxy, or arylsulfonyloxy;
   R¹ is hydrogen, C₁₋₆ alkyl, or C₁₋₆ alkylene-R¹¹;
   R¹¹ is SO₃H, PO₃H₂, COOH, or an ester group of SO₃H, PO₃H₂, or COOH;
   L¹ is 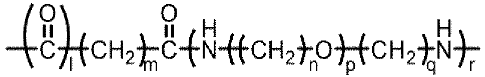 wherein each methylene is optionally substituted with polyethylene glycol or a saccharide (e.g., oligosaccharide); L² is a bond or a cleavable linker;
   L³ is a bond, p-aminobenzyloxycarbonyl represented by formula 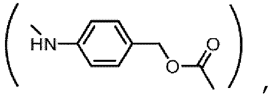
   p-aminobenzyl represented by formula 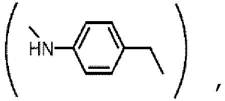
   or aminomethyl represented by 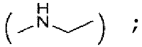 wherein the benzene rings in the p-aminobenzyloxycarbonyl and the p-aminobenzyl are each independently optionally substituted with one or more substituents;
   with the proviso that L² and L³ are not a bond at the same time;
   l and r are each independently 0 or 1; and
   m, n, p, and q are each independently an integer of 1 to 6).
[3-5] The drug-linker according to any one of [3-1] to [3-4], wherein the cleavable linker is a cathepsin-cleavable linker, a pyrophosphatase/phosphoesterase-cleavable linker, a glycosidase-cleavable linker (β-glucuronidase-cleavable linker), a carboxylesterase-cleavable linker, a legumain-cleavable linker, a neutrophil elastase-cleavable linker, a matrix metalloproteinase-cleavable linker, or a fibroblast activation protein-cleavable linker.
[3-6] The drug-linker according to any one of [3-1] to [3-4], wherein the cleavable linker is a hydroxymethylbenzoic acid residue substituted with glucuronic acid, a peptide residue, a pyrophosphate (diphosphate) residue, or disulfide.
[3-7] The drug-linker according to [3-6], wherein the hydroxymethylbenzoic acid substituted with glucuronic acid is 2-(1-D-glucuronyl)-5-hydroxymethylbenzoic acid.
[3-8] The drug-linker according to [3-6], wherein the peptide is a dipeptide consisting of valine and citrulline, a dipeptide consisting of two alanines, a tripeptide consisting of glutamic acid, D-alanine, and alanine, a tripeptide consisting of glutamic acid, D-alanine, and alanine with polyethylene glycol or oligosaccharide added to the terminal carboxylic acid of the glutamic acid, or a tetrapeptide consisting of phenylalanine and three glycines.
[3-9] The drug-linker according to any one of [3-1] to [3-8], wherein X is halogen.
[3-10] The drug-linker according to any one of [3-1] to [3-9], wherein X is chlorine, bromine, or iodine.
[3-11] The drug-linker according to any one of [3-1] to [3-10], wherein L¹ is 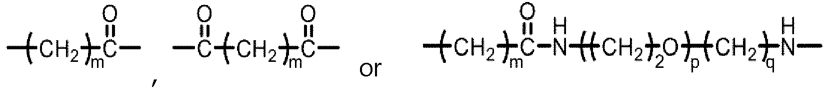 wherein m, p, and q are as defined above.
[3-12] The drug-linker according to any one of [3-1] to [3-11], wherein L¹ is 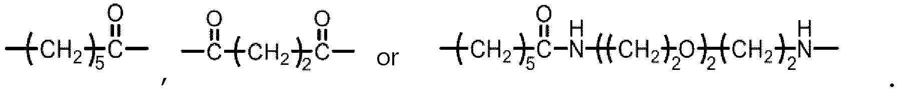
[3-13] The drug-linker according to any one of [3-1] to [3-12], which is a compound or a salt thereof, the compound being represented by formula [ADL-Ia], formula [ADL-IIa], formula [ADL-IIb], formula [ADL-IIc], or formula [ADL-IIf]: 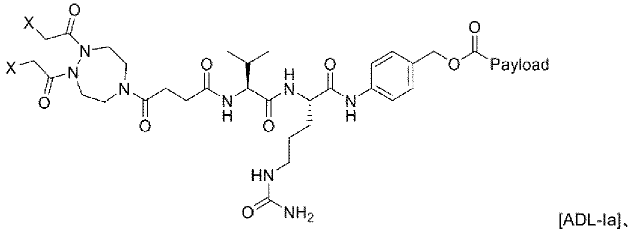 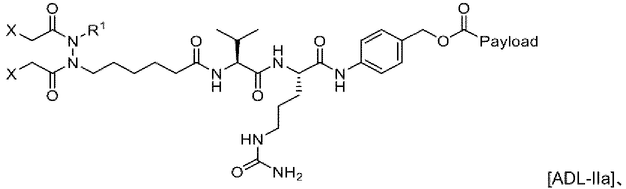 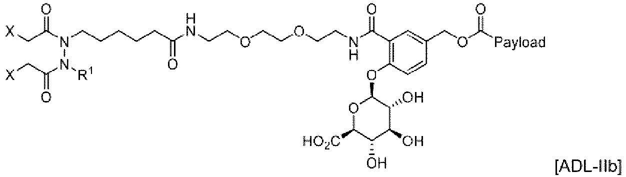 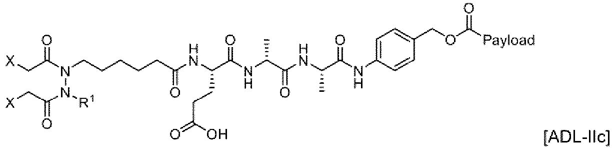 or 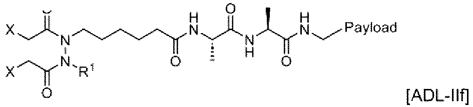 wherein Payload, X, and R¹ are as defined above.
[4-1] An antibody-drug conjugate, which is a compound or a salt thereof, the compound being represented by formula [ADC-Iα] or formula [ADC-IIα], 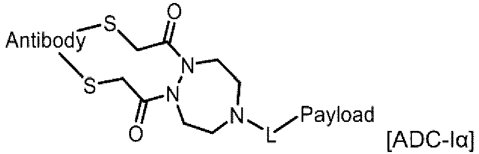 or 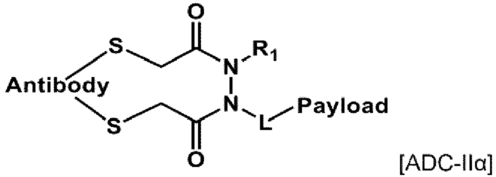 (wherein Payload is a drug residue;
   Antibody is an antibody residue;
   S is a sulfur atom of a cysteine residue in the antibody moiety;
   R¹ is hydrogen, C₁₋₆ alkyl optionally substituted with one or more substituents, or C₁₋₆ alkylene-R¹¹ optionally substituted with one or more substituents;
   R¹¹ is SO₃H, PO₃H₂, COOH, or an ester group of SO₃H, PO₃H₂, or COOH;
   L is a linker comprising L¹, L², and L³, wherein
   L¹ is 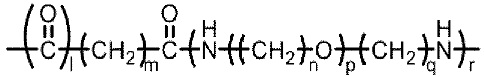 wherein each methylene is optionally substituted with one or more substituents (such as polyethylene glycol, saccharides (e.g., oligosaccharide), crown ether, polysarcosine, sulfonic acid-containing groups, phosphonic acid-containing groups, or amine- or ammonium salt-containing groups);
   L² is a bond or a cleavable linker;
   L³ is a bond, amino(aryl or heteroaryl)methyloxycarbonyl represented by 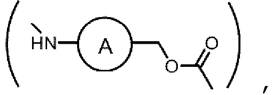
   amino(aryl or heteroaryl)methyl represented by 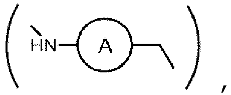
   aminomethyl represented by 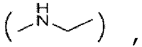
   or oxymethyl represented by 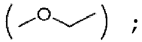 and
   A is aryl or heteroaryl each optionally substituted with one or more substituents;
   with the proviso that L² and L³ are not a bond at the same time; l and r are each independently 0 or 1; and
   m, n, p, and q are each independently an integer of 1 to 6).
[4-2] The antibody-drug conjugate according to [4-1], wherein L is L¹-L²-L³.
[4-3] The antibody-drug conjugate according to [4-1] or [4-2], wherein
   L¹ is 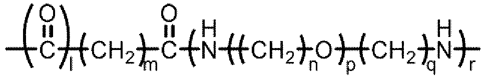 wherein each methylene is optionally substituted with polyethylene glycol or a saccharide (e.g., oligosaccharide);
   L² is a bond or a cleavable linker; and
   L³ is a bond, p-aminobenzyloxycarbonyl represented by 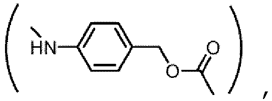
   p-aminobenzyl represented by 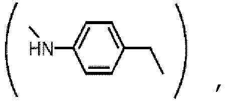
   or aminomethyl represented by 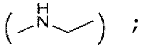 wherein the benzene rings in the p-aminobenzyloxycarbonyl and the p-aminobenzyl are each independently optionally substituted with one or more substituents.
[4-4] The antibody-drug conjugate according to any one of [4-1] to [4-3], which is a compound or a salt thereof, the compound being represented by formula [ADC-I] or formula [ADC-II]: 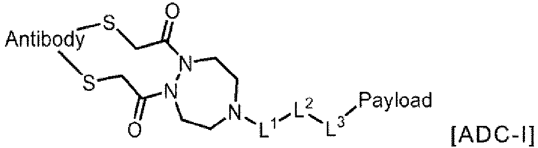 or 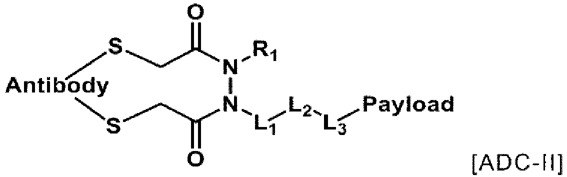 (wherein Payload is a drug residue; and
   Antibody is an antibody residue;
   S is a sulfur atom of a cysteine residue in the antibody moiety;
   R¹ is hydrogen, C₁₋₆ alkyl, or C₁₋₆ alkylene-R¹¹;
   R¹¹ is SO₃H, PO₃H₂, COOH, or an ester group of SO₃H, PO₃H₂, or COOH;
   L¹ is 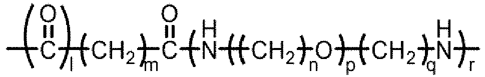 wherein each methylene is optionally substituted with polyethylene glycol or a saccharide (e.g., oligosaccharide);
   L² is a bond or a cleavable linker;
   L³ is a bond,
   L³ is a bond, p-aminobenzyloxycarbonyl represented by 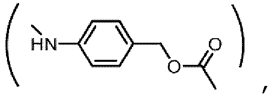
   p-aminobenzyl represented by 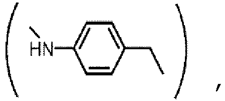
   or aminomethyl represented by 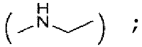 wherein the benzene rings in the p-aminobenzyloxycarbonyl and the p-aminobenzyl are each independently optionally substituted with one or more substituents;
   with the proviso that L² and L³ are not a bond at the same time;
   l and r are each independently 0 or 1; and
   m, n, p, and q are each independently an integer of 1 to 6).
[4-5] The antibody-drug conjugate according to any one of [4-1] to [4-4], wherein the cleavable linker is a cathepsin-cleavable linker, a pyrophosphatase/phosphoesterase-cleavable linker, a glycosidase-cleavable linker (a β-glucuronidase-cleavable linker), a carboxylesterase-cleavable linker, a legumain-cleavable linker, a neutrophil elastase-cleavable linker, a matrix metalloproteinase-cleavable linker, or a fibroblast activation protein-cleavable linker.
[4-6] The antibody-drug conjugate according to any one of [4-1] to [4-4], wherein the cleavable linker is a hydroxymethylbenzoic acid residue substituted with glucuronic acid, a peptide residue, a pyrophosphate (diphosphate) residue, or disulfide.
[4-7] The antibody-drug conjugate according to [4-6], wherein the hydroxymethylbenzoic acid residue substituted with glucuronic acid is 2-(1-D-glucuronyl)-5-hydroxymethylbenzoic acid.
[4-8] The antibody-drug conjugate according to [4-6], wherein the peptide is a dipeptide consisting of valine and citrulline, a dipeptide consisting of two alanines, a tripeptide consisting of glutamic acid, D-alanine, and alanine, a tripeptide consisting of glutamic acid, D-alanine, and alanine with polyethylene glycol or oligosaccharide added to the terminal carboxylic acid of the glutamic acid, or a tetrapeptide consisting of phenylalanine and three glycines.
[4-9] The antibody-drug conjugate according to any one of [4-1] to [4-8], wherein L¹ is 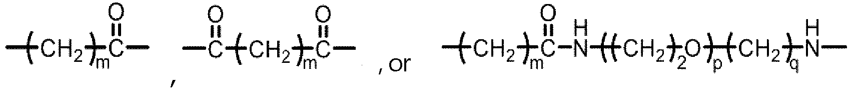 wherein m, p, and q are as defined above.
[4-10] The antibody-drug conjugate according to any one of [4-1] to [4-9], wherein L¹ is 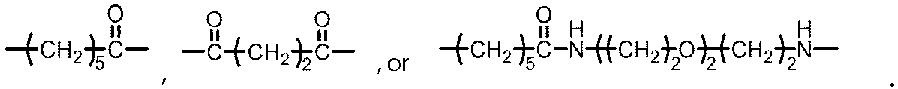
[4-11] The antibody-drug conjugate according to any one of [4-1] to [4-10], which is a compound or a salt thereof, the compound being represented by formula [ADC-Ia], formula [ADC-IIa], formula [ADC-IIb], formula [ADC-IIc], or formula [ADC-IId]: 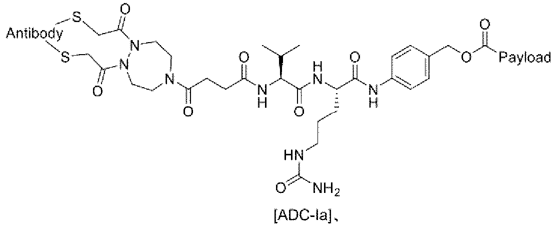 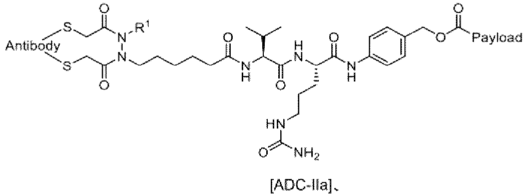 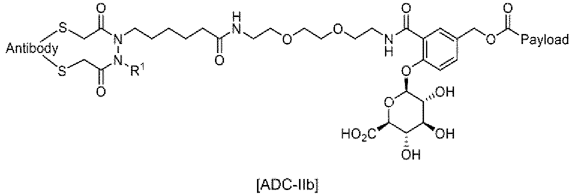 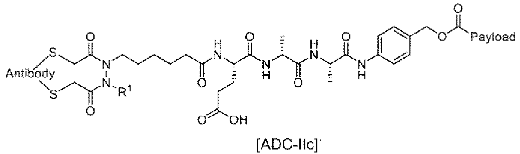 or 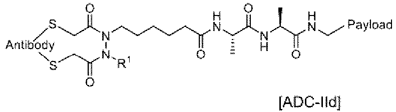 (wherein Payload, Antibody, S, and R¹ are as defined above).
[5-1] The antibody-drug conjugate according to any one of [4-1] to [4-11], wherein the antibody is IgG, IgM, IgA, or IgD.
[5-2] The antibody-drug conjugate according to any one of [4-1] to [4-11], wherein the antibody is IgG.
[5-3] The antibody-drug conjugate according to any one of [4-1] to [4-11], wherein the antibody is rituximab, trastuzumab, nivolumab, pembrolizumab, ipilimumab, mirikizumab, dupilumab, bevacizumab, infliximab, adalimumab, abciximab, daclizumab, palivizumab, etanercept, basiliximab, gemtuzumab, alemtuzumab, ibritumomab, alefacept, omalizumab, efalizumab, tositumomab, cetuximab, natalizumab, ranibizumab, panitumumab, eculizumab, rilonacept, certolizumab, romiplostim, belimumab, tocilizumab, atrizumab, milatuzumab, mepolizumab, pertuzumab, tremelimumab, inotuzumab, aflibercept, catumaxomab, oregovomab, motavizumab, efungumab, or raxibacumab.
[6-1] The antibody-drug conjugate according to any one of [4-1] to [4-11], wherein the drug is a low- or medium-molecular weight compound (preferably a compound with a molecular weight of about 15000 or less (including, for example, siRNA and antisense nucleic acid), or a compound with a molecular weight of about 6000 or less (including, for example, a peptide), or a compound with a molecular weight of about 2000 or less (including, for example, a natural product)).
[6-2] The antibody-drug conjugate according to any one of [4-1] to [4-11], wherein the drug is an anticancer drug, a cytocidal agent, an immunomodulator, or a toxin.
[6-3] The antibody-drug conjugate according to any one of [4-1] to [4-10], wherein the anticancer drug or the cytocidal agent is monomethyl auristatin E, monomethyl auristatin F, dolastatin, maytansinoid (e.g., maytansine or ansamitocin), camptothecin (e.g., synthetic analog topotecan, irinotecan, RFS2000), cryptophycin (e.g., cryptophycin 1 or cryptophycin 8), duocarmycin (e.g., synthetic analogs, KW-2189, CC-1065, carzelesin, bizelesin, and CB1-TM1), an antibiotic (e.g., enediyne antibiotics, such as calicheamicins selected from calicheamicin γ1I and calicheamicin ωI1, or dynemicin, such as dynemicin A), epothilone, rizoxin, taxoid (e.g., paclitaxel, docetaxel), a platinum analog (e.g., cisplatin or carboplatin), doxorubicin or a derivative thereof (e.g., morpholinodoxorubicin, cyanomorpholinodoxorubicin, 2-pyrrolino-doxorubicin, pirarubicin, daunorubicin, or deoxydoxorubicin), venetoclax, erlotinib, bortezomib, sunitinib, imatinib mesylate, lapatinib, lonafarnib, sorafenib, gefitinib, bisphosphonate (e.g., clodronate, ibandronate), neocarzinostatin chromophore, or related chromoproteins, enediyne antibiotic chromophore, dactinomycin, vinblastine, vincristine, vinorelbine, bryostatin, etoposide, teniposide, rapamycin, deofoamine, an antimetabolite (e.g., 5-fluorouracil (5-FU), capecitabine, gemcitabine, fludarabine, 6-mercaptopurine, thiamiprine, thioguanine, ancitabine, azacitidine, 6-azauridine, carmofur, cytarabine, dideoxy-uridine, doxifluridine, enocitabine, or floxuridine), a folic acid analog (e.g., denopterin, methotrexate, edatrexate, pteropterin, aminopterin, or trimetrexate), an androgen (e.g., calusterone, dromostanolone propionate, epitiostanol, or testolactone), or an anti-adrenal drug (e.g., aminoglutethimide).
[6-4] The antibody-drug conjugate according to [6-2], wherein the immunomodulator is a TLR7 agonist (e.g., imiquimod, resiquimod), a TLR8 agonist (e.g., VTX-2337), a TLR9 agonist (e.g., ODN2216, ODN1826, ODN2006, ODN2395, or ODN21798), a STING agonist (e.g., cGAMP, diABZI, or SR-717), a steroid (e.g., budesonide, dexamethasone, budenoside, triamcinolone, or triamcinolone acetonide), a PDE4 inhibitor (e.g., GSK256066), or an LCK inhibitor (e.g., dasatinib).
[6-5] The antibody-drug conjugate according to [6-2], wherein the toxin is diphtheria toxin, botulinum toxin, tetanus toxin, dysentery toxin, cholera toxin, tetrodotoxin, brevetoxin, shiga toxin, ricin, or AM-toxin.
[7-1] The antibody-drug conjugate according to any one of [4-1] to [4-11], wherein the drug-to-antibody ratio (DAR) is in the range of 2 to 6.
[7-2] The antibody-drug conjugate according to any one of [4-1] to [4-11], wherein the drug-to-antibody ratio (DAR) is in the range of 3.2 to 4.8.
[8-1] A method for producing an antibody-drug conjugate, comprising the steps of:
   a) reducing a disulfide of an antibody using a reducing agent in the presence of a chelating agent; and
   b) crosslinking the reduced product with the drug-linker intermediate of any one of [2-1] to [2-17].
[8-2] The method according to [8-1], wherein step b) is a crosslinking step performed in the absence of sodium iodide.
[8-3] The method according to [8-1], wherein step b) is a crosslinking step performed in the presence of sodium iodide.
[8-4] The method according to [8-1], wherein the sodium iodide is present in an amount of 100 to 40000 molar equivalents per molar equivalent of the antibody.
[8-5] The method according to [8-1], wherein the reaction time in step b) is 0.5 days to 7 days.

### Advantageous Effects of Invention

The compound or a salt thereof according to the present invention can strongly bond to an antibody to form an antibody-drug conjugate that is stable in blood. Further, the compound or a salt thereof according to the present invention allows an antibody-drug conjugate with a uniform structure to be easily obtained.

### Brief Description of Drawings

Fig. 1 is a high-performance liquid chromatography (HIC) chart of the antibody-drug conjugate obtained in Example 12.
Fig. 2 is an HIC chart of Example 21.
Fig. 3 is an HIC chart of Example 25.
Fig. 4 is a mass spectrum (MS) chart of Example 25.
Fig. 5 is an HIC chart 2 days after addition of 12000 equivalents of NaI when the reaction was performed using the antibody and drug-linker of Example 26.
Fig. 6 is a graph showing the stability of the antibody-drug conjugate of Example 17 in human plasma.
Fig. 7 is a graph showing the stability of the antibody-drug conjugate of Example 17 in mouse plasma.
Fig. 8 is a DSC chart of Example 17.

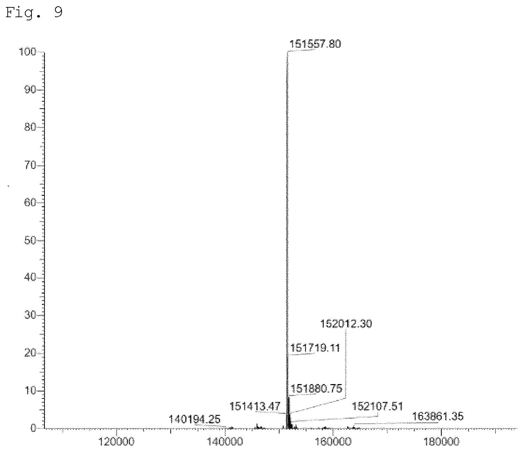

Fig. 9 is an MS chart of Example 37.

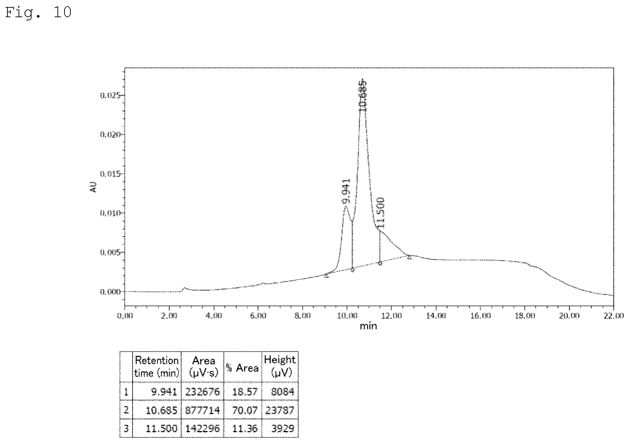

Fig. 10 is an HIC chart of Example 37.

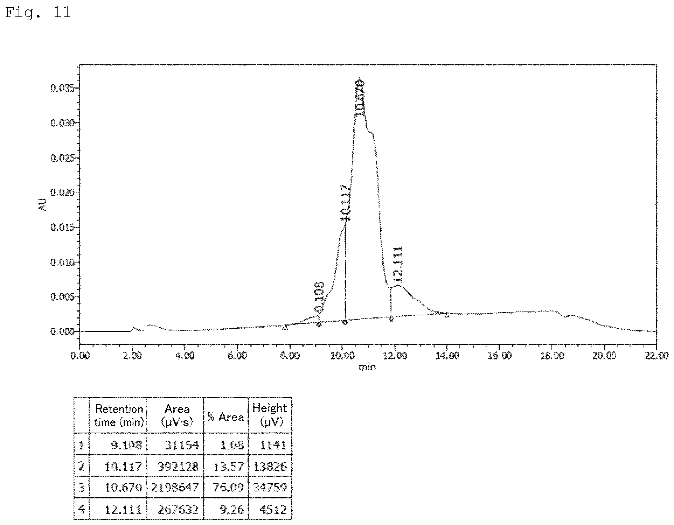

Fig. 11 is an HIC chart of Example 29.

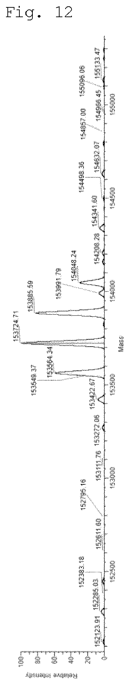

Fig. 12 is an MS chart of Example 29.

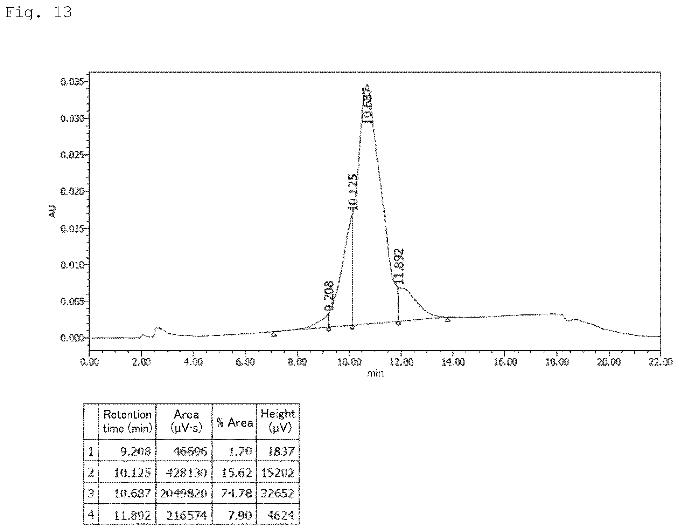

Fig. 13 is an HIC chart of Example 33.

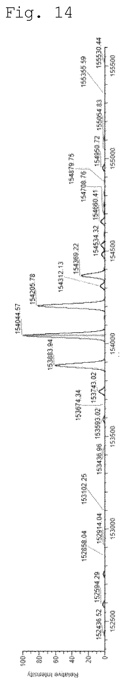

Fig. 14 is an MS chart of Example 33.

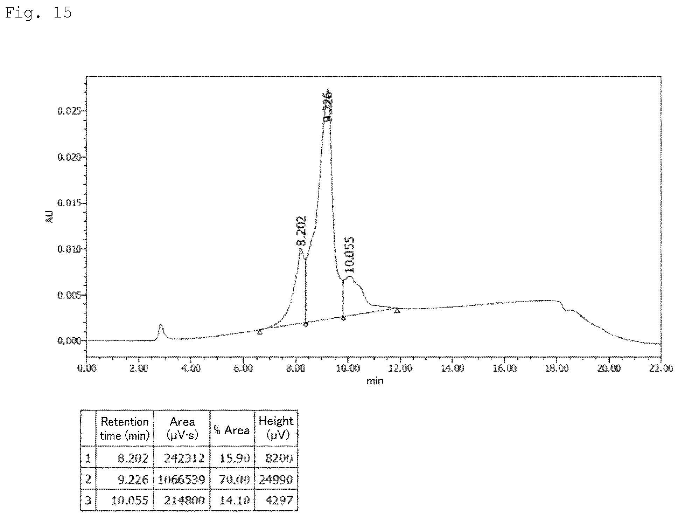

Fig. 15 is an HIC chart of Example 36.

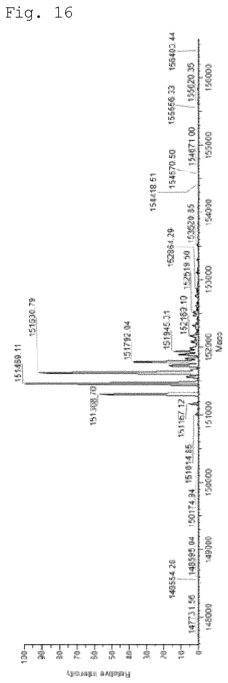

Fig. 16 is an MS chart of Example 36.

### Description of Embodiments

The phrases and terms used in the present specification are described in detail below.

In the present specification, "halogen" is fluorine, chlorine, bromine, or iodine; preferably fluorine, chlorine, or bromine; and more preferably fluorine or chlorine.

In the present specification, "C₁₋₆ alkyl" is linear or branched alkyl having 1 to 6 carbon atoms (C₁₋₆). Specific examples include methyl, ethyl, n-propyl, isopropyl, n-butyl, isobutyl, sec-butyl, tert-butyl, n-pentyl, isopentyl, neopentyl, n-hexyl, isohexyl, and 3-methylpentyl.

Examples of the "C₁₋₆ alkyl" further include C₁₋₆ alkyl in which 1 to 7 hydrogen atoms are replaced by deuterium atoms.

In the present specification, "C₁₋₆ alkylene" includes linear or branched alkylene having 1 to 6 carbon atoms (C₁₋₆). Specific examples include methylene, ethylene, 1-methylethylene, 2-methylethylene, trimethylene, 2-methyltrimethylene, 2,2-dimethyltrimethylene, 1-methyltrimethylene, methylmethylene, ethylmethylene, dimethylmethylene, tetramethylene, pentamethylene, and hexamethylene.

In the present specification, "aryl" includes aryl having 6 to 14 carbon atom (C₆₋₁₄ aryl), preferably C₆₋₁₀ aryl. Specific examples include monocyclic, bicyclic, or tricyclic aromatic hydrogen ring groups, such as phenyl, naphthyl, anthryl, and phenanthryl.

In the present specification, "heteroaryl" includes (monocyclic or polycyclic) aromatic heterocyclic groups containing 1 to 5 heteroatoms independently selected from the group consisting of nitrogen, oxygen, and sulfur as ring-constituting atoms. Examples include 3- to 15-membered monocyclic, bicyclic, or tricyclic aromatic heterocyclic groups. The heteroaryl is preferably 5- to 10-membered heteroaryl. Specific examples of heteroaryl groups include pyridyl, imidazolyl, triazolyl, indolyl, quinolinyl, oxazolyl, and thiazolyl.

In the present specification, "polyethylene glycol" is a polyether with a structure of polymerized ethylene glycol and preferably has a degree of polymerization of 2 to 96, more preferably a degree of polymerization of 4 to 48, and even more preferably a degree of polymerization of 8 to 24. The polyethylene glycol can be a mixture of polyethylene glycols with different degrees of polymerization, but preferably has a single degree of polymerization.

In the present specification, "oligosaccharide" is an oligomer of saccharides in which 2 to about 10 monosaccharides are linked by glycosidic bonds. Specific examples include sucrose, maltose, lactose, trehalose, maltotriose, raffinose, panose, melezitose, stachyose, maltooligosaccharides, oligosialic acids, hyaluronic acid, oligo(GlcNAcβ1-4GlcAβ1-3), chitosan, oligo(β1→4-N-glucosamine), oligo(β1→4-N-acetylglucosamine), cyclodextrin, or

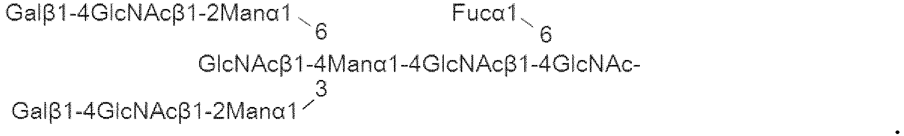

In the present specification, when a distinction is made between L-form and D-form amino acids or amino acid residues, unless otherwise specified, the term "amino acid" or "amino acid residue" may refer to the L-form even without explicitly stating "L-amino acid" or "L-amino acid residue"; whereas when the D-form of an amino acid or an amino acid residue is meant, it may be denoted as "D-amino acid" or "D-amino acid residue."

In the present specification, the amino acid or the amino acid residue also includes amino acids or amino acid residues substituted with polyethylene glycol, saccharides (e.g., oligosaccharide), crown ethers, polysarcosine, sulfonic acid-containing groups, phosphonic acid-containing groups, and/or amine- or ammonium salt-containing groups.

In the present specification, crown ethers are cyclic polyethers of ethylene glycol. Some crown ethers are entirely composed of ethylene glycol units, and others may contain one or more additional atoms (such as nitrogen atoms) or one or more substituents (such as carboxy groups, hydroxy groups, amino groups, aldehyde groups, or benzene rings) in the ring. Specific examples of crown ethers include those described in WO2017/178828.

In the present specification, the polysarcosine is a polymer with sarcosine (N-methyl glycine) as its monomer. Specific examples of polysarcosine include those described in WO2019/081455.

In the present specification, the sulfonic acid-containing group can be, for example, a group having -SO₃H at its end. The phosphonic acid-containing group can be, for example, a group having -PO₃H₂ at its end. The amine- or ammonium salt-containing group can be, for example, a group having -NH₂ or - N⁺(CH₃)₃ at its end. Specific example of these groups include those described in WO 2019/094395.

In the present specification, the "cleavable linker" is not particularly limited as long as it is a linker cleavable by an enzyme in vivo; however, the cleavable linker is preferably a linker cleavable by an enzyme present at the target site. Examples of cleavable linkers include cathepsin-cleavable linkers, pyrophosphatase/phosphoesterase-cleavable linkers, glycosidase-cleavable linkers (β-glucuronidase-cleavable linkers), carboxylesterase-cleavable linkers, legumain-cleavable linkers, neutrophil elastase-cleavable linkers, matrix metalloproteinase-cleavable linkers, and fibroblast activation protein-cleavable linkers.

In the present specification, the "cathepsin-cleavable linker" refers to a linker that is cleaved by cathepsin B. Examples include a dipeptide residue consisting of a valine residue-citrulline residue, and a dipeptide residue consisting of a valine residue-alanine residue.

In the present specification, the "pyrophosphatase/phosphoesterase-cleavable linker" refers to a linker that can be cleaved by pyrophosphatase/phosphoesterase, such as a pyrophosphate residue and a triphosphate residue.

In the present specification, the "glycosidase-cleavable linker (β-glucuronidase-cleavable linker)" refers to a linker that is cleaved by glycosidase (β-glucuronidase). Examples include a 2-(1-D-glucuronyl)-5-hydroxymethylbenzoic acid residue.

In the present specification, the "carboxylesterase-cleavable linker" refers to a linker that is cleaved by carboxylesterase. The linker is, for example, valine-citrulline (this linker is synonymous with a valine residue-citrulline residue; for the sake of convenience, the term "residue" is omitted. The same applies to the linkers described below), phenylalanine-lysine, valine-lysine, alanine-lysine, phenylalanine-citrulline, leucine-citrulline, isoleucine-citrulline, tryptophan-citrulline, phenylalanine-arginine, valine-alanine, alanine-alanine, alanine-D-alanine-alanine, glutamic acid-D-alanine-alanine, glutamic acid-valine-citrulline,

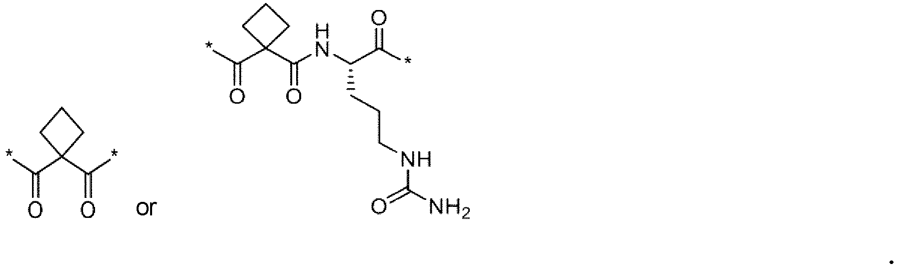

In the present specification, the "legumain-cleavable linker" refers to a linker that is cleaved by legumain. Examples include asparagine-asparagine, asparagine-alanine, and alanine-D-alanine-asparagine.

In the present specification, the "neutrophil elastase-cleavable linker" refers to a linker that is cleaved by neutrophil elastase. Examples include asparagine-proline-valine, alanine-proline-valine, D-alanine-proline-valine, alanine-proline-norvaline, alanine-alanine-valine, and alanine-alanine-proline-alanine.

In the present specification, the "matrix metalloproteinase-cleavable linker" refers to a linker that is cleaved by matrix metalloproteinases. Examples include serine-glycine-alanine-arginine-tyrosine-arginine-tryptophan-leucine-threonine-alanine, serine-glycine-arginine-serine-tyrosine-alanine-isoleucine-leucine-threonine-alanine, serine-arginine-serine-glycine-arginine-serine-proline-alanine-isoleucine-phenylalanine-threonine-alanine-threonine-glycine, glycine-serine-serine-glycine-arginine-serine-proline-alanine-isoleucine-phenylalanine-threonine-alanine-glycine-serine, serine-glycine-phenylalanine-isoleucine-alanine-asparagine-proline-valine-threonine-alanine, isoleucine-serine-serine-glycine-leucine-leucine-serine-serine, glutamine-asparagine-glutamine-alanine-leucine-arginine-methionine-alanine, alanine-glutamine-asparagine-leucine-leucine-glycine-methionine-valine, serine-threonine-phenylalanine-proline-phenylalanine-glycine-methionine-phenylalanine, proline-valine-glycine-tyrosine-threonine-serine-serine-leucine, aspartic acid-tryptophan-leucine-tyrosine-tryptophan-proline-glycine-isoleucine, methionine-isoleucine-alanine-proline-valine-alanine-tyrosine-arginine, arginine-proline-serine-proline-methionine-tryptophan-alanine-tyrosine, tryptophan-alanine-threonine-proline-arginine-proline-methionine-arginine, phenylalanine-arginine-leucine-leucine-aspartic acid-tryptophan-glutamine-tryptophan, leucine-lysine-alanine-alanine-proline-arginine-tryptophan-alanine, glycine-proline-serine-histidine-leucine-valine-leucine-threonine, leucine-proline-glycine-glycine-leucine-serine-proline-tryptophan, methionine-glycine-leucine-phenylalanine-serine-glutamic acid-alanine-glycine, serine-proline-leucine-proline-leucine-arginine-valine-proline, arginine-methionine-histidine-leucine-arginine-serine-leucine-glycine, leucine-alanine-alanine-proline-leucine-glycine-leucine-leucine, alanine-valine-glycine-leucine-leucine-alanine-proline-proline, leucine-leucine-alanine-proline-serine-histidine-arginine-alanine, proline-alanine-glycine-leucine-tryptophan-leucine-aspartic acid-proline, and isoleucine-serine-serine-glycine-leucine-serine-serine.

In the present specification, the "fibroblast activation protein-cleavable linker" refers to a linker that is cleaved by a fibroblast activation protein. Examples include D-alanine-proline, glycine-proline, alanine-proline, arginine-proline, lysine-proline, serine-proline, tyrosine-proline, proline-leucine, and proline-asparagine.

In the present specification, the "hydroxymethylbenzoic acid substituted with glucuronic acid" refers to a compound in which glucuronic acid is substituted with hydroxymethylbenzoic acid. Examples include 2-(1-D-glucuronyl)-5-hydroxymethylbenzoic acid.

In the present specification, examples of the "substituent" include polyethylene glycol, saccharides (e.g., oligosaccharide), or the following groups optionally substituted with polyethylene glycol, a saccharide (e.g., oligosaccharide), carboxylic acid, sulfonic acid, or phosphonic acid:
(A) halogen,
(B) -CN,
(C) -NO₂,
(E) -OH,
(F) -CHO,
(G) -COOH or ester groups thereof,
(H) -SO₃H or ester groups thereof,
(I) -SO₂H,
(J) -SH,
(K) =O,
(L) =S,
(M) -PO₃H₂ or ester groups thereof,
(N) alkyl optionally having at least one group independently selected from substituent groups (Ia) and (Ib),
(O) alkenyl optionally having at least one group independently selected from substituent groups (Ia) and (Ib),
(P) alkynyl optionally having at least one group independently selected from substituent groups (Ia) and (Ib),
(Q) alkoxy optionally having at least one group independently selected from substituent groups (Ia) and (Ib),
(R) alkenyl-O- optionally having at least one group independently selected from substituent groups (Ia) and (Ib),
(S) alkynyl-O- optionally having at least one group independently selected from substituent groups (Ia) and (Ib),
(T) alkyl-CO- optionally having at least one group independently selected from substituent groups (Ia) and (Ib),
(U) alkenyl-CO- optionally having at least one group independently selected from substituent groups (Ia) and (Ib),
(V) alkynyl-CO- optionally having at least one group independently selected from substituent groups (Ia) and (Ib),
(W) alkyl-COO- optionally having at least one group independently selected from substituent groups (Ia) and (Ib),
(X) alkoxy-CO- optionally having at least one group independently selected from substituent groups (Ia) and (Ib),
(Y) alkyl-S- optionally having at least one group independently selected from substituent groups (Ia) and (Ib),
(Z) alkyl-SO- optionally having at least one group independently selected from substituent groups (Ia) and (Ib),
(AA) alkyl-SO₂- optionally having at least one group independently selected from substituent groups (Ia) and (Ib),
(BB) cycloalkyl optionally having at least one group independently selected from substituent groups (Ia), (Ib), (Ic), and =O,
(CC) cycloalkoxy optionally having at least one group independently selected from substituent groups (Ia), (Ib), (Ic), and =O,
(DD) cycloalkenyl optionally having at least one group independently selected from substituent groups (Ia), (Ib), (Ic), and =O,
(EE) cycloalkenyl-O- optionally having at least one group independently selected from substituent groups (Ia), (Ib), (Ic), and =O,
(FF) cycloalkyl-CO- optionally having at least one group independently selected from substituent groups (Ia), (Ib), (Ic), and =O,
(GG) cycloalkoxy-CO- optionally having at least one group independently selected from substituent groups (Ia), (Ib), (Ic), and =O,
(HH) aryl optionally having at least one group independently selected from substituent groups (Ia), (Ib), and (Ic),
(II) aryl-O- optionally having at least one group independently selected from substituent groups (Ia), (Ib), and (Ic),
(JJ) aryl-CO- optionally having at least one group independently selected from substituent groups (Ia), (Ib), and (Ic),
(KK) aryl-O-CO- optionally having at least one group independently selected from substituent groups (Ia), (Ib), and (Ic),
(LL) aralkyl optionally having at least one group independently selected from substituent groups (Ia), (Ib), and (Ic),
(MM) aralkyl-O- optionally having at least one group independently selected from substituent groups (Ia), (Ib), and (Ic),
(NN) aralkyl-CO- optionally having at least one group independently selected from substituent groups (Ia), (Ib), and (Ic),
(OO) aralkyl-O-CO- optionally having at least one group independently selected from substituent groups (Ia), (Ib), and (Ic),
(PP) a heterocyclic group optionally having at least one group independently selected from substituent groups (Ia), (Ib), (Ic), and =O,
(QQ) heterocycle-O- optionally having at least one group independently selected from substituent groups (Ia), (Ib), (Ic), and =O,
(RR) heterocycle-CO- optionally having at least one group independently selected from substituent groups (Ia), (Ib), (Ic), and =O,
(SS) heterocycle-O-CO- optionally having at least one group independently selected from substituent groups (Ia), (Ib), (Ic), and =O,
(TT) amino optionally having at least one group independently selected from substituent groups (Ia) and (Ic), and
(UU) carbamoyl optionally having at least one group independently selected from substituent groups (Ia) and (Ic).

The number of substituents is not limited as long as it is chemically acceptable. The number is, for example, 1 to 4, 1 to 3, or 1 or 2.

The "substituent group (Ia)" is a group consisting of:
(a) alkyl-CO- optionally having at least one group independently selected from substituent groups (IIa) and (IIb),
(b) alkenyl-CO- optionally having at least one group independently selected from substituent groups (IIa) and (IIb),
(c) alkynyl-CO- optionally having at least one group independently selected from substituent groups (IIa) and (IIb),
(d) alkoxy-CO- optionally having at least one group independently selected from substituent groups (IIa) and (IIb),
(e) alkyl-S- optionally having at least one group independently selected from substituent groups (IIa) and (IIb),
(f) alkyl-SO- optionally having at least one group independently selected from substituent groups (IIa) and (IIb),
(g) alkyl-SO₂- optionally having at least one group independently selected from substituent groups (IIa) and (IIb),
(h) cycloalkyl optionally having at least one group independently selected from substituent groups (IIa), (IIb), (IIc) and =O,
(i) cycloalkoxy optionally having at least one group independently selected from substituent groups (IIa), (IIb), (IIc), and =O,
(j) cycloalkenyl optionally having at least one group independently selected from substituent groups (IIa), (IIb), (IIc), and =O,
(k) cycloalkyl-CO- optionally having at least one group independently selected from substituent groups (IIa), (IIb), (IIc), and =O,
(l) cycloalkoxy-CO- optionally having at least one group independently selected from substituent groups (IIa), (IIb), (IIc), and =O,
(m) aryl optionally having at least one group independently selected from substituent groups (IIa), (IIb), and (IIc),
(n) aryl-CO- optionally having at least one group independently selected from substituent groups (IIa), (IIb), and (IIc),
(o) aryl-O-CO- optionally having at least one group independently selected from substituent groups (IIa), (IIb), and (IIc),
(p) aralkyl optionally having at least one group independently selected from substituent groups (IIa), (IIb), and (IIc),
(q) aralkyl-CO- optionally having at least one group independently selected from substituent groups (IIa), (IIb), and (IIc),
(r) aralkyl-O-CO- optionally having at least one group independently selected from substituent groups (IIa), (IIb), and (IIc),
(s) heterocyclic group optionally having at least one group independently selected from substituent groups (IIa), (IIb), (IIc) and =O,
(t) heterocycle-CO- optionally having at least one group independently selected from substituent groups (IIa), (IIb), (IIc), and =O,
(v) heterocycle-O-CO- optionally having at least one group independently selected from substituent groups (IIa), (IIb), (IIc), and =O, and
(w) carbamoyl optionally having at least one group independently selected from substituent groups (IIa) and (IIc).

The "substituent group (Ib)" is a group consisting of:
(a) halogens,
(b) -CN,
(c) -NO₂,
(d) -OH,
(e) -CHO,
(f) -COOH,
(g) -SO₃H,
(h) -SH,
(i) alkoxy optionally having at least one group independently selected from substituent groups (IIa) and (IIb),
(j) alkenyl-O- optionally having at least one group independently selected from substituent groups (IIa) and (IIb),
(k) alkynyl-O- optionally having at least one group independently selected from substituent groups (IIa) and (IIb),
(l) alkyl-COO- optionally having at least one group independently selected from substituent groups (IIa) and (IIb),
(m) cycloalkenyl-O- optionally having at least one group independently selected from substituent groups (IIa), (IIb), (IIc), and =O,
(n) aryl-O- optionally having at least one group independently selected from substituent groups (IIa), (IIb), and (IIc),
(o) aralkyl-O- optionally having at least one group independently selected from substituent groups (IIa), (IIb), and (IIc),
(p) heterocycle-O- optionally having at least one group independently selected from substituent groups (IIa), (IIb), (IIc), and =O, and
(q) amino optionally having at least one group independently selected from substituent groups (IIa) and (IIc).

The "substituent group (Ic)" is a group consisting of
(a) alkyl optionally having at least one group independently selected from substituent groups (IIa) and (IIb),
(b) alkenyl optionally having at least one group independently selected from substituent groups (IIa) and (IIb), and
(c) alkynyl optionally having at least one group independently selected from substituent groups (IIa) and (IIb).

The "substituent group (IIa)" is a group consisting of -CHO, alkyl-CO-, alkenyl-CO-, alkynyl-CO-, alkoxy-CO-, alkyl-SO₂-, cycloalkyl, cycloalkoxy, cycloalkenyl, cycloalkyl-CO-, cycloalkoxy-CO-, aryl, aryl-CO-, aryl-O-CO-, aralkyl, aralkyl-CO-, aralkyl-O-CO-, heterocycle, heterocycle-CO-, heterocycle-O-CO-, mono- or di-(alkyl-CO)-carbamoyl, and mono- or dialkylcarbamoyl.

The "substituent group (IIb)" is a group consisting of halogen, -CN, -NO₂, -OH, -COOH, -SO₃H, -SH, -NH₂, alkoxy, alkenyl-O-, alkynyl-O-, alkyl-COO-, alkyl-S-, alkyl-SO-, cycloalkenyl-O-, aryl-O-, aralkyl-O-, heterocycle-O-, mono- or di-alkylamino, mono- or di-(alkyl-CO)-amino, mono- or di-alkoxycarbonylamino, mono- or di-arylcarbonylamino, and mono- or di-aralkylcarbonylamino.

The "substituent group (IIc)" is a group consisting of alkyl, alkenyl, and alkynyl.

In the present specification, the "antibody" is not particularly limited as long as it has high affinity for the antigen present at the target site. The antibody is, for example, IgM, IgA, or IgD, and is preferably IgG. The IgG is, for example, IgG1, IgG2, IgG3, or IgG4, and is preferably IgG1 or IgG4. The antibody can be a polyclonal antibody, but is preferably a monoclonal antibody. The antibody can be, for example, a murine antibody, a chimeric antibody, a humanized antibody, or a human antibody. The antibody can be an antigen-binding fragment. Examples of antigen-bonded fragments include Fab, Fab', F(ab')2, Fv, scFv, scFv-Fc, diabody, triabody, tetrabody, minibody, and nanobody (VHH).

Antibodies that can be used in the present invention may themselves have pharmacological effects. Such antibodies have enhanced affinity for antigens present at the target site and thus allow for efficient delivery of ADCs to the target site. Following effective release of the drug at the target site, such antibodies can treat the target site through their own efficacy. Examples of such antibodies include rituximab, trastuzumab, nivolumab, pembrolizumab, ipilimumab, mirikizumab, dupilumab, bevacizumab, infliximab, adalimumab, abciximab, daclizumab, palivizumab, etanercept, basiliximab, gemtuzumab, alemtuzumab, ibritumomab, alefacept, omalizumab, efalizumab, tositumomab, cetuximab, natalizumab, ranibizumab, panitumumab, eculizumab, rilonacept, certolizumab, romiplostim, belimumab, tocilizumab, atrizumab, milatuzumab, mepolizumab, pertuzumab, tremelimumab, inotuzumab, aflibercept, catumaxomab, oregovomab, motavizumab, efungumab, and laxibacumab.

In the present specification, the "Payload" (medicament or drug) can be loaded onto the antibody-drug conjugate, and it can be a low molecular weight compound or medium molecular weight compound that is released in the target tissue when the cleavable linker is cleaved by enzymes in the body, and can exert its pharmacological effect in the tissue.

In the present specification, the "low molecular weight compound" refers to a compound with a molecular weight of about 500 or less, and the "medium molecular weight compound" preferably refers to a compound with a molecular weight of about 500 to about 2000. In the present specification, "about" means within an acceptable error range for the indicated value, for example, within ±10% (preferably ±5%) of the indicated value.

The Payload can be modified as long as the desired pharmacological effect is maintained. In particular, modifications with an amino group, carboxylic acid, or hydroxy group at the end are preferred to form a covalent bond with the drug-linker intermediate.

Specific examples of medicaments include anticancer drugs, cytocidal agents, immunomodulators, and toxins.

Examples of anticancer drugs and cytocidal agents include monomethyl auristatin E, monomethyl auristatin F, venetoclax, erlotinib, bortezomib, fulvestrant, sutent, letrozole, imatinib mesylate, oxaliplatin, leucovorin, rapamycin, lapatinib, lonafarnib, sorafenib, gefitinib, alkylating agents (e.g., thiotepa, cyclophosphamide), alkylsulfonates (e.g., busulfan, improsulfan, or piposulfan), aziridines (e.g., benzodopa, carboquone, meturedopa, or uredopa), ethylenimines, methylmelamine, altretamine, triethylenemelamine, triethylene phosphoramide, triethylene thiophosphoramide, trimethylmelamine, acetogenins (e.g., bullatacin or bullatacinone), camptothecins (e.g., synthetic analogs topotecan, irinotecan, topoisomerase inhibitors (RFS2000)), bryostatins, kallistatins, adozelesin, cryptophycins (e.g., cryptophycin 1 or cryptophycin 8), dolastatins, duocarmycins (e.g., synthetic analogs KW-2189, CC-1065, carzelesin, bizelesin, and CB1-TM1), eloterobin, pancratistatins, sarcodictins, spongiostatins, nitrogen mustards (e.g., chlorambucil, chlornaphazine, chlorophosphamide, estramustine, ifosfamide, mechlorethamine, mechlorethamine oxide hydrochloride, melphalan, nobembitine, phenesterine, prednimustine, trofosfamide, or uracil mustard), nitrosoureas (e.g., carmustine, chlorozotocin, fotemustine, lomustine, nimustine, or ranimustine), antibiotics (e.g., enediyne antibiotics, such as calicheamicin selected from calicheamicin γ1I and calicheamicin ω11, or dynemicin such as dynemicin A), bisphosphonates (e.g., clodronate, iband, lonate, esperamicin, neocarzinostatin chromophore, or related chromoproteins, enediyne antibiotic chromophores, aclacinomycin, actinomycin, anthramycin, azaserine, bleomycin, cactinomycin, carabicin, carminomycin, carzinophilin, chromomycin, dactinomycin, daunorubicin, detorubicin, 6-diazo-5-oxo-L-norleucine, doxorubicin (e.g., morpholinodoxorubicin, cyanomorpholinodoxorubicin, 2-pyrrolino-doxorubicin, liposomal doxorubicin, or deoxydoxorubicin), epirubicin, esorubicin, marcelomycin, mitomycins (e.g., mitomycin C, mycophenolic acid, nogalamycin, olivomycin, peplomycin, potfilomycin, puromycin, quelamycin, rodorubicin, streptonigrin, streptozocin, tubercidin, ubenimex, zinostatin, or zorubicin), antimetabolites (e.g., 5-fluorouracil (5-FU), capecitabine, gemcitabine, folic acid analogues (e.g., denopterin, methotrexate, edatrexate, pteropterin, aminopterin, or trimetrexate), purine analogues (e.g., fludarabine, 6-mercaptopurine, thiamiprine, or thioglucose), pyrimidine analogues (e.g., ancitabine, azacitidine, 6-azauridine, carmofur, cytarabine, dideoxyuridine, doxifluridine, enocitabine, or floxuridine), androgens (e.g., calsterone, dromostanolone propionate, epithiostanol, mepitiostane) or testolactone), antiadrenal drugs (e.g. aminoglutethimide, mitotane, or trilostane), folic acid supplements (e.g. folinic acid), aceglatone, aldophosphamide glycosides, aminolevulinic acid, eniluracil, amsacrine, bestravcil, bisantrene, edatraxate, defofamine, demecolcine, diaziquone, elfornithine, elliptinium acetate, epothilone, etoglucide, gallium nitrate, hydroxyurea, lentinan, lonidain, maytansinoids (e.g., maytansine or ansamitocin), trichothecenes (in particular, T-2 toxin, veracrine A, roridin A, or anguidine), mitoguazone, mitoxantrone, mopidamol, nitraelin, pentostatin, phenamet, pirarubicin, losoxantrone, 2-ethylhydrazide, procarbazine, razoxane, rhizoxin, schizofuran, spirogermanium, tenuazonic acid, triaziquone, 2,2',2"-trichlorotriethylamine, urethane, vindesine, dacarbazine, mannomustine, mitobronitol, mitolactol, pipobroman, gacytosine, arabinoside ("Ara-C"), cyclophosphamide, thiotepa, taxoids (e.g., paclitaxel and doxetaxel), chlorambucil, 6-thioguanine, mercaptopurine, platinum analogs (e.g., cisplatin or carboplatin), vinblastine, platinum, etoposide, ifosfamide, mitoxantrone, vincristine, vinorelbine, novantrone, teniposide, daunomycin, xeloda, CPT-11, difluoromethylornithine (DFMO), and retinoids (e.g., retinoic acid).

Examples of immunomodulators include TLR7 agonists (e.g., imiquimod, resiquimod), TLR8 agonists (e.g., VTX-2337), TLR9 agonists (e.g., ODN2216, ODN1826, ODN2006, ODN2395, ODN21798), STING agonists (e.g., cGAMP, diABZI, SR-717), steroids (e.g., budesonide, dexamethasone, budenoside, triamcinolone, triamcinolone acetonide), PDE4 inhibitors (e.g., GSK256066), and LCK inhibitors (e.g., dasatinib).

Examples of toxins include plant toxins and animal toxins. Examples of animal toxins include diphtheria toxin, botulinum toxin, tetanus toxin, dysentery toxin, cholera toxin, tetrodotoxin, brevetoxin, and shiga toxin. Examples of plant toxins include ricin and AM-toxin.

The antibody-drug conjugate provides no or only weak efficacy as long as the medicament and the antibody are integrated. Even the medicament that is highly cytotoxic and cannot be administered alone can be administered in the form of an antibody-drug conjugate.

In the present specification, "Drug-to-Antibody Ratio (DAR)" represents the average number of drug molecules loaded per antibody molecule. In the present invention, the DAR is preferably 1, 2, 3, 4, 5, 6, 7, or 8, more preferably 2, 4, 6, or 8, and even more preferably 4. In the present invention, the DAR is preferably 2 to 6, more preferably 3.2 to 4.8, and even more preferably 3.6 to 4.4.

In the present specification, the "protecting group" is not particularly limited as long as it functions as a protecting group. Examples include alkyl groups (e.g., methyl, ethyl, isopropyl, tert-butyl, trifluoromethyl, hydroxymethyl, 2-hydroxyethyl, and acetylmethyl); alkyl(alkenyl)carbonyl groups (e.g., acetyl, propionyl, butyryl, isobutyryl, pentanoyl, pivaloyl, valeryl, isovaleryl, chloroacetyl, dichloroacetyl, trichloroacetyl, trifluoroacetyl, methoxyacetyl, acryloyl, propioloyl, methacryloyl, crotonoyl, isocrotonoyl, and (E)-2-methyl-2-butenoyl); arylcarbonyl groups (e.g., benzoyl, α-naphthoyl, β-naphthoyl, 2-bromobenzoyl, 4-chlorobenzoyl, 2,4,6-trimethylbenzoyl, 4-toluoyl, 4-anisoyl, 4-nitrobenzoyl, 2-nitrobenzoyl, 2-(methoxycarbonyl)benzoyl, and 4-phenylbenzoyl); tetrahydro(thio)pyranyl(furanyl) groups (e.g., tetrahydropyran-2-yl and 3-bromotetrahydropyran-2-yl); silyl groups (e.g., trimethylsilyl, triethylsilyl, isopropyldimethylsilyl, tert-butyldimethylsilyl, tert-butyldiphenylsilyl, methyldiisopropylsilyl, methyldi-tert-butylsilyl, triisopropylsilyl, diphenylmethylsilyl, diphenylbutylsilyl, diphenylisopropylsilyl, phenyldiisopropylsilyl, triphenylsilyl, and di-tert-butylisobutylsilyl); alkoxymethyl groups (e.g., methoxymethyl, 1,1-dimethyl-1-methoxymethyl, ethoxymethyl, propoxymethyl, isopropoxymethyl, butoxymethyl, tert-butoxymethyl, 2-methoxyethoxymethyl, 2,2,2-trichloroethoxymethyl, and bis(2-chloroethoxy)methyl); aralkyl groups (e.g., benzyl, α-naphthylmethyl, β-naphthylmethyl, diphenylmethyl, triphenylmethyl, α-naphthyldiphenylmethyl, 9-anthrylmethyl, 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl, and 4-cyanobenzyl); and carbamate groups (e.g., tert-butyl carbamate, allyl carbamate, and benzyl carbamate).

In the present specification, the "protecting agent" is not particularly limited as long as it can introduce a protecting group to the desired functional group. Examples include alkylating agents (e.g., dimethyl sulfate, diazomethane, methyl bromide, methyl iodide, Meerwein's reagent, methyl trifluoromethanesulfonate, ethyl bromide, isobutylene, and 2-hydroxyethyl bromide); alkyl (alkenyl) carbonylating agents (e.g., acetic anhydride, acetyl chloride, ketene, propionyl chloride, butyryl chloride, pivaloyl chloride, chloroacetyl chloride, and trifluoroacetic anhydride); aryl carbonylating agents (e.g., benzoyl chloride, benzoic anhydride, benzoyl cyanide, and α-naphthoyl chloride); tetrahydro(thio)pyranyl(furanyl)ating agents (3,4-dihydro-2H-pyran, 2,3-dihydrofuran, 2-chlorotetrahydrofuran); silylating agents (using, for example, trimethylsilyl chloride, triethylsilyl chloride, isopropyldimethylsilyl chloride, tert-butyldimethylsilyl chloride, methyldiisopropylsilyl chloride, methyl-di-tert-butylsilyl chloride, triisopropylsilyl chloride, diphenylmethylsilyl chloride, diphenylbutylsilyl chloride, diphenylisopropylsilyl chloride, phenyldiisopropylsilyl chloride, triphenylsilyl chloride, or di-tert-butylisobutylsilyl triflate, and further using a base such as imidazole, pyridine, or 2,6-lutidine); alkoxymethylating agents (e.g., methoxymethyl chloride, methoxymethyl bromide, dimethoxymethane, ethoxymethyl chloride, 2-methoxyethoxymethyl chloride, 2,2,2-trichloroethoxymethyl chloride, 2-trimethylsilylethoxymethyl chloride, benzyloxymethoxymethyl chloride, and ethyl vinyl ether); aralkylating agents (e.g., benzyl chloride, benzyl bromide, benzyl 2,2,2-trichloroacetimidate, 4-methoxybenzyl chloride, triphenylmethyl chloride, and triphenylmethyl bromide); and carbamate groups (e.g., di-tert-butyl dicarbonate, allyl chloroformate, diallyl dicarbonate, benzyl chloroformate, and dibenzyl dicarbonate).

In the present specification, the "deprotecting agent" is not particularly limited as long as it can deprotect a protecting group. Examples include alkyl groups (e.g., trimethylsilyl iodide, boron tribromide, and aluminum chloride/ethanethiol); alkyl(alkenyl)carbonyl groups (e.g., strong alkaline aqueous solutions, ammonia water, methylamine, 2-aminoethanethiol, thiourea, tetrabutylammonium hydroxide, diisobutylaluminum hydride, lithium aluminum hydride, hydrazine, and boron trifluoride diethyl ether complex/dimethyl sulfide); arylcarbonyl groups (deprotecting agents for
alkyl(alkenyl)carbonyl groups can be used); tetrahydropyranyl (furanyl) groups (e.g., pyridinium p-toluenesulfonate, p-toluenesulfonic acid, acetic acid, hydrochloric acid, and trifluoroacetic acid); silyl groups (e.g., tetra-n-butylammonium fluoride/tetrahydrofuran, potassium carbonate/methanol, 2% hydrofluoric acid, and hydrofluoric acid/pyridine); alkoxymethyl groups (e.g., pyridinium p-toluenesulfonate, thiophenol/boron trifluoride diethyl ether complex, catecholboron bromide, trimethylsilyl bromide, bromodimethylborane, lithium tetrafluoroborate, hydrochloric acid, trifluoroacetic acid, zinc dibromide, titanium tetrachloride, trimethylsilyl chloride/sodium iodide, tetrafluoroboric acid, zinc, zinc/copper, and lithium/ammonia); allyl groups (e.g., hydrogen/palladium carbon, ammonium formate/palladium carbon, Raney nickel, trimethylsilyl iodide, boron tribromide, boron trichloride, dichlorodicyanoquinone, and cerium ammonium nitrite); and carbamate groups (for example, for deprotecting tert-butyl carbamate groups, hydrochloric acid/ethyl acetate, trifluoroacetic acid, trimethylsilyl iodide, and aluminum chloride/anisole; for deprotecting allyl carbamate groups, combinations of a palladium(0) catalyst (e.g., tetrakis(triphenylphosphine)palladium or tris(dibenzylideneacetone)dipalladium) and a nucleophile (e.g., morpholine, dimedone, formic acid, or 2-ethylhexanoic acid), and iodine/aqueous acetonitrile; for deprotecting benzyl carbamate groups, catalytic hydrogenolysis using palladium carbon, trimethylsilyl iodide, trifluoroacetic acid).

In the present specification, the "condensing agent" is not particularly limited. Specific example include 1-[3-(dimethylamino)propyl]-3-ethylcarbodiimide hydrochloride (WSC·HCl), N,N'-dicyclohexylcarbodiimide (DCC), N,N'-diisopropylcarbodiimide (DIC), N,N'-carbonyldiimidazole (CDI), 4-(4,6-dimethoxy-1,3,5-triazin-2-yl)-4-methylmorpholinium chloride (DMTMM), benzotriazol-1-yloxytris(dimethylamino)phosphonium hexafluorophosphate (BOP), benzotriazol-1-yloxytripyrrolidinophosphonium hexafluorophosphate (PyBOP), O-(7-azabenzotriazol-1-yl)-1,1,3,3-tetramethyluronium hexafluorophosphate (HATU), and (1-cyano-2-ethoxy-2-oxoethylidenaminooxy)dimethylaminomorpholinocarbenium hexafluorophosphate (COMU), and preferably WSC·HCl, HATU, and COMU.

In the present specification, "acid" is not particularly limited. Examples include inorganic acids and organic acids. Examples of "inorganic acids" include hydrochloric acid, sulfuric acid, nitric acid, hydrobromic acid, and phosphoric acid. Examples of "organic acids" include acetic acid, trifluoroacetic acid, oxalic acid, phthalic acid, fumaric acid, tartaric acid, maleic acid, citric acid, succinic acid, methanesulfonic acid, p-toluenesulfonic acid, and 10-camphorsulfonic acid. These can be appropriately selected, mixed, and used either singly or in a combination of two or more.

In the present specification, "base" includes inorganic bases and organic bases. Examples of "inorganic bases" include alkali metal hydroxides (e.g., sodium hydroxide, potassium hydroxide), alkaline earth metal hydroxides (e.g., magnesium hydroxide, calcium hydroxide), alkali metal carbonates (e.g., sodium carbonate, potassium carbonate), alkaline earth metal carbonates (e.g., magnesium carbonate, calcium carbonate), alkali metal bicarbonates (e.g., sodium bicarbonate, potassium bicarbonate), alkali metal phosphates (e.g., sodium phosphate, potassium phosphate), and alkaline earth metal phosphates (e.g., sodium phosphate, potassium phosphate). Examples of "organic bases" include trialkylamine (e.g., trimethylamine, triethylamine, diisopropylethylamine), picoline, 1,5-diazabicyclo[4.3.0]non-5-ene, 1,4-diazabicyclo[2.2.2]octane, and 1,8-diazabicyclo[5.4.0]undec-7-ene.

In the present specification, the "reducing agent" is not particularly limited as long as it can reduce the desired functional group. Examples include lithium aluminum hydride, diisobutylaluminum hydride, sodium dihydrobis(2-methoxyethoxy)aluminate, and lithium borohydride.

In the present specification, the "leaving group" is, for example, a halogen, alkanesulfonyl optionally substituted with one or more substituents (e.g., C₁₋₁₈ alkanesulfonyl such as methanesulfonyl), an O-electron-withdrawing group (e.g., acyloxy optionally substituted with one or more substituents, aryloxy optionally substituted with one or more substituents (e.g., C₆₋₁₄ aryloxy such as phenyloxy), alkanesulfonyloxy optionally substituted with one or more substituents (e.g., C₁₋₆ alkanesulfonyloxy such as methylsulfonyloxy or ethylsulfonyloxy), arylsulfonyloxy optionally substituted with one or more substituents (e.g., C₆₋₁₄ arylsulfonyloxy such as benzenesulfonyloxy, p-toluenesulfonyloxy, 2,4,6-trimethylbenzenesulfonyloxy, 2-nitrobenzenesulfonyloxy, 4-nitrobenzenesulfonyloxy), aralkylsulfonyloxy, perhaloalkanesulfonyloxy (e.g., perhalo C₁₋₆ alkanesulfonyloxy such as trifluoromethylsulfonyloxy), sulfonio, or arylsulfoxy optionally substituted with one or more substituents (e.g., C₆₋₁₄ arylsulfoxy such as toluenesulfoxy), and is preferably a halogen.

The "halogen" is fluorine, chlorine, bromine, or iodine.

Examples of the "alkanesulfonyl" include linear or branched alkanesulfonyl having 1 to 18 carbon atoms (C₁₋₁₈ alkanesulfonyl). Specific examples include methanesulfonyl, 1-propanesulfonyl, 2-propanesulfonyl, butanesulfonyl, cyclohexanesulfonyl, dodecanesulfonyl, and octadecanesulfonyl.

The "electron-withdrawing group" means a group whose substituent constant (σ) in the Hammett equation has a positive value. Examples of electron-withdrawing groups include trifluoromethyl, cyano, acyl, phenyl optionally substituted with substituents, C₁₋₆ alkanesulfonyl, arylsulfonyl, aralkylsulfonyl, and perhaloalkanesulfonyl.

Examples of the "acyloxy" include linear or branched alkylcarbonyloxy having 1 to 6 carbon atoms. Specific examples include acetoxy and propionyloxy.

Examples of the "aryloxy optionally substituted with one or more substituents" include phenyloxy that may have 1 to 3 groups selected from the group consisting of linear or branched alkyl having 1 to 6 carbon atoms, linear or branched alkoxy groups 1 to 6 carbon atoms, nitro, and halogen as substituents on the phenyl ring. Specific examples include p-nitrophenyloxy.

Examples of the "alkanesulfonyloxy" include linear or branched alkanesulfonyloxy having 1 to 6 carbon atoms (C₁₋₆ alkanesulfonyloxy). Specific examples include methanesulfonyloxy, ethanesulfonyloxy, 1-propanesulfonyloxy, 2-propanesulfonyloxy, 1-butanesulfonyloxy, 3-butanesulfonyloxy, 1-pentanesulfonyloxy, and 1-hexanesulfonyloxy.

Examples of the "arylsulfonyloxy" include phenylsulfonyloxy optionally having 1 to 3 groups selected from the group consisting of C₁₋₆ linear or branched alkyl, C₁₋₆ linear or branched alkoxy, nitro, and halogen as substituents on the phenyl ring, and naphthylsulfonyloxy. Specific examples of the "phenylsulfonyloxy optionally having substituents" include phenylsulfonyloxy, 4-methylphenylsulfonyloxy, 2-methylphenylsulfonyloxy, 4-nitrophenylsulfonyloxy, 4-methoxyphenylsulfonyloxy, 2-nitrophenylsulfonyloxy, and 3-chlorophenylsulfonyloxy. Specific examples of the "naphthylsulfonyloxy" include α-naphthylsulfonyloxy and β-naphthylsulfonyloxy.

Specific examples of the "aralkylsulfonyloxy" include linear or branched alkanesulfonyloxy having 1 to 6 carbon atoms substituted with phenyl optionally having 1 to 3 substituents selected from the group consisting of linear or branched alkyl having 1 to 6 carbon atoms, linear or branched alkoxy having 1 to 6 carbon atoms, nitro, and halogen on the phenyl ring; and linear or branched alkanesulfonyloxy having 1 to 6 carbon atoms substituted with naphthyl. Specific examples of the "phenyl-substituted alkanesulfonyloxy" include benzylsulfonyloxy, 2-phenylethylsulfonyloxy, 4-phenylbutylsulfonyloxy, 4-methylbenzylsulfonyloxy, 2-methylbenzylsulfonyloxy, 4-nitrobenzylsulfonyloxy, 4-methoxybenzylsulfonyloxy, and 3-chlorobenzylsulfonyloxy. Specific examples of the "alkanesulfonyloxy substituted with naphthyl" include α-naphthylmethylsulfonyloxy and β-naphthylmethylsulfonyloxy.

Specific examples of the "perharoalkanesulfonyloxy" include perfluoroalkanesulfonyloxy such as trifluoromethanesulfonyloxy.

Specific examples of the "sulfonio" include dimethylsulfonio, diethylsulfonio, dipropylsulfonio, di(2-cyanoethyl)sulfonio, di(2-nitroethyl)sulfonio, di(aminoethyl)sulfonio, di(2-methylaminoethyl)sulfonio, di(2-dimethylaminoethyl)sulfonio, di(2-hydroxyethyl)sulfonio, di(3-hydroxypropyl)sulfonio, di(2-methoxyethyl)sulfonio, di(2-carbamoylethyl)sulfonio, di(2-carboxylethyl)sulfonio, di(2-methoxycarbonylethyl)sulfonio, and diphenylsulfonio.

In the present specification, the "solvent" includes solvents that are inert to the reaction. Examples include water, ethers (e.g., dioxane, tetrahydrofuran, diethyl ether, 1,2-dimethoxyethane, diethylene glycol dimethyl ether, ethylene glycol dimethyl ether), halogenated hydrocarbons (e.g., methylene chloride, chloroform, 1,2-dichloroethane, carbon tetrachloride), aromatic hydrocarbons (e.g., benzene, toluene, xylene), C₁₋₆ alcohols (e.g., methanol, ethanol, isopropanol), and polar solvents (e.g., N,N-dimethylformamide (DMF), N-methylpyrrolidone (NMP), dimethyl sulfoxide (DMSO), hexamethylphosphoric triamide, acetonitrile). These solvents are used alone or as a mixture of two or more. The reaction can also be carried out without a solvent.

In the present specification, the "buffer solution" is not particularly limited as long as it is a solution with a buffering action that can maintain the pH within a predetermined range. Examples include phosphate buffer solutions and borate buffer solutions. The buffer solution may contain a chelating agent. Examples of chelating agents include ethylenediaminetetraacetic acid (EDTA) and glycol ether diamine tetraacetic acid (EGTA).

The substituents in the compound represented by formula [ML-I] of the present invention (hereinafter referred to as "the compound [ML-I] of the present invention") are explained below.

In the compound [ML-I] of the present invention, X is a leaving group and is not particularly limited as long as it is a group capable of being detached and bound to, for example, antibodies. X is preferably a halogen, alkanesulfonyloxy, or arylsulfonyloxy, more preferably halogen, still more preferably chlorine, bromine or iodine, even more preferably chlorine, bromine, or iodine, and particularly preferably chlorine.

In the compound [ML-I] of the present invention, R¹ and R² are the same or different, and each independently represents hydrogen, C₁₋₆ alkyl optionally substituted with one or more substituents, or C₁₋₆ alkylene-R¹¹ optionally substituted with one or more substituents, and preferably hydrogen, C₁₋₆ alkyl, or C₁₋₆ alkylene-R¹¹ (provided that R¹ and R² are not hydrogen at the same time), wherein R¹¹ is COOH, SO₃H, or PO₃H₂, or an ester group of COOH, SO₃H, or PO₃H₂. The ester group is preferably an alkyl ester group, more preferably a C₁₋₆ alkyl ester group, and even more preferably a C₁₋₄ alkyl ester group. R¹¹ is preferably COOH or SO₃H, and is also preferably PO₃H₂ or P(=O)(OC₂H₅)(OC₂H₅). R¹ and R² are each independently preferably hydrogen, methyl, ethyl, propyl, -(CH₂)₃-SO₃H, or -(CH₂)₅-COOH, and are also preferably - (CH₂)₃-PO₃H₂ or - (CH₂)₃-P(=O)(OC₂H₅)(OC₂H₅). More preferably, R¹ is hydrogen, methyl, propyl, - (CH₂)₃-SO₃H, - (CH₂)₃-PO₃H₂, or - (CH₂)₃-P(=O)(OC₂H₅)(OC₂H₅), and R² is - (CH₂)₅-COOH.

In the compound [ML-I] of the present invention, R¹ and R², together with nitrogen atoms adjacent thereto, may form a seven-membered ring that further contains a nitrogen atom as a ring-constituting atom (preferably triazepane, more preferably 1,2,5-triazepane). The ring has a substituent. The substituent may be -C(=O)-C₁₋₆ alkylene-R¹¹ optionally substituted with one or more substituents, preferably -C(=O)-C₁₋₆ alkylene-R¹¹, more preferably -C(=O)-C₁₋₆ alkylene-COOH, and even more preferably - C(=O)-(CH₂)₂-COOH.

The compound [ML-I] of the present invention is preferably a compound represented by formula [ML-Ia] or formula [ML-Ib].

The substituents in the drug-linker intermediate represented by formula [MCL-Iα], [MCL-I], [MCL-IIα], or [MCL-II] of the present invention (hereinafter referred to as "drug-linker intermediate [MCL-Iα], [MCL-I], [MCL-IIα], or [MCL-II] of the present invention") are explained below.

In the drug-linker intermediate [MCL-Iα], [MCL-I], [MCL-IIα], or [MCL-II] of the present invention, X is a leaving group, preferably a halogen, alkanesulfonyloxy, or arylsulfonyloxy, more preferably a halogen, even more preferably chlorine, bromine or iodine, still even more preferably chlorine or iodine, and particularly preferably chlorine.

In the drug-linker intermediate [MCL-IIα] or [MCL-II] of the present invention, R¹ is hydrogen, C₁₋₆ alkyl optionally substituted with one or more substituents, or C₁₋₆ alkylene-R¹¹ optionally substituted with one or more substituents, and is preferably hydrogen, C₁₋₆ alkyl, or C₁₋₆ alkylene-R¹¹, wherein R¹¹ is COOH, SO₃H, PO₃H₂, or an ester group of COOH, SO₃H, or PO₃H₂, preferably COOH, SO₃H, -(CH₂)₃-PO₃H₂, or -(CH₂)₃-P(=O) (OC₂H₅)(OC₂H₅), and more preferably SO₃H, -(CH₂)₃-PO₃H₂, or -(CH₂)₃-P(=O)(OC₂H₅)(OC₂H₅). Preferably, R¹ is hydrogen, methyl, propyl, -(CH₂)₃-SO₃H, -(CH₂)₃-PO₃H₂, or -(CH₂)₃-P(=O) (OC₂H₅)(OC₂H₅).

In the drug-linker intermediates [MCL-Iα], [MCL-I], [MCL-IIα], or [MCL-II] of the present invention, L¹ is 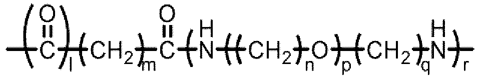 or 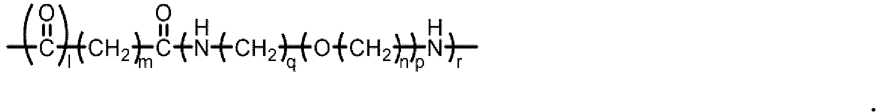 The right end of the above formulas preferably bonds to L².

In the above formula, each methylene (-CH₂-) may be substituted with one or more substituents, preferably polyethylene glycol, a saccharide (e.g., oligosaccharide), crown ether, polysarcosine, a sulfonic acid-containing group, a phosphonic acid-containing group, or an amine or ammonium salt-containing group. In one embodiment, the saccharide is a monosaccharide, a disaccharide, or a cyclodextrin, and may be substituted with one or two or more saccharides that are the same or different from each other (e.g., two disaccharides, two cyclodextrins).

l and r are each independently 0 or 1, and m, n, p, and q are each independently an integer of 1 to 6. The combination of l and r expressed by (l, r) is preferably (0, 0), (1, 0), or (0, 1). In the case of drug-linker intermediate [MCL-Iα] or [MCL-I], l is preferably 1. In the case of drug-linker intermediate [MCL-IIα] or [MCL-II], l is preferably 0. m is preferably an integer of 2 to 5. n, p, and q are preferably each independently an integer of 2 to 5. L¹ is preferably 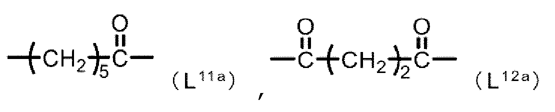 or 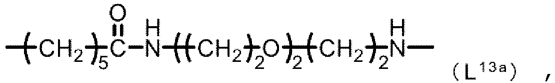 and more preferably 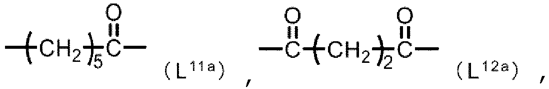 or

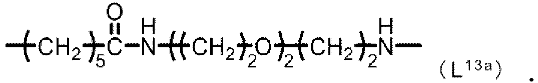

In the case of drug-linker intermediate [MCL-Iα] or [MCL-I], L¹ is preferably L¹², and more preferably L^{12a}. In the case of drug-linker intermediate [MCL-IIα] or [MCL-II], L¹ is preferably L¹¹ or L¹³, and more preferably L^{11a} or L^{13a}.

In the drug-linker intermediates [MCL-Iα], [MCL-I], [MCL-IIα], or [MCL-II] of the present invention, L² is a bond or a cleavable linker.

In the drug-linker intermediates [MCL-Iα], [MCL-I], [MCL-IIα], or [MCL-II] of the present invention, the cleavable linker is preferably a cathepsin-cleavable linker, a pyrophosphatase/phosphoesterase-cleavable linker, a glycosidase-cleavable linker (β-glucuronidase-cleavable linker), a carboxylesterase-cleavable linker, a legumain-cleavable linker, a sulfatase-cleavable linker, a neutrophil-elastase cleavable linker, a matrix metalloproteinase-cleavable linker, or a fibroblast activation protein-cleavable linker.

In the drug-linker intermediate [MCL-Iα], [MCL-I], [MCL-IIα], or [MCL-II] of the present invention, the cleavable linker is preferably a hydroxymethylbenzoic acid residue substituted with glucuronic acid, a peptide residue, a pyrophosphate (diphosphate) residue, or disulfide. The hydroxymethylbenzoic acid residue substituted with glucuronic acid is preferably a 2-(1-D-glucuronyl)-5-hydroxymethylbenzoic acid residue. Examples of the peptide residue include monopeptide residues, dipeptide residues, and polypeptide residues (for example, tripeptide residues, tetrapeptide residues), and are preferably composed of 1 or 2 to 20 amino acid residues.

The peptide residue that is one of the cleavable linkers in the drug-linker intermediate [MCL-Iα], [MCL-I], [MCL-IIα], or [MCL-II] of the present invention is preferably a dipeptide consisting of valine and citrulline (Val-Cit), a dipeptide consisting of alanine and/or D-alanine (e.g., Ala-Ala, D-Ala-D-Ala, Ala-D-Ala, D-Ala-Ala), a tripeptide consisting of glutamic acid, D-alanine and/or alanine (e.g., Glu-D-Ala-Ala, Glu-Ala-Ala), a tripeptide consisting of glutamic acid, D-alanine and/or alanine with polyethylene glycol (PEG) or a saccharide (e.g., oligosaccharide (Oligo)) added to the terminal carboxylic acid of the glutamic acid (e.g., Glu(PEG)-D-Ala-Ala, Glu(PEG)-Ala-Ala, Glu(Oligo)-D-Ala-Ala, Glu(Oligo)-Ala-Ala), or a tetrapeptide consisting of phenylalanine and three glycines (e.g., Gly-Gly-Phe-Gly). It is preferable that the left end of these linkers is bonded to L¹ and the right end of these linkers is bonded to L³ (for example, in the case of Val-Cit, the valine residue is bonded to L¹ and the citrulline residue is bonded to L³).

In one embodiment, L¹-L² in the drug-linker intermediate [MCL-I] or [MCL-II] of the present invention is preferably 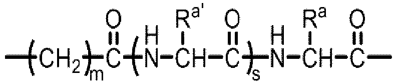 (wherein
R^{a} and R^{a'} each independently represent a side chain of an amino acid;
s is an integer of 1 to 5; and
m is as defined above).

In the drug-linker intermediates [MCL-Iα], [MCL-I], [MCL-IIα], or [MCL-II] of the present invention, L³ is a bond, amino(aryl or heteroaryl)methyloxycarbonyl represented by 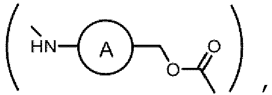 amino(aryl or heteroaryl)methyl represented by 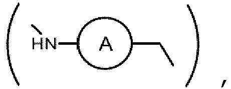 aminomethyl represented by 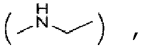 or oxymethyl represented by 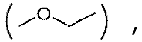 wherein A is an aryl or heteroaryl optionally substituted with one or more (for example, 1 or 2) substituents, and is preferably phenyl. The substituent is preferably polyethylene glycol, a saccharide, or a group having polyethylene glycol or a saccharide at the end. L³ is preferably a bond, p-aminobenzyloxycarbonyl, p-aminobenzyl, aminomethyl, or oxymethyl, and more preferably p-aminobenzyloxycarbonyl. The left end of these linkers is preferably bonded to L².

In the drug-linker intermediate [MCL-Iα], [MCL-I], [MCL-IIα], or [MCL-II] of the present invention, LG is preferably a leaving group. LG is preferably a halogen or an O-electron-withdrawing group, and these can be leaving groups. LG is preferably halogen, acyloxy optionally substituted with one or more substituents, or aryloxy optionally substituted with one or more substituents, more preferably a halogen, acyloxy, or aryloxy, and even more preferably chlorine, acetoxy, or p-nitrophenyloxy.

In the drug-linker intermediate [MCL-Iα] or [MCL-IIα] of the present invention, L is not particularly limited as long as it comprises L¹, L², and L³. L can be a structure where L¹, L², and L³ are connected in any order in a linear or branched chain (for example, L¹-L²-L³). In the drug-linker intermediate [MCL-I] or [MCL-II] of the present invention, L¹-L²-L³ is preferably 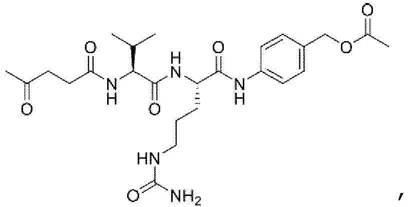 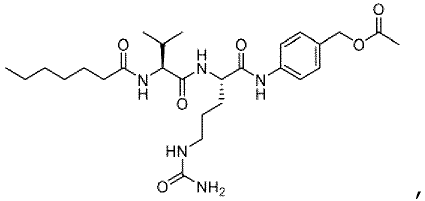 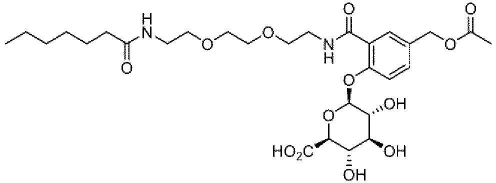 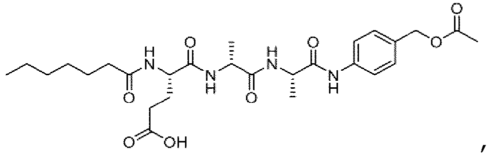 or

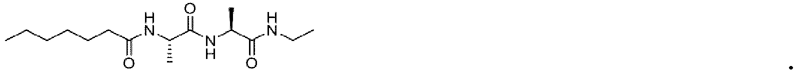

The drug-linker intermediate [MCL-Iα], [MCL-I], [MCL-IIα], or [MCL-II] of the present invention is preferably a drug-linker intermediate represented by formula [MCL-Ia], formula [MCL-IIa], formula [MCL-IIb], or formula [MCL-IIc].

The substituents in the drug-linker represented by formula [ADL-Iα], [ADL-I], [ADL-IIα], or [ADL-II] of the present invention (referred to below as "drug-linker [ADL-Iα], [ADL-I], [ADL-IIα], or [ADL-II] of the present invention") are explained below.

In the drug-linker [ADL-Iα], [ADL-I], [ADL-IIα], or [ADL-II] of the present invention, examples of groups represented by X, R¹, L¹, L², and L³ are the same as those mentioned in the drug-linker intermediate [MCL-Iα], [MCL-I], [MCL-IIα], or [MCL-II] of the present invention, and examples of preferred groups are also the same.

The drug denoted as Payload in the drug-linker [ADL-Iα], [ADL-I], [ADL-IIα], or [ADL-II] of the present invention is not particularly limited as long as it is a drug that can be loaded on the antibody-drug conjugate and that is released in the target tissue and capable of exerting its pharmacological effects in the target tissue when the cleavable linker is cleaved by an enzyme *in vivo* at the target tissue. The drug can be, for example, a low molecular weight compound or a medium molecular weight compound, and has a molecular weight of, for example, about 15000 or less, about 6000 or less, or about 2000 or less. Low molecular weight compounds are usually compounds with a molecular weight of about 500 or less. Medium molecular weight compounds include, for example, compounds (e.g., natural products) with a molecular weight of about 500 to about 2000, compounds (e.g., peptides) with a molecular weight of about 600 to about 6000, and compounds (e.g., siRNA, antisense nucleic acids) with a molecular weight of about 3000 to 15000. Examples of the drug include anticancer drugs, cytocidal agents, immunomodulators, and toxins.

In the drug-linker [ADL-Iα], [ADL-I], [ADL-IIα] or [ADL-II] of the present invention, examples of the drug denoted as Payload include the following drugs (1) to (3).

### (1) Anticancer drugs or cytocidal agents

Monomethyl auristatin E, monomethyl auristatin F, dolastatin, maytansinoid (e.g., maytansine or ansamitocin), camptothecin (e.g., synthetic analogue topotecan, topotecan, irinotecan, and RFS2000), cryptophycin (e.g., cryptophycin 1 or cryptophycin 8), duocarmycin (e.g., synthetic analogues, KW-2189, CC-1065, carzelesin, bizelesin, and CB1-TM1), antibiotics (e.g., enediyne antibiotics, such as calicheamicin selected from calicheamicin γ1I and calicheamicin ωI1, or dynemicin such as dynemicin A), epothilone, rhizoxin, taxoids (e.g., paclitaxel, docetaxel), platinum analogues (e.g., cisplatin or carboplatin), doxorubicin and derivatives thereof (e.g., morpholinodoxorubicin, cyanomorpholinodoxorubicin, 2-pyrrolino-doxorubicin, pirarubicin, daunorubicin, or deoxydoxorubicin), venetoclax, erlotinib, bortezomib, sutent, imatinib mesylate, lapatinib, lonafarnib, sorafenib, gefitinib, bisphosphonates (e.g., clodronate, ibandronate), neocarzinostatin chromophore, or related chromoproteins, enediyne antibiotic chromophores, dactinomycin, vinblastine, vincristine, vinorelbine, bryostatin, etoposide, teniposide, rapamycin, deofoamine, antimetabolites (e.g., 5-fluorouracil (5-FU), capecitabine, gemcitabine, fludarabine, 6-mercaptopurine, thiamiprine, thioguanine, ancitabine, azacitidine, 6-azauridine, carmofur, cytarabine, dideoxy-uridine, doxifluridine, enocitabine, or floxuridine), folic acid analogues (e.g., denopterin, methotrexate, edatrexate, pteropterin, aminopterin, or trimetrexate), androgens (e.g., calusterone, dromostanolone propionate, epitiostanol, or testolactone), anti-adrenal drugs (e.g., aminoglutethimide).

### (2) Immunomodulators

TLR7 agonists (e.g., imiquimod, resiquimod, etc.), TLR8 agonists (e.g., VTX-2337 etc.), TLR9 agonists (e.g., ODN2216, ODN1826, ODN2006, ODN2395, ODN21798, etc.), STING agonists (e.g., cGAMP, diABZI, SR-717, etc.), steroids (e.g., budesonide, dexamethasone, budenoside, etc.), or PDE4 inhibitors (e.g., GSK256066, etc.), and LCK inhibitors (e.g., dasatinib, etc.)

### (3) Toxins

Diphtheria toxin, botulinum toxin, tetanus toxin, dysentery toxin, cholera toxin, tetrodotoxin, brevetoxin, shiga toxin, ricin, or AM-toxin.

The drug-linker [ADL-Iα], [ADL-I], [ADL-IIα], or [ADL-II] of the present invention is preferably a drug-linker represented by formula [ADL-Ia], formula [ADL-IIa], formula [ADL-IIb], or formula [ADL-IIc].

The substituents in the antibody-drug conjugate represented by formula [ADC-Iα], [ADC-I], [ADC-IIα], or [ADC-II] of the present invention (hereinafter referred to as "the antibody-drug conjugate [ADC-Iα], [ADC-I], [ADC-IIα], or [ADC-II] of the present invention") are explained below.

Examples of R¹, L¹, L², L³, and Payload in the antibody-drug conjugate [ADC-Iα], [ADC-I], [ADC-IIα], or [ADC-II] of the present invention are the same as those in the drug-linker [ADL-Iα], [ADL-I], [ADL-IIα], or [ADL-II] of the present invention, and preferable examples of groups are also the same.

In the antibody-drug conjugate [ADC-Iα], [ADC-I], [ADC-IIα], or [ADC-II] of the present invention, S is a sulfur atom of a cysteine residue in the antibody moiety.

In the antibody-drug conjugate [ADC-Iα], [ADC-I], [ADC-IIα], or [ADC-II] of the present invention, the antibody denoted as Antibody is preferably IgG, IgM, IgA, or IgD, and more preferably IgG (in particular, IgG1 or IgG4).

In the antibody-drug conjugate [ADC-Iα], [ADC-I], [ADC-IIα], or [ADC-II] of the present invention, the antibody denoted as Antibody can be, for example, rituximab, trastuzumab, nivolumab, pembrolizumab, ipilimumab, mirikizumab, dupilumab, bevacizumab, infliximab, adalimumab, abciximab, daclizumab, palivizumab, etanercept, basiliximab, gemtuzumab, alemtuzumab, ibritumomab, alefacept, omalizumab, efalizumab, tositumomab, cetuximab, natalizumab, ranibizumab, panitumumab, eculizumab, rilonacept, certolizumab, romiplostim, belimumab, tocilizumab, atrizumab, milatuzumab, mepolizumab, pertuzumab, tremelimumab, inotuzumab, aflibercept, catumaxomab, oregovomab, motavizumab, efungumab, or raxibacumab.

The antibody-drug conjugate [ADC-Iα], [ADC-I], [ADC-IIα], or [ADC-II] of the present invention is preferably an antibody-drug conjugate represented by formula [ADC-Ia], formula [ADC-IIa], formula [ADC-IIb], or formula [ADC-IIc].

In the present specification, presentation of preferred embodiments and options regarding different features of the compound, method, and composition of the present invention also includes presentation of combinations of preferred embodiments and options regarding the different features, as long as they are combinable and not contradictory.

The method for producing compound [ML-I] of the present invention is described below. Compound [ML-I] of the present invention can be produced, for example, by any of the production methods shown below. The production methods shown below are merely examples, and the method for producing compound [ML-I] is not limited to these.

In the following reaction schemes, when performing an alkylation reaction, a hydrolysis reaction, an amination reaction, an esterification reaction, an amidation reaction, an etherification reaction, a nucleophilic substitution reaction, an addition reaction, an oxidation reaction, a reduction reaction, etc., these reactions can be performed according to known methods. Examples of such methods include those described in, for example, Experimental Chemistry Course (5th ed., edited by the Chemical Society of Japan, Maruzen Co., Ltd.); Organic Functional Group Preparations, 2nd ed., Academic Press, Inc., published in 1989; Comprehensive Organic Transformations, VCH Publishers Inc., published in 1989; and P. G. M. Wuts and T. W. Greene, "Greene's Protective Groups in Organic Synthesis" (4th ed., 2006).

### General Synthetic Route (1) for Compound [ML-I]

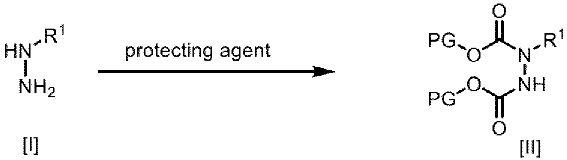

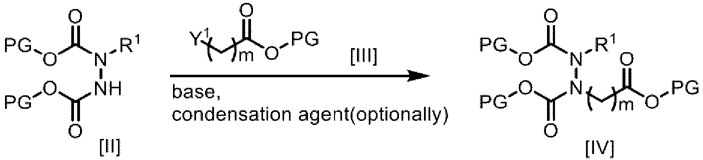

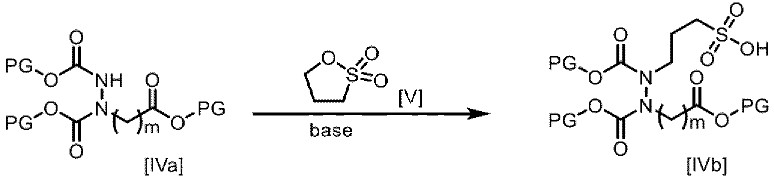

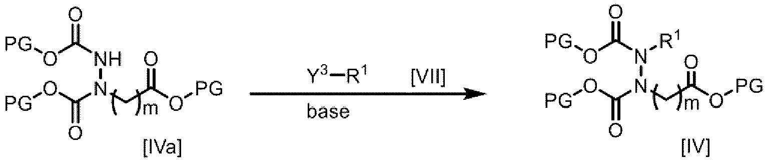

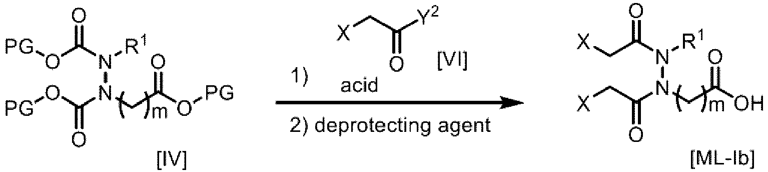

wherein Y¹, Y², and Y³ are leaving groups; PG is a protecting group; and other symbols are as defined above.

### Step 1: Production of Compound [II]

Intermediate compound [II] of compound [ML-Ib], which is included in compound [ML-I] of the present invention, can be produced by the reaction shown above. Specifically, intermediate compound [II] can be produced by introducing a protecting agent into compound [I] in a solvent inert to the reaction. As intermediate compound [II], a commercially available product may be used.

### Step 2: Production of Compound [IV]

Intermediate compound [IV] of compound [ML-Ib], which is included in compound [ML-I] of the present invention, can be produced by the reaction shown above. Specifically, intermediate compound [IV] can be produced by subjecting compound [II] to a condensation reaction with compound [III] in a solvent inert to the reaction, in the presence of a base and a condensing agent (optional).

### Step 3: Production of Compound [IVb]

Compound [IVb], which is included in intermediate compound [IV] of compound [ML-Ib], which is included in compound [ML-I] of the present invention, can be produced by the reaction shown above. Specifically, compound [IVb] can be produced by reacting compound [IVa] with compound [V] in a solvent inert to the reaction, in the presence of a base.

### Step 3b: Production 2 of Compound [IV]

Intermediate compound [IV] of compound [ML-Ib], which is included in compound [ML-I] of the present invention, can be produced by the reaction shown above. Specifically, intermediate compound [IV] can be produced by reacting compound [IVa] with compound [VII] in a solvent inert to the reaction, in the presence of a base.

### Step 4: Production of Compound [ML-Ib]

Compound [ML-Ib], which is included in compound [ML-I] of the present invention, can be produced by the reaction shown above. Specifically, compound [ML-Ib] can be produced by subjecting compound [IV] to a substitution reaction with compound [VI] in a solvent inert to the reaction, in the presence of an acid. In this reaction, a deprotection reaction proceeds simultaneously. When the deprotection reaction does not proceed or proceeds only partially, the deprotection reaction can be completed by using a deprotecting agent.

### General Synthetic Route (2) for Compound [ML-I]

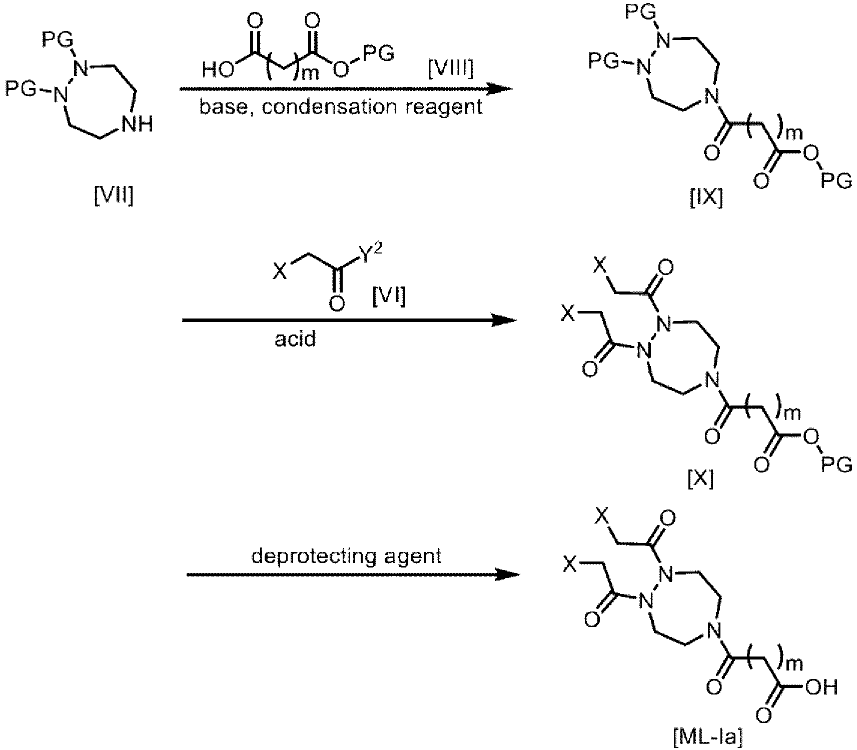

wherein all symbols are as defined above.

### Step 1: Production of Compound [IX]

Intermediate compound [IX] of compound [ML-Ia], which is included in compound [ML-I] of the present invention, can be produced by the reaction shown above. Specifically, intermediate compound [IX] can be produced by subjecting compound [I] to a condensation reaction with compound [II] in a solvent inert to the reaction, in the presence of a base and a condensing agent.

### Step 2: Production of Compound [X]

Intermediate compound [X] of compound [ML-Ia], which is included in compound [ML-I] of the present invention, can be produced by the reaction shown above. Specifically, intermediate compound [X] can be produced by subjecting compound [IX] to a substitution reaction with compound [VI] in a solvent inert to the reaction, in the presence of an acid.

### Step 3: Production of Compound [ML-Ia]

Compound [ML-Ia], which is included in compound [ML-I] of the present invention, can be produced by the reaction shown above. Specifically, compound [ML-Ia] can be produced by subjecting compound [X] to a deprotection reaction using a deprotecting agent in a solvent inert to the reaction.

In each of the reactions of the reaction schemes described above, the product in the form of a reaction mixture or of a crude product may be used in the subsequent reaction, or the product may be isolated from the reaction mixture according to an ordinary method and easily purified by a common separation method. Examples of common separation methods include recrystallization, distillation, and chromatography.

Compound [ML-I] of the present invention can be produced by the synthesis method shown in each of the above reaction schemes, or by a method equivalent thereto.

The raw material compounds in the production of compound [ML-I] of the present invention may be commercially available or produced according to known methods or equivalent methods, unless a specific production method is described.

The starting raw material compound and desired compound in each of the above steps can be used in appropriate salt forms. Such salts include the same as those mentioned below as examples of salts of compound [I] of the present invention.

Compound [ML-I] of the present invention includes its salt (preferably pharmaceutically acceptable salt) form, and may form acid addition salts and salts with bases depending on the type of substituent. Examples of such acids include inorganic acids (e.g., hydrochloric acid, hydrobromic acid, nitric acid, sulfuric acid, and phosphoric acid); organic acids (e.g., methanesulfonic acid, p-toluenesulfonic acid, acetic acid, trifluoroacetic acid, citric acid, tartaric acid, maleic acid, fumaric acid, malic acid, and lactic acid); and the like. Examples of such bases include inorganic bases (e.g., sodium hydroxide, potassium hydroxide, calcium hydroxide, sodium carbonate, potassium carbonate, sodium hydrogencarbonate, and potassium hydrogencarbonate); organic bases (e.g., methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, and choline); ammonium salts; and the like. Compound [ML-I] of the present invention may also form salts with amino acids, such as lysine, arginine, aspartic acid, and glutamic acid.

Compound [ML-I] of the present invention also includes hydrates, solvates, and crystal polymorphs thereof, and hydrates, solvates, and crystal polymorphs of its salts.

Compound [ML-I] of the present invention includes compounds in which one or more atoms are replaced by one or more isotopic atoms. Examples of isotopic atoms include deuterium (²H), tritium (³H), ¹³C, ¹⁵N, ¹⁸O, and the like.

Compound [ML-I] of the present invention or a salt thereof may be a co-crystal or a co-crystal salt. Co-crystals or co-crystal salts refer to crystalline substances composed of two or more unique solids at room temperature, each having different physical properties (e.g., structure, melting point, and heat of fusion). Co-crystals and co-crystal salts can be produced by applying known co-crystallization methods.

### General Production Method (1) for Drug-linker (ADL-II)

An example of the method for producing drug-linker [ADL-IId] in which the cleavable linker is a peptide residue is described below. 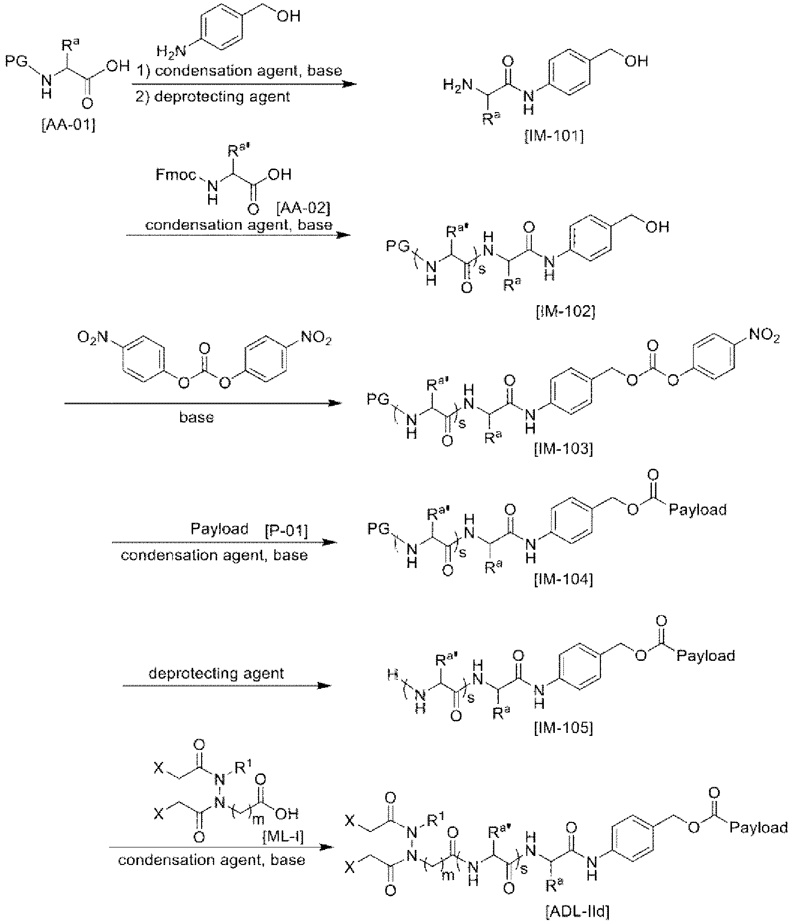 wherein all symbols are as defined above.

### Step 1: Production of Compound IM-101

Compound IM-101 can be synthesized by subjecting p-benzyl alcohol and a desired amino acid (AA-01) with a protecting group introduced at the amino group to a condensation reaction in a solvent inert to the reaction, in the presence of a condensing agent and a base, and then performing a deprotection reaction.

### Step 2: Production of Compound IM-102

Compound IM-102 can be synthesized by subjecting compound IM-101 and a desired amino acid (AA-02) with a protecting group introduced at the amino group to a condensation reaction in a solvent inert to the reaction, in the presence of a condensing agent and a base.

By repeating this reaction and a deprotection reaction, compound IM-102 having the desired number of amino acid residues can be produced.

### Step 3: Production of Compound IM-103

Compound IM-103 (a compound having L²-L³) can be produced by reacting compound IM-102 with bis(4-nitrophenyl) carbonate in a solvent inert to the reaction, in the presence of a base.

### Step 4: Production of Compound IM-104

Compound IM-104 (a compound having L²-L³-Payload) can be produced by subjecting compound IM-103 and Payload (P-01) (in particular, a drug having an unsubstituted or monosubstituted amino group) to a condensation reaction in a solvent inert to the reaction, in the presence of a condensing agent and a base.

### Step 5: Production of Compound IM-105

Compound IM-105 can be produced by subjecting compound IM-104 to a deprotection reaction in a solvent inert to the reaction, in the presence of a deprotecting agent.

### Step 6: Production of Compound ADL-IId

Compound ADL-IId (a drug-linker) can be produced by subjecting compound IM-105 and compound ML-Ib to a condensation reaction in a solvent inert to the reaction, in the presence of a condensing agent and a base.

### General Production Method (2) for Drug-linker (ADL-II)

An example of the method for producing drug-linker [ADL-IIe] in which the cleavable linker is a hydroxymethylbenzoic acid residue substituted with glucuronic acid is described below. 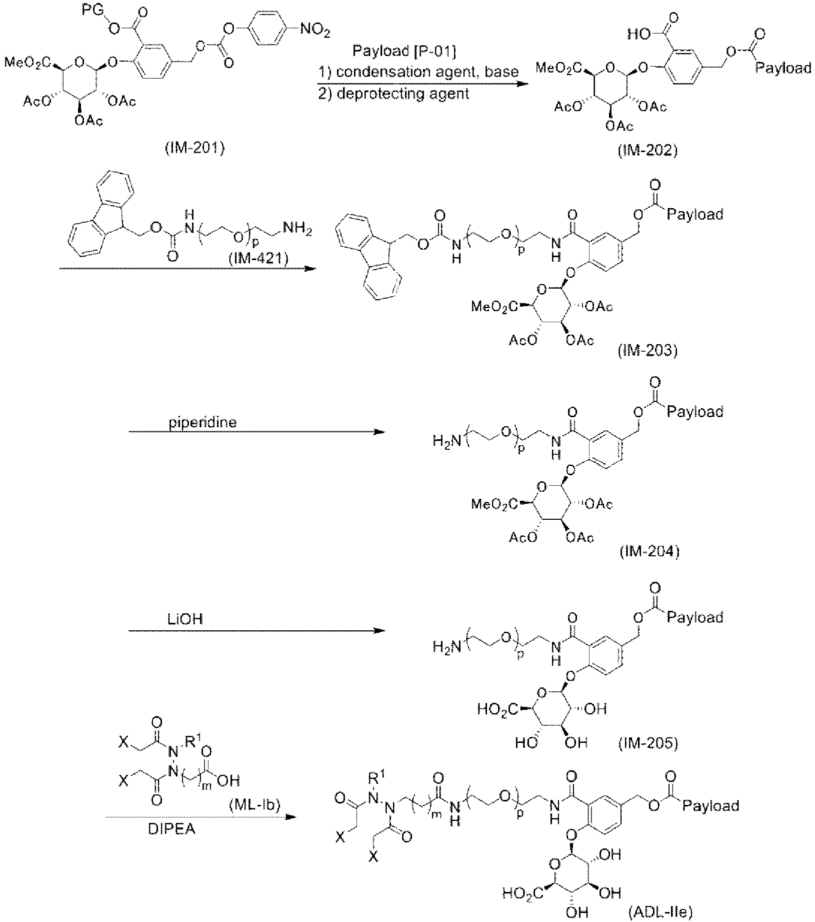 wherein all symbols are as defined above.

### Step 1: Production of Compound IM-202

Compound IM-202 (a compound having L²-L³-Payload) can be synthesized by subjecting compound IM-201 and Payload (P-01) (in particular, a drug having an unsubstituted or monosubstituted amino group) to a condensation reaction in a solvent inert to the reaction, in the presence of a condensing agent and a base, and then performing a deprotection reaction.

### Step 2: Production of Compound IM-203

Compound IM-203 can be synthesized by subjecting compound IM-202 and compound IM-421 to a condensation reaction in a solvent inert to the reaction. In this reaction, a condensing agent and a base may or may not be added.

### Step 3: Production of Compound IM-204

Compound IM-204 can be synthesized by deprotecting IM-203 in a solvent inert to the reaction, in the presence of a base.

### Step 4: Production of Compound IM-205

Compound IM-205 can be synthesized by deprotecting IM-204 in a solvent inert to the reaction, in the presence of a base.

### Step 5: Production of Compound ADL-IIe

Compound ADL-IIe (a drug-linker) can be synthesized by subjecting IM-205 and ML-Ib to a condensation reaction in a solvent inert to the reaction, in the presence of a condensing agent and a base.

### General Production Method (3) for Drug-linker (ADL-II)

An example of the method for producing drug-linker [ADL-IIg] in which L³ is aminomethyl is described below.

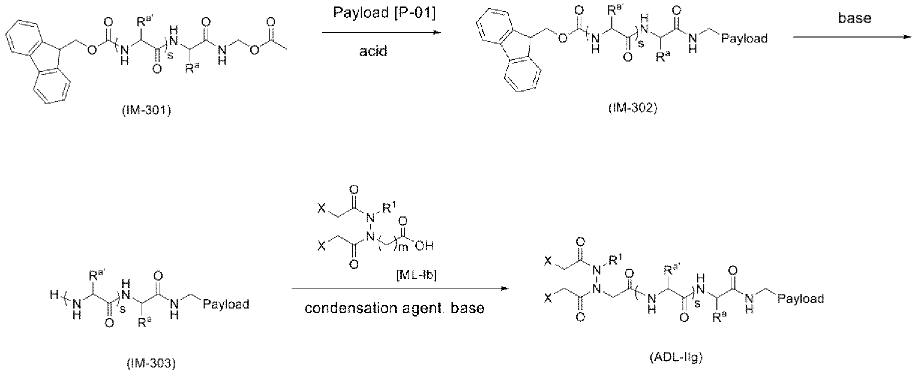

### Step 1: Production of Compound IM-302

Compound IM-302 can be synthesized by reacting compound IM-301 and Payload (P-01) (in particular, a drug having a hydroxy group) in a solvent inert to the reaction under acidic (TFA etc.) conditions.

### Step 2: Production of Compound IM-303

Compound IM-303 can be synthesized by deprotecting IM-302 in a solvent inert to the reaction, in the presence of a base.

### Step 3: Production of Compound ADL-IIg

Compound ADL-IIg (a drug-linker) can be synthesized by subjecting IM-303 and ML-Ib to a condensation reaction in a solvent inert to the reaction, in the presence of a condensing agent and a base.

### General Production Method for Antibody-drug Conjugates (ADC-I and ADC-II)

The desired antibody-drug conjugate can be formed by a method including the steps of cleaving (reducing) disulfide bonds (preferably disulfide bonds forming covalent bonds between heavy chain-light chain and between heavy chain-heavy chain) of an antibody, and introducing (crosslinking) a drug-linker (ADL-I or ADL-II) containing a payload to the reduced product (preferably its thiols) in the presence or absence of sodium iodide. In particular, when X of the drug-linker is chlorine, it is preferable to perform the process in the presence of sodium iodide. In another form, it is preferable to perform the process in the absence of sodium iodide.

Specifically, a reducing agent (TCEP) and a buffer solution (PBS, EDTA) are added to an antibody, followed by, for example, shaking or rotating at 20 to 40°C (preferably about 37°C) for 5 minutes to 5 hours (preferably about 2 hours), thereby reducing disulfide bonds.

After purifying the reduced product as necessary, a drug-linker, sodium iodide, and a borate buffer solution are added, and the mixture is, for example, shaked or rotated at 20°C for 1 hour to 10 days, whereby an antibody-drug conjugate can be formed.

The amount of sodium iodide added is, for example, 0 to 100000 molar equivalents (preferably 10 to 60000 molar equivalents, more preferably 50 to 50000 molar equivalents, and even more preferably 100 to 40000 molar equivalents) relative to the antibody. The reaction time is, for example, 1 hour to 10 days, preferably 0.5 to 7 days. Although the reaction proceeds even without the addition of sodium iodide, the reaction proceeds rapidly when the amount of sodium iodide added is increased. However, the number of drug-linkers attached to the antibody (DAR) also tends to increase.

The antibody-drug conjugate containing the compound of the present invention as part of its structure can be selected from a variety of forms for systemic or local administration, depending on the therapeutic purpose. Typical examples include injections, transmucosal agents, suppositories, transnasal agents, transpulmonary agents, transdermal agents, and the like. Examples of injections include liquids, suspensions, emulsions, and the like. The antibody-drug conjugate may also be formulated together with a pharmaceutically acceptable excipient and/or carrier into solid or powder form so that it can be dissolved, suspended, or emulsified by the addition of a solvent at the time of use. Injections can be administered, for example, intravenously, intramuscularly, subcutaneously, or intradermally.

Examples of pharmaceutically acceptable excipients and/or carriers include stabilizers, solubilizing agents, suspending agents, emulsifiers, surfactants, soothing agents, buffers, preservatives, pH adjusters, antioxidants, and the like. These may be used singly or in a combination of two or more.

Examples of stabilizers include various amino acids, albumin, globulin, gelatin, mannitol, glucose, dextran, ethylene glycol, propylene glycol, polyethylene glycol, ascorbic acid, sodium hydrogen sulfite, sodium thiosulfate, sodium edetate, sodium citrate, dibutylhydroxytoluene, and the like.

Examples of solubilizing agents include alcohols (e.g., ethanol), polyalcohols (e.g., propylene glycol and polyethylene glycol), nonionic surfactants (e.g., polysorbate), and the like.

Examples of suspending agents include glycerol monostearate, aluminum monostearate, methylcellulose, carboxymethyl cellulose, hydroxymethyl cellulose, sodium lauryl sulfate, and the like.

Examples of emulsifiers include gum arabic, sodium alginate, tragacanth, and the like.

Examples of soothing agents include benzyl alcohol, chlorobutanol, sorbitol, and the like.

Examples of buffers include phosphate buffer solutions, acetate buffer solutions, borate buffer solutions, carbonate buffer solutions, citrate buffer solutions, Tris buffer solutions, glutamate buffer solutions, epsilon-aminocaproic acid buffer solutions, and the like.

Examples of preservatives include methyl parahydroxybenzoate, ethyl parahydroxybenzoate, propyl parahydroxybenzoate, butyl parahydroxybenzoate, chlorobutanol, benzyl alcohol, benzalkonium chloride, sodium dehydroacetate, sodium edetate, boric acid, borax, and the like.

Examples of pH adjusters include hydrochloric acid, sodium hydroxide, phosphoric acid, acetic acid, and the like.

Examples of antioxidants include water-soluble antioxidants, such as ascorbic acid, cysteine hydrochloride, sodium bisulfate, sodium metabisulfite, and sodium sulfite; oil-soluble antioxidants, such as ascorbyl palmitate, butylated hydroxyanisole, butylated hydroxytoluene, lecithin, propyl gallate, and α-tocopherol; metal chelating agents, such as citric acid, ethylenediaminetetraacetic acid, sorbitol, tartaric acid, and phosphoric acid; and the like.

When the antibody-drug conjugate is prepared in the form of an injection, the liquid, emulsion, or suspension is sterilized and preferably isotonic to blood. Diluents for use in forming these liquids, emulsions, or suspensions can be selected from a wide range of known diluents, such as water, ethanol, propylene glycol, ethoxylated isostearyl alcohol, polyoxylated isostearyl alcohol, and polyoxyethylene sorbitan fatty acid esters. In this case, the pharmaceutical formulation may contain a salt, glucose, or glycerol in an amount sufficient to prepare an isotonic solution, as well as a commonly used solubilizing agent, buffer, soothing agent, etc., and may further contain, if necessary, a coloring agent, a preservative, a fragrance, and/or other pharmaceutical products.

The route of administration of the antibody-drug conjugate is not particularly limited, and the antibody-drug conjugate is administered by a route suitable for the form of the formulation, the subject's or patient's (in particular, a human) age and sex, conditions of the disease, and other conditions. For example, injections may be administered intravenously singly or as mixed with usual replacement fluids, such as glucose and amino acids; or singly administered intramuscularly, intradermally, subcutaneously, or intraperitoneally, as required.

The dosage of the antibody-drug conjugate is suitably selected according to the method of use, the subject's or patient's (in particular, a human) age and sex, severity of the disease, and other conditions, and is usually about 0.001 to 100 mg/kg body weight/day, and preferably about 0.001 to 50 mg/kg body weight/day, in single or divided doses (e.g., two or three doses) .

Since the above dosage varies according to various conditions, there may be cases in which a dosage smaller than the above range is sufficient or where a dosage larger than the above range is required.

The disclosures of all patent and non-patent documents cited in the present specification are incorporated herein by reference in their entirety.

### Examples

The present invention is further described below in detail with reference to Test Examples, Reference Examples, and Examples; however, these do not limit the present invention, and modification may be made without departing from the scope of the present invention.

In the present specification, the following abbreviations may be used.

| Abbreviation | Term |
|---|---|
| REx | Reference Example number |
| Ex | Example number |
| CEx | Comparative Example number |
| STR | Structural formula (in the formula, the structure indicated by "chiral" indicates the absolute configuration) |
| RProp | Production method (the number indicates that the compound was produced using corresponding raw materials, as in the Reference Example compound having that number as the Reference Example number) |
| Prop | Production method (the number indicates that the compound was produced using corresponding raw materials, as in the Example compound having that number as the Example number) |
| Data | Physical property data (NMR1: δ (ppm) in ¹H-NMR in DMSO-d₆; NMR2: δ (ppm) in ¹H-NMR in CDCl₃; NMR3: δ (ppm) in ¹H-NMR in CD₃OD; MS: mass spectrum) |
| AcOEt | Ethyl acetate |
| t-BuOH | Tertiary-butanol |
| Cs₂CO₃ | Cesium carbonate |
| DCM | Dichloromethane |
| DEA | Diethylamine |
| DIPEA | N,N-Diisopropylethylamine (Hunig's base) |
| DMF | N,N-Dimethylformamide |
| DMSO | Dimethyl sulfoxide |
| DMTMM | 4-(4,6-Dimethoxy-1,3,5-triazin-2-yl)-4-methylmorpholinium chloride |
| EDTA | Ethylenediaminetetraacetic acid |
| EtOH | Ethanol |
| Fmoc | 9-Fluorenylmethyloxycarbonyl |
| HATU | 1-[Bis(dimethylamino)methylene]-1H-1,2,3-triazolo[4,5-b]pyridinium 3-oxide hexafluorophosphate |
| HCl | Hydrochloric acid |
| Hexane | Hexane |
| H₂O | Water |
| HOBt | 1-Hydroxybenzotriazole |
| MeCN | Acetonitrile |
| MESNa | Sodium mercaptoethanesulfonate |
| MMAE | Monomethyl auristatin E |
| NaHCO₃ | Sodium hydrogencarbonate |
| Nal | Sodium iodide |
| PBS | Phosphate-buffered saline |
| Pd/C | Palladium on carbon |
| TCEP | Tris(2-carboxyethyl)phosphine |
| TFA | Trifluoroacetic acid |
| THF | Tetrahydrofuran |

The term "room temperature" in the following Examples generally refers to about 10°C to about 35°C. Ratios shown for mixed solvents indicate volume ratios unless otherwise specified. % indicates mass% unless otherwise specified.

¹HNMR (proton nuclear magnetic resonance spectrum) was measured by Fourier transform NMR (Bruker AVANCE III 400 (400 MHz) or Bruker AVANCE III HD (500 MHz)).

MS (mass spectrum) was measured by LC/MS (ACQUITY UPLC H-Class/Waters Xevo G2-XS QTof). As the ionization method, the ESI method was used. The data indicates actual measured values (found). Molecular ion peaks ([M+H]⁺, [M-H]⁻, etc.) are generally observed. In the case of a salt, a molecular ion peak or fragment ion peak of free form is generally observed.

Reverse-phase column chromatography was performed using a flash automatic purification apparatus (Biotage (registered trademark) Isolera One or Biotage (registered trademark) Selekt) or a high-performance liquid chromatography purification apparatus (Waters (registered trademark) AutoPurification System with 2767 Sample manager and 2489 UV/Vis Detector). In the flash automatic purification, an ODS column (Biotage (registered trademark) Sfar Bio C18) was used. In the high-performance liquid chromatography purification, an ODS column (YMC-Actus Triart C18, 250 × 20 mm I.D.) was used.

In silica gel column chromatography, when denoted as basic, aminopropylsilane-bonded silica gel was used.

The absolute configuration of the compound was determined by a known X-ray crystal structure analysis method (e.g., Shigeru Oba and Shigenobu Yano (1999). Kagakusha notameno Kisokoza 12, Xsen Kessho Kozo Kaiseki [Basic Course for Chemists 12, X-ray Crystal Structure Analysis]. 1st ed.) or inferred from the empirical rule of Shi asymmetric epoxidation (Waldemar Adam, Rainer T. Fell, Chantu R. Saha-Moller and Cong-Gui Zhao, Tetrahedron: Asymmetry 1998, 9, 397-401; and Yuanming Zhu, Yong Tu, Hongwu Yu, Yian Shi, Tetrahedron Lett. 1988, 29, 2437-2440).

### Reference Examples

### Reference Example 1: Production of Di-tert-butyl 5-(4-(benzyloxy)-4-oxobutanoyl)-1,2,5-triazepane-1,2-dicarboxylate

DIPEA (0.217 mL) and HATU (208 mg) were added to a mixture of di-tert-butyl 1,2,5-triazepane-1,2-dicarboxylate (150 mg), 3-(benzyloxycarbonyl)propanoic acid (114 mg), and DCM (3 mL), and the mixture was stirred at room temperature for 2 hours. After the reaction mixture was concentrated, the residue was purified by silica gel column chromatography (hexane/AcOEt), thereby obtaining the target product (227 mg).

### Reference Example 2: Production of Benzyl 4-(1,2-bis(2-chloroacetyl)-1,2,5-triazepan-5-yl)-4-oxobutanoate

Chloroacetyl chloride (0.368 mL) and 4 M HCl/AcOEt (2 mL) were added to a suspension of di-tert-butyl 5-(4-(benzyloxy)-4-oxobutanoyl)-1,2,5-triazepane-1,2-dicarboxylate (227 mg) in toluene (3 mL), and the mixture was stirred at 80°C overnight. After the reaction mixture was concentrated, purification was performed by medium-pressure silica gel column chromatography (hexane/AcOEt) and by using a flash automatic purification apparatus (H₂O/MeCN (0.1% TFA), Sfar Bio C18), followed by freeze-drying, thereby obtaining the target product (182 mg).

### Reference Example 3: Production of Di-tert-butyl 1-(6-(tert-butoxy)-6-oxohexyl)hydrazine-1,2-dicarboxylate

Cs₂CO₃ (5.79 g) was added to a solution of tert-butyl 6-bromohexanoate (2.23 g) and N'-[(tert-butoxy)carbonyl](tert-butoxy)carbohydrazide (4.12 g) in DMF (15 mL), and the mixture was stirred overnight. H₂O was added to the reaction mixture, followed by extraction with AcOEt. The organic layer was then concentrated. The residue was purified by silica gel chromatography (Hexane/AcOEt), thereby obtaining the target product (2.1 g).

### Reference Example 4: Production of Di-tert-butyl 1-methylhydrazine-1,2-dicarboxylate

Boc-anhydride (10.58 mL) was added to a solution of methylhydrazine (1.155 mL) and EtOH (15 mL), and the mixture was stirred at room temperature for 3 days. The reaction mixture was concentrated, thereby obtaining the target product (5.7 g).

### Reference Example 8: Production of 3-(2-(6-(Benzyloxy)-6-oxohexyl)-1,2-bis(tert-butoxycarbonyl)hydrazineyl)propane-1-sulfonic acid

1,3-Propane sultone (0.760 mL) and sodium hydroxide (0.346 g) were added to di-tert-butyl 1-(6-(benzyloxy)-6-oxohexyl)hydrazine-1,2-dicarboxylate (1.26 g) and t-BuOH (10 mL), and the mixture was stirred at room temperature. A saturated NH₄Cl aqueous solution and water were added thereto, followed by extraction with AcOEt. The organic layer was then concentrated. The residue was purified with a flash automatic purification apparatus (H₂O/MeCN (0.1% TFA), Sfar Bio C18), thereby obtaining the target product (102.3 mg).

### Reference Example 10: Production of Di-tert-butyl 1-(6-(tert-butoxy)-6-oxohexyl)-2-propylhydrazine-1,2-dicarboxylate

Potassium tert-butoxide (50.2 mg) was added to a solution of di-tert-butyl 1-(6-(tert-butoxy)-6-oxohexyl)hydrazine-1,2-dicarboxylate (150 mg) and 1-Iodopropane (0.073 mL) in THF (3 mL), and the mixture was stirred at room temperature for 1 hour and at 50°C for 2 hours. Purification was performed with a flash automatic purification apparatus (H₂O/MeCN (0.1% TFA), Sfar Bio C18), thereby obtaining the target product (153 mg).

Each of the compounds of Reference Examples 5 to 7, 9, and 11 was produced as in Reference Examples 1 to 4, 8, or 10. Table 1 shows the structural formulas and physicochemical data of the compounds of Reference Examples (REx) 1 to 11.

**Table 1**

| REx | Str | RProp | Data |
|---|---|---|---|
| 1 | 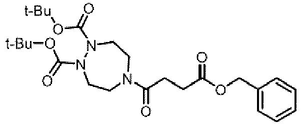 | 1 | MS m/z 492.6 (M+1). |
| 2 | 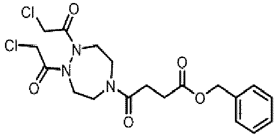 | 2 | MS m/z 444.4, 446.4 (M+1). |
| 3 | 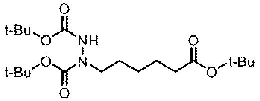 | 3 | MS m/z 425.5 (M+Na). |
| 4 | 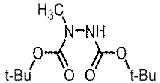 | 4 | NMR2; 6.62-6.11 (1H, m), 3.11 (3H, s), 1.50-1.43 (18H, m). |
| 5 | 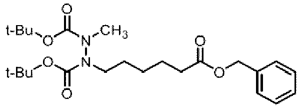 | 3 | MS m/z 473.5 (M+Na). |
| 6 | 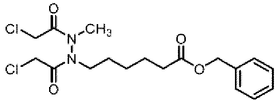 | 2 | MS m/z 403.3, 405.3 (M+1). |
| 7 | 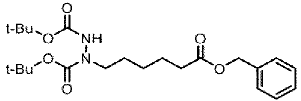 | 3 | MS m/z 459.5 (M+Na). |
| 8 | 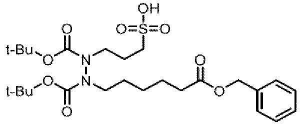 | 8 | MS m/z 581.1 (M+Na). |
| 9 | 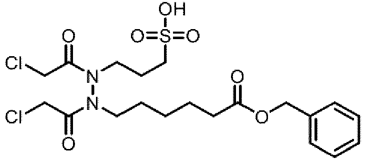 | 2 | MS m/z 511.0, 512.9 (M+1). |
| 10 | 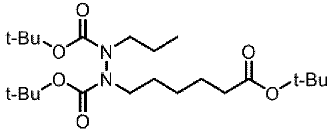 | 10 | MS m/z 445.5 (M+H). |
| 11 | 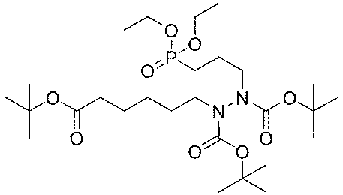 | 10 | MS m/z 581.6 (M+H). |

### Examples

### Example 1: Production of 4-(1,2-Bis(2-chloroacetyl)-1,2,5-triazepan-5-yl)-4-oxobutanoic acid

Benzyl 4-(1,2-bis(2-chloroacetyl)-1,2,5-triazepan-5-yl)-4-oxobutanoate (180 mg) was dissolved in EtOH-THF (2:1) (6 mL), and Pd/C (21.56 mg) was added. The mixture was stirred at room temperature under a hydrogen atmosphere for 2 hours. The reaction mixture was filtered through Celite, and the filtrate was concentrated. The residue was purified with a flash automatic purification apparatus (H₂O/MeCN (0.1% TFA), Sfar Bio C18), thereby obtaining the target product (136 mg).

### Example 2: Production of 6-(1,2-Bis(2-chloroacetyl)hydrazineyl)hexanoic acid

Chloroacetyl chloride (1.253 mL) and 4 M HCl in AcOEt (2 mL) were added to a solution of di-tert-butyl 1-(6-(tert-butoxy)-6-oxohexyl)hydrazine-1,2-dicarboxylate (634 mg) in toluene (7 mL), and the mixture was stirred at 80°C. Further, 4 M HCl (2 mL) was added, and the mixture was stirred at room temperature overnight. Water was added to the reaction mixture, and the reaction mixture was concentrated. The residue was purified with a flash automatic purification apparatus (H₂O/MeCN (0.1% TFA), Sfar Bio C18), thereby obtaining the target product (320 mg).

### Example 31: Production of 6-(1,2-Bis(2-bromoacetyl)-2-(3-phosphonopropyl)hydrazineyl)hexanoic acid

The compound of Example 30 (65 mg) was dissolved in MeCN (3 mL). Bromotrimethylsilane (0.539 ml) was added thereto, and the mixture was stirred at room temperature overnight. The reaction mixture was concentrated. The residue was purified with a flash automatic purification apparatus (H₂O/MeCN (0.1% TFA), Sfar Bio C18), followed by freeze-drying, thereby obtaining the target product (67 mg).

Each of the compounds of Examples 3, 4, 27, and 30 was produced as in Example 1 or 2. Table 2 shows the structural formulas and physicochemical data of the compounds of Examples (Ex) 1 to 4, 27, 30, and 31.

**Table 2**

| Ex | Str | Prop | Data |
|---|---|---|---|
| 1 | 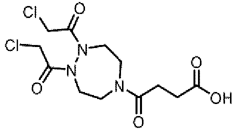 | 1 | NMR1; 4.72-3.93 (6H, m), 3.76-2.95 (6H, m), 2.67-2.37 (4H, m). |
| | | | MS m/z 354.1, 356.4 (M+1). |
| 2 | 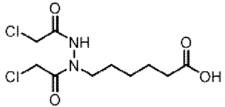 | 2 | NMR2; 8.74 (1H, s), 4.15 (2H, s), 4. 03 (2H, s), 3.75-3.52 (2H, m), 2.41 ( 2H, t, J = 8.9 Hz), 1.74-1.55 (4H, m) , 1.46-1.34 (2H, m). |
| | | | MS m/z 299.3, 301.2 (M+1). |
| 3 | 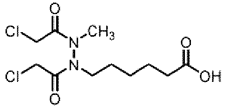 | 1 | NMR1; 4.62-4.02 (4H, m), 3.70-2.93 (5H, m), 2.27-2.16 (2H, m) , 1.70-1. 41 (4H, m) , 1.37-1.21 (2H, m). |
| | | | MS m/z 313.2, 315.2 (M+1). |
| 4 | 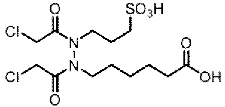 | 1 | NMR3; 4.60-3.49 (8H, m), 2.96-2.78 (2H, m), 2.39-2.01 (4H, m), 1.81-1.5 6 (4H, m), 1.50-1.30 (2H, m). |
| | | | MS m/z 420.9, 423.0 (M+1). |
| 27 | 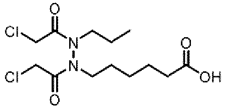 | 2 | NMR1; 4.67-4.29 (4H, m), 4.06-3.32 (4H, m), 2.26-2.14 (2H, m) , 1.71-1.19 (8H, m) , 0.95-0.78 (3H, m). |
| | | | MS m/z 341.3, 343.3 (M+1). |
| 30 | 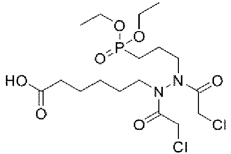 | 2 | MS m/z 477.3 (M+H). |
| 31 | 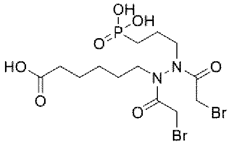 | 31 | NMR1; 5.23 (2H, brs), 4.39-3.34 (8H, m), 2.26-2.14 (2H, m) , 1.94-1.16 (10H, m) |
| | | | MS m/z 511.2, 513.2 (M+1). |

### Example 6

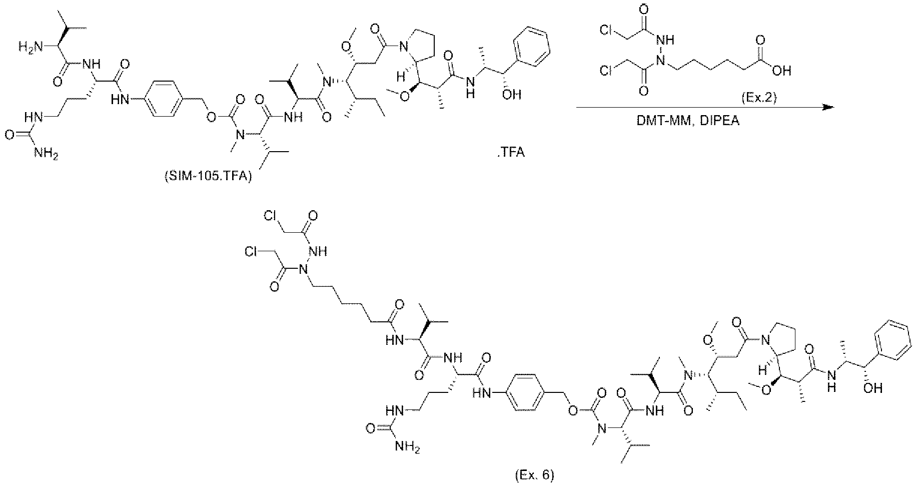

A mixture of the compound of Example 2 (2.68 mg), DIPEA (20 µL), DMTMM (2.091 mg), and DMF (450 µL) was stirred at room temperature for 5 minutes. Thereafter, a TFA salt (8.5 mg) of compound SIM-105 was added, and the mixture was stirred at room temperature for 30 minutes. The reaction mixture was concentrated, and the residue was purified by high-performance liquid chromatography (H₂O/MeCN (0.1% TFA), YMC-Actus Triart C18, 250 × 20 mm I.D.), followed by freeze-drying, thereby obtaining the target product.

### Example 9

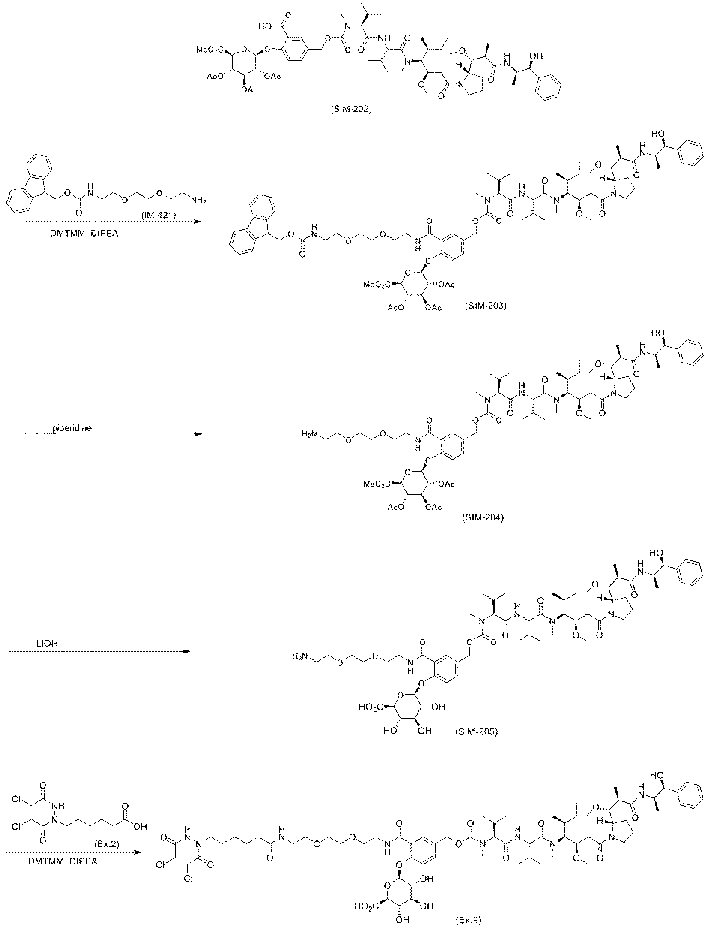

### Step 1: Production of Compound SIM-203

DMTMM (13.52 mg) and DIPEA (12.48 µL) were added to a solution of compound SIM-202 (40 mg) and compound IM-421 (20.51 mg) in DMF (2 mL), and the mixture was stirred at room temperature for 30 minutes. The reaction mixture was concentrated, and the residue was purified with a flash automatic purification apparatus (H₂O/MeCN (0.1% AcOH), Sfar Bio C18), thereby obtaining the target product (44.7 mg).

### Step 2: Production of Compound SIM-204

Piperidine (6.06 µL) was added to a solution of compound SIM-203 (44 mg) in DMSO (1 mL)-MeCN (2 mL), and the mixture was stirred at 0°C for 3 hours. Further, 3.03 µL and 2.009 µL of piperidine were added 1 and 3 hours after the start of stirring, respectively. Acetic acid (1 mL) was added to the reaction mixture, and then the mixture was concentrated. The residue was purified with a flash automatic purification apparatus (H₂O/MeCN (0.1% AcOH), Sfar Bio C18), followed by freeze-drying, thereby obtaining the target product (42.9 mg).

### Step 3: Production of Compound SIM-205

LiOH·H₂O (240 µL) and H₂O (240 µL) were added to a solution of compound SIM-204 (42 mg) in THF (2 mL) at 0°C, and the mixture was then stirred at room temperature for 1 hour. LiOH·H₂O (120 µL) and DMSO (500 µL) were added, and the mixture was further stirred for 2 hours. Acetic acid (20 µL) was added to the reaction mixture, and the mixture was concentrated. The residue was purified with a flash automatic purification apparatus (H₂O/MeCN (0.1% TFA), Sfar Bio C18), thereby obtaining the target product (34.1 mg).

### Step 4: Production of Compound of Example 9

DMTMM (3.74 mg) and DIPEA (7.84 µL) were added to a solution of the compound of Example 2 (4.04 mg) in DMF (0.5 mL), and the mixture was stirred at room temperature for 2 minutes. Further, a solution of SIM-205 (12 mg) in DMF (0.5 mL × 2) was added, and the mixture was stirred at room temperature for 15 minutes. The reaction mixture was concentrated, and the residue was purified with a flash automatic purification apparatus (H₂O/MeCN (0.1% AcOH), Sfar Bio C18), thereby obtaining the target product (11.5 mg).

### Example 11

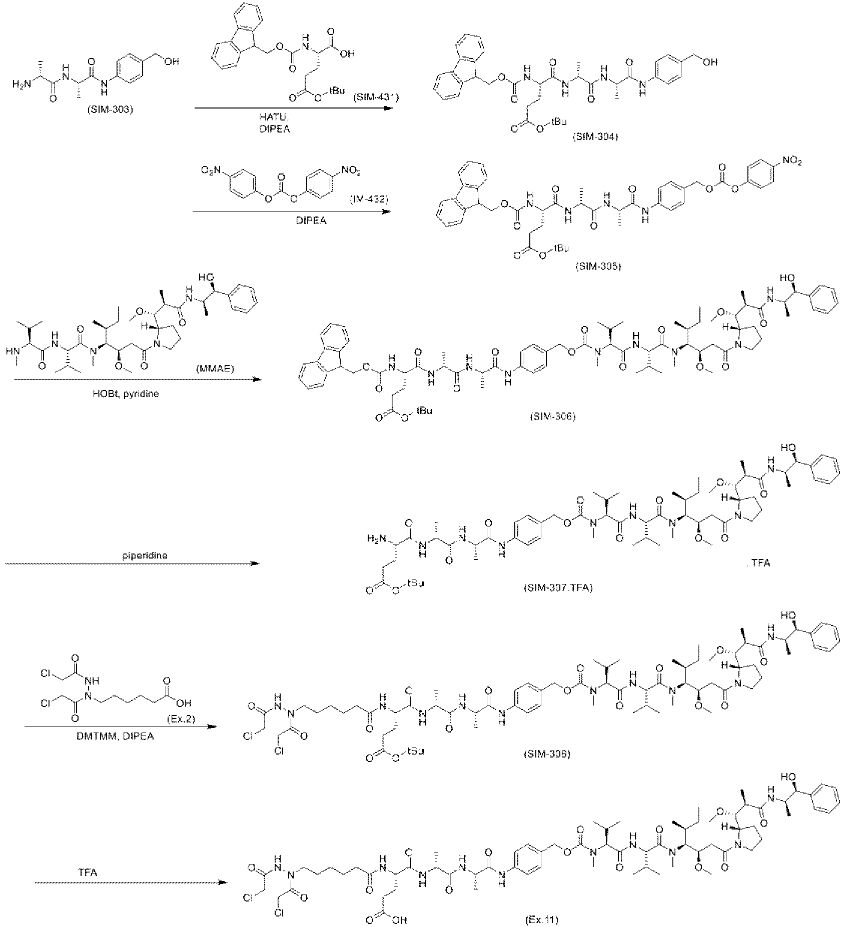

### Step 1: Production of Compound SIM-304

Compound SIM-303 (400 mg), compound SIM-431 (418 mg), HATU (491 mg), and DIPEA (429 µL) were dissolved in DMF (5 mL), and the mixture was stirred at room temperature for 38 hours. A saturated NaHCO₃ aqueous solution was added to the reaction mixture, followed by dispersing and washing. The precipitated solid was collected by filtration. The solid was dissolved in DMSO, followed by purification with a flash automatic purification apparatus (H₂O/MeCN (0.1% AcOH), Sfar Bio C18), thereby obtaining the target product (63 mg).

### Step 2: Production of Compound SIM-305

DIPEA (45.8 µL) was added to compound SIM-304 (120 mg) and bis(4-nitrophenyl) carbonate (110 mg) in THF (5 mL), and the mixture was stirred at room temperature overnight. Bis(4-nitrophenyl) carbonate (55 mg) and DIPEA (22 µL) were added, and the mixture was stirred for a total of 30 hours. The reaction mixture was concentrated and purified by silica gel chromatography (Hexane/AcOEt), thereby obtaining the target product (198 mg).

### Step 3: Production of Compound SIM-306

Compound SIM-305 (198 mg) and MMAE (161 mg) were dissolved in DMF (10 ml). HOBt (31.1 mg) and pyridine (468 µL) were added, and the mixture was stirred at room temperature overnight. The reaction mixture was concentrated and purified with a flash automatic purification apparatus (H₂O/MeOH, Sfar Bio C18), thereby obtaining the target product (174 mg).

### Step 4: Production of Compound SIM-307 (TFA Salt)

Compound SIM-306 (35 mg) was added to 20% piperidine (6719 µL)/MeCN, and the mixture was stirred at room temperature for 12 hours. The reaction mixture was concentrated and purified with a flash automatic purification apparatus (H₂O/MeOH (0.1% TFA), Sfar Bio C18), thereby obtaining a TFA salt (38.4 mg), which is the target product.

### Step 5: Production of Compound SIM-308

DMTMM (7.30 mg) and DIPEA (4.59 µL) were added to a solution of the compound of Example 2 (7.89 mg) and the SIM-307 TFA salt (23 mg) in DMF (1 mL), and the mixture was stirred at room temperature for 10 minutes. DIPEA (8 µL) was added, and the mixture was further stirred for 10 minutes. The reaction mixture was concentrated and purified with a flash automatic purification apparatus (H₂O/MeOH, Sfar Bio C18), thereby obtaining the target product (20.9 mg).

### Step 6: Production of Compound of Example 11

Compound SIM-308 (20.9 mg) was added to a solution of TFA (1398 µL) in DCM (5.6 mL), and the mixture was stirred at 0°C for 4 hours. A saturated NaHCO₃ aqueous solution (3 mL) was added to the reaction mixture, and the reaction mixture was then concentrated. The residue was purified with a flash automatic purification apparatus (H₂O/MeOH (0.1% TFA), Sfar Bio C18), thereby obtaining the target product (12.7 mg).

### Example 32

A mixture of the compound of Example 31 (6.18 mg), DIPEA (10.6 µL), DMTMM (5.03 mg), and DMF (400 µL) was added to a solution of a TFA salt of compound SIM-105 (15 mg) in DMF (100 µL), and the mixture was stirred at room temperature for 10 minutes. Further, a mixture of the compound of Example 31 (6.18 mg), DIPEA (10.6 µL), DMTMM (5.03 mg), and DMF (400 µL) was separately prepared and added to the reaction mixture. Potassium chloride (9.04 mg) was added, and the mixture was stirred for 1 hour. The reaction mixture was diluted with a 0.1% TFA aqueous solution and DMSO, and purification was performed by high-performance liquid chromatography (H₂O/MeCN (0.1% TFA), YMC-Actus Triart C18, 250 × 20 mm I.D.), followed by freeze-drying, thereby obtaining the target product (3.5 mg).

### Example 34

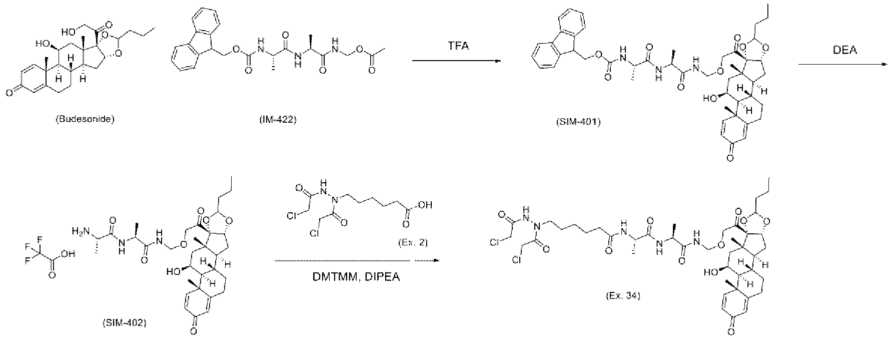

### Step 1: Production of Compound SIM-401

TFA (7.16 µL) was added to a suspension of budesonide (20 mg), IM-422 (37.9 mg), and DCM (400 µL), and the mixture was stirred overnight. The reaction mixture was diluted with DMSO, and purification was performed by high-performance liquid chromatography (H₂O/MeCN (0.1% TFA), YMC-Actus Triart C18, 250 × 20 mm I.D.), followed by freeze-drying, thereby obtaining the target product (8 mg).

### Step 2: Production of Compound SIM-402

DEA (100 µL) was added to a suspension of SIM-401 (8 mg) and THF (500 µL), and the mixture was stirred at room temperature for 3 hours. The reaction mixture was concentrated. The residue was purified by high-performance liquid chromatography (H₂O/MeCN (0.1% TFA), YMC-Actus Triart C18, 250 × 20 mm I.D.), followed by freeze-drying, thereby obtaining the target product (4.3 mg).

### Step 3: Production of Compound of Example 34

A 1 M DMSO solution (10.06 µL) of the compound of Example 2, a solution of DMTMM (2.320 mg) and DIPEA (4.87 µL) in DMF (100 µL) were added to a DMF solution (400 µL) of compound SIM-402 (4 mg), and the mixture was stirred at room temperature for 15 minutes. The reaction mixture was diluted with DMSO, and purification was performed by high-performance liquid chromatography (H₂O/MeCN (0.1% TFA), YMC-Actus Triart C18, 250 × 20 mm I.D.), followed by freeze-drying. Further, dilution was performed with hydrous DMSO, and purification was performed by high-performance liquid chromatography (H₂O/MeCN, YMC-Actus Triart C18, 250 × 20 mm I.D.), followed by freeze-drying, thereby obtaining the target product (2 mg).

Each of the compounds of Examples 5, 7 to 10, and 28 was produced as in Example 6, 9, or 11. Table 3 shows the structural formulas and physicochemical data of the compounds of Examples 5 to 11, 28, and 32.

**Table 3**

| Ex | Str | Data |
|---|---|---|
| 5 | 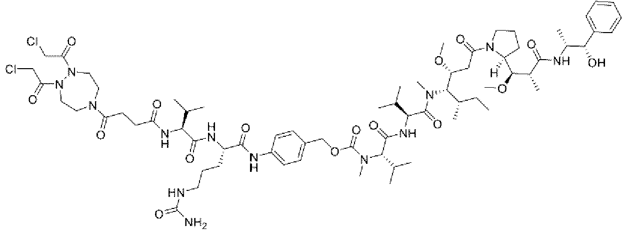 | MS m/z 1460.6 (M+1). |
| 6 | 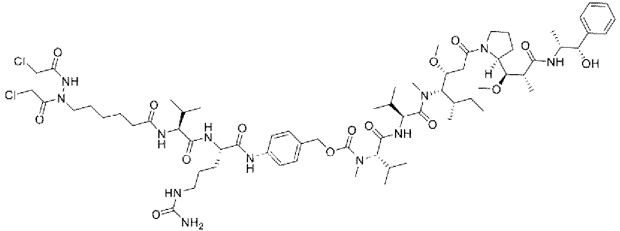 | MS m/z 1404.6 (M+1). |
| 7 | 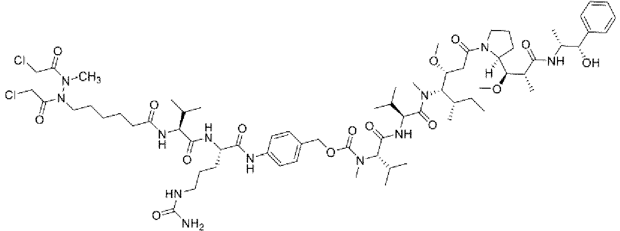 | MS m/z 1420.0 (M+1). |
| 8 | 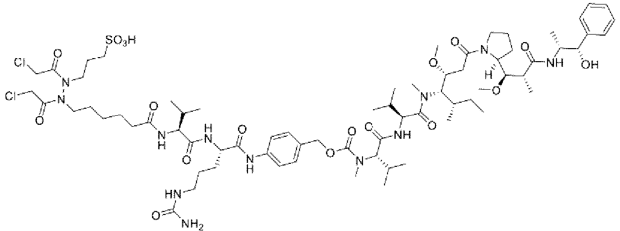 | MS m/z 1527.5 (M+1). |
| 9 | 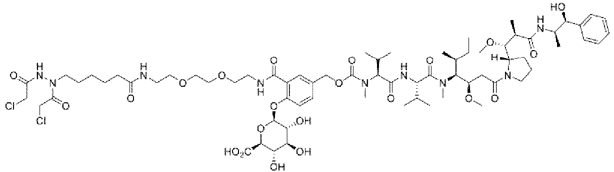 | MS m/z 1500.1 (M+1). |
| 10 | 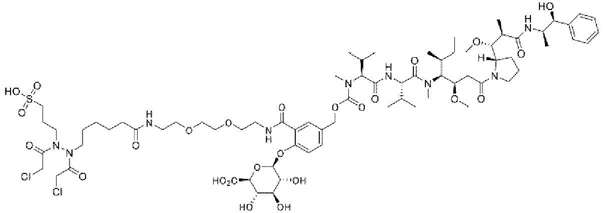 | MS m/z 1621.9 (M+1). |
| 11 | 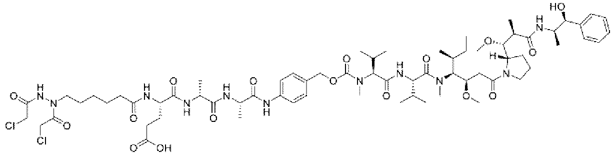 | MS m/z 1421.5 (M+1). |
| 28 | 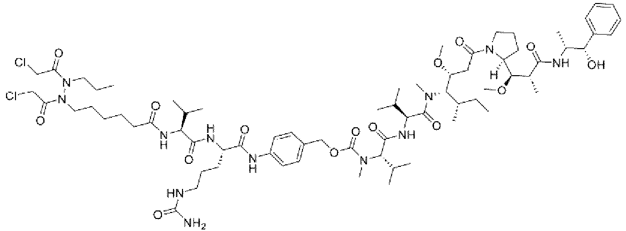 | MS m/z 1445.9 (M+1). |
| 32 | 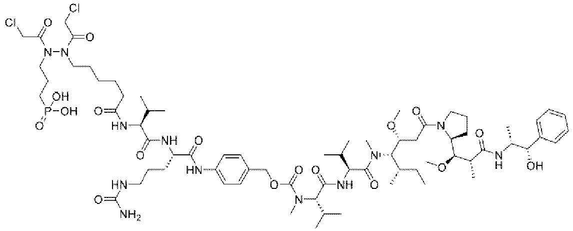 | MS m/z 1526.7 (M+1). |
| 34 | 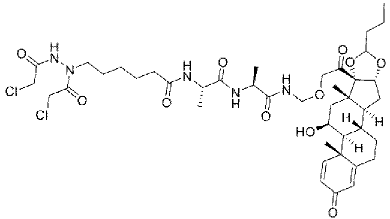 | MS m/z 904.4 (M+Na) |

### Example 12 to 26, 29, 33, and 35 to 37

### Example 12

A PBS solution (44.3 µL), a 0.5 M EDTA buffer solution (pH 8.0) (2.83 µL) and a 10 mM TCEP aqueous solution (2.024 µL) were added to an aqueous solution (15.69 µL) of transtuzumab (0.300 mg), and the mixture was shaken at 37̊C for 4 hours.

A borate buffer solution (0.1 M, pH 8.70 (315 µL) and a solution of the compound (1.619 µL) of Example 5 in DMSO were then added to the reaction mixture, and the mixture was shaken at 20̊C for 1 day.

A MESNa aqueous solution (1.214 µL) was added to the reaction mixture, and the mixture was shaken at 37°C for 2 hours.

The reaction mixture was loaded onto a NAP-5 column (Cytiva) equilibrated with PBS, and elution was performed with PBS according to the manual. The target fraction was subjected to centrifugal concentration (Model 3740, Kubota) using Amicon Ultra 0.5 mL, 50 kDa (Merck). The absorbance of the concentrated solution was measured at 280 nm (NanoDrop One, ThermoFisher), and the concentration was calculated from the molar extinction coefficient of the antibody and multiplied by the volume of the solution to give a yield of 1.943 mg/mL × 100 µL = 194 µg. In the calculation, the extinction coefficient of Example 5 at 280 nm was not taken into account because it was extremely small compared to that of the antibody.

The concentrated solution was subjected to HIC analysis. Since peaks corresponding to DAR3, 4, and 5 were observed, a weighted average value was calculated from their proportions to obtain an average DAR value of 4.06. In the calculation, the extinction coefficient of Example 5 at 280 nm was not taken into account because it was extremely small compared to that of the antibody. The measurement conditions were as follows.

| | |
|---|---|
| · System: | Acquity UPLC H-Class Bio (Waters) |
| · Column: | Protein-Pak Hi Res HIC, 2.5 *µ* m, 2.1 mm x 100 mm |
| · Temperature: | 30 °C |
| · Flow rate: | 0.3 mL/min |
| · Mobile phase A: | 1.5 M (NH₄)₂SO₄, 25 mM Phophate buffer pH 7.0 |
| · Mobile phase B: | 25 mM Phophate buffer pH 7.0 / 2-propanol (8/2) |
| · Gradient: | 20% B to 99% B over 15 min, 99% B for 0.5 min, 99% B to 20% B over 0.1 min, then 20% B for 6.4 min |

### Example 24

A PBS solution (44.4 µL), an EDTA buffer solution (pH 8.0) (2.4 µL) and a 10 mM TCEP aqueous solution (2.024 µL) were added to an aqueous solution (15.58 µL) of trastuzumab (0.300 mg), and the mixture was shaken at 25°C for 3 hours.

A borate buffer solution (0.1 M, pH 8.7) (315 uL), a 1 M NaI aqueous solution (48.6 µL) and a solution of the compound of Example 9 (3.24 µL) in DMSO were then added to the reaction mixture, and the mixture was shaken at 20°C for 1.5 days.

A MESNa aqueous solution (1.214 µL) was added to the reaction mixture, and the mixture was shaken at 37°C for 1 hour.

The reaction mixture was loaded onto a NAP-5 column (Cytiva) equilibrated with PBS, and elution was performed with PBS according to the manual. The target fraction was subjected to centrifugal concentration (Model 3740, Kubota) using Amicon Ultra 0.5 mL, 50 kDa (Merck). The absorbance of the concentrated solution was measured at 280 nm (NanoDrop One, ThermoFisher), and the concentration was calculated from the molar extinction coefficient of the antibody and multiplied by the volume of the solution to give a yield of 1.684 mg/mL × 150 µL = 252 µg. In the calculation, the extinction coefficient of Example 9 at 280 nm was not taken into account because it was extremely small compared to that of the antibody.

The concentrated solution was subjected to HIC analysis under the same conditions as in Example 12.

### Example 25

A 10 mM TCEP aqueous solution (2.024 µL) and a 0.5 M EDTA buffer solution (pH 8.0) (2.83 µL) were added to an aqueous solution (59.4 µL) of trastuzumab (0.300 mg) in PBS, and the mixture was shaken at 37°C for 3 hours.

A borate buffer solution (0.1 M, pH 8.7) (315 uL), a 1 M NaI aqueous solution (2.429 µL), and a 10 mM solution of the compound of Example 10 (3.24 µL) in DMSO were then added to the reaction mixture, and the mixture was shaken at 20°C for 3 days.

A MESNa aqueous solution (1.214 µL) was added to the reaction mixture, and the mixture was shaken at 37°C for 1 hour.

The reaction mixture was loaded onto HiPrep Desalting 26/10 (Cytiva) equilibrated with PBS, and elution was performed with AKTA Explorer S10 (Cytiva) using PBS as a mobile phase. The target fraction was subjected to centrifugal concentration (Model 3740, Kubota) using Amicon Ultra 4 mL, 50 kDa (Merck). The absorbance of the concentrated solution was measured at 280 nm (NanoDrop One, ThermoFisher), and the concentration was calculated from the molar extinction coefficient of the antibody and multiplied by the volume of the solution to give a yield of 1.04 mg/mL × 180 µL = 187 µg. In the calculation, the extinction coefficient of Example 10 at 280 nm was not taken into account because it was extremely small compared to that of the antibody.

The concentrated solution was subjected to HIC analysis under the same conditions as in Example 12.

SEC/MS analysis was performed using the same solution as in the HIC analysis. The MS corresponding to DAR4 was observed as the major component. The measurement conditions were as follows.

### LC conditions

| | |
|---|---|
| · System: | ACQUITY UPLC H-Class Bio |
| · Column: | ACQUITY UPLC Protein BEH SEC (1.7 µm, 2.1 × 150 mm) |
| · Temp.: | 25 °C |
| · Solvent A: | 5% IPA / 100 mM NH₄OAc aq. |
| · Gradient: | Isocratic 100% A in 10 min |
| · Flow Rate: | 0.065 mL/min |
| · Inj.: | 8 µL (1.04 mg/mL sample) |
| · UV: | 280 nm |

### MS conditions

| | |
|---|---|
| · System: | Waters Xevo G2-XS QTof |
| · Ion Mode: | ESI⁺ (TOF-MS) |
| · MS: | 500-8000 |
| · Capillary: | 3 kV |
| · Sampling cone: | 200 V |
| · Source offset: | 150 V |
| · Source temp.: | 150 °C |
| · Desolvation temp.: | 500 °C |
| · Cone gas flow: | 0 L/hr |
| · Desolvation gas flow: | 800 L/hr |
| · Calibration: | Nal in 50% IPA aq. (m/z = 400 to 7000 amu) |
| · Lockmass: | Leucine-Enkephalin |

### Example 29

A 10 mM TCEP aqueous solution (2.024 µL) and an EDTA buffer solution (pH 8.0) (2.48 µL) were added to a solution of trastuzumab in PBS (300 µg, 59.1 µL), and the mixture was shaken at 37°C for 1.5 hours.

A borate buffer solution (0.1 M, pH 8.7) (315 µL) and a 10 mM solution of the compound of Example 28 (3.24 µL) in DMSO were then added to the reaction mixture, and the mixture was shaken at 20°C for 44 hours.

A 20 mM MESNa aqueous solution (2.429 µL) was added to the reaction mixture, and the mixture was shaken at 37°C for 1 hour.

The reaction mixture was loaded onto a NAP-5 column (Cytiva) equilibrated with PBS, and elution was performed with PBS. The target fraction was subjected to centrifugal concentration (Model 3740, Kubota) using Amicon Ultra 0.5 mL, 50 kDa (Merck). The absorbance of the solution was measured at 280 nm (NanoDrop One, Thermo Scientific), and the concentration was calculated from the molar extinction coefficient of the antibody and multiplied by the volume of the solution to give a yield of 2.630 mg/mL × 100 µL = 263 µg. In the calculation, the extinction coefficient of Example 28 at 280 nm was not taken into account because it was extremely small compared to that of the antibody.

The concentrated solution was subjected to HIC analysis under the same conditions as in Example 12. Since peaks corresponding to DAR2, 3, 4, and 5 were observed, a weighted average value was calculated from their proportions to obtain an average DAR value of 3.94. In the calculation, the extinction coefficient of Example 28 at 280 nm was not taken into account because it was extremely small compared to that of the antibody. The measurement conditions were as follows.

| | |
|---|---|
| · System: | Acquity UPLC H-Class Bio (Waters) |
| · Column: | Protein-Pak Hi Res HIC, 2.5 µm, 2.1 mm x 100 mm |
| · Temperature: | 30 °C |
| · Flow rate: | 0.3 mL/min |
| · Mobile phase A: | 1.5 M (NH4)2SO4, 25 mM Phophate buffer pH 7.0 |
| · Mobile phase B: | 25 mM Phophate buffer pH 7.0 / 2-propanol (8/2) |
| · Gradient: | 20% B to 99% B over 15 min, 99% B for 0.5 min, 99% to 20% B over 0.1 min, then 20% B for 6.4 min |

SEC/MS analysis was performed using the same sample solution as in the HIC analysis. The MS corresponding to DAR4 was observed as the major component. The measurement conditions were as follows.

### LC conditions

| | |
|---|---|
| System: | Vanquish Flex |
| Column: | XBridge Premier SEC (Particle size; 2.5 um, Pore size; 25 nm) (4.6 mm ID x 15 cm) + Guard column |
| Temp.: | 25 °C |
| Solvent A: | 5% IPA / 100 mM NH₄OAc aq. |
| Gradient: | Isocratic 100% A for 35 min |
| Flow Rate: | 0.1 mL/min |

| MS conditions | |
|---|---|
| · System: | Orbitrap Exploris 240 |
| · Ion Mode: | ESI+ |
| · MS: | 500-8000 |
| · Capillary: | 3 kV |
| · Sampling cone: | 200 V |
| · Source offset: | 150 V |
| · Source temp.: | 150 °C |
| · Desolvation temp.: | 500 °C |
| · Cone gas flow: | 0 L/Hr |
| · Desolvation gas flow: | 800 L/Hr |
| · Calibration: | AHFP |

### Example 33

A 10 mM TCEP aqueous solution (2.024 µL) and an EDTA buffer solution (pH 8.0) (2.48 µL) were added to a solution of trastuzumab in PBS (300 µg, 59.1 µL), and the mixture was shaken at 37°C for 1.5 hours.

A borate buffer solution (0.1 M, pH 8.7) (315 µL) and a 10 mM solution of the compound of Example 32 (3.24 µL) in DMSO were then added to the reaction mixture, and the mixture was shaken at 20°C for 45 hours.

A 20 mM MESNa aqueous solution (2.429 µL) was added to the reaction mixture, and the mixture was shaken at 37°C for 1 hour.

The reaction mixture was loaded onto a NAP-5 column (Cytiva) equilibrated with PBS, and elution was performed with PBS. The target fraction was subjected to centrifugal concentration (Model 3740, Kubota) using Amicon Ultra 0.5 mL, 50 kDa (Merck). The absorbance of the solution was measured at 280 nm (NanoDrop One, Thermo Scientific), and the concentration was calculated from the molar extinction coefficient of the antibody and multiplied by the volume of the solution to give a yield of 2.493 mg/mL × 100 µL = 249 µg. In the calculation, the extinction coefficient of Example 32 at 280 nm was not taken into account because it was extremely small compared to that of the antibody.

The concentrated solution was subjected to HIC analysis under the same conditions as in Example 12. Since peaks corresponding to DAR2, 3, 4, and 5 were observed, a weighted average value was calculated from their proportions to obtain an average DAR value of 3.89. In the calculation, the extinction coefficient of Example 32 at 280 nm was not taken into account because it was extremely small compared to that of the antibody.

### Example 35

A 10 mM TCEP aqueous solution (2.024 µL) and an EDTA buffer solution (pH 8.0) (2.8 µL) were added to a solution of trastuzumab in PBS (300 µg, 59.1 µL), and the mixture was shaken at 25°C for 2 hours.

A borate buffer solution (0.1 M, pH 8.7) (315 µL) and a solution of the compound of Example 34 (3.24 µL) in DMSO were then added to the reaction mixture, and the mixture was shaken at 20°C for 19 hours.

A MESNa aqueous solution (5.06 µL) was added to the reaction mixture, and the mixture was shaken at 37°C for 1.5 hours.

The reaction mixture was loaded onto HiPrep Desalting 26/10 (Cytiva) equilibrated with PBS, and elution was performed with AKTA Explorer S10 (Cytiva) using PBS as a mobile phase. The target fraction was subjected to centrifugal concentration (Model 3740, Kubota) using Amicon Ultra 4 mL, 50 kDa (Merck). The absorbance of the solution was measured at 280 nm (NanoDrop One, Thermo Scientific), and the concentration was calculated from the molar extinction coefficient of the antibody and multiplied by the volume of the solution to give a yield of 1.27 mg/mL × 160 µL = 203 µg. In the calculation, the extinction coefficient of Example 34 at 280 nm was not taken into account because it was extremely small compared to that of the antibody.

The concentrated solution was subjected to HIC analysis under the same conditions as in Example 12. Since peaks corresponding to DAR3, 4, and 5 were observed, a weighted average value was calculated from their proportions to obtain an average DAR value of 3.89. In the calculation, the extinction coefficient of Example 34 at 280 nm was not taken into account because it was extremely small compared to that of the antibody. The measurement conditions were as follows.

| | |
|---|---|
| · System: | Acquity UPLC H-Class Bio (Waters) |
| · Column: | Protein-Pak Hi Res HIC, 2.5 um, 2.1 mm x 100 mm |
| · Temperature: | 30 °C |
| · Flow rate: | 0.3 mL/min |
| · Mobile phase A: | 1.5 M (NH4)2SO4, 25 mM Phophate buffer pH 7.0 |
| · Mobile phase B: | 25 mM Phophate buffer pH 7.0 / 2-propanol (8/2) |
| · Gradient: | 20% B to 99% B over 15 min, 99% B for 0.5 min, 99% to 20% B over 0.1 min, then 20% B for 6.4 min |

SEC/MS analysis was performed using the same solution as in the HIC analysis. The MS corresponding to DAR4 was observed as the major component. The measurement conditions were as follows.

### LC conditions

| | |
|---|---|
| System: | Vanquish Flex |
| Column: | XBridge Premier SEC (Particle size; 2.5 um, Pore size; 25 nm) (4.6 mm ID x 15 cm) + Guard column |
| Temp.: | 25 °C |
| Solvent A: | 5% IPA / 100 mM NH₄OAc aq. |
| Gradient: | Isocratic 100% A for 35 min |
| Flow Rate: | 0.1 mL/min |

| MS conditions | |
|---|---|
| · System: | Orbitrap Exploris 240 |
| ▪ Ion Mode: | ESI+ |
| ▪ MS: | 500-8000 |
| ▪ Capillary: | 3 kV |
| ▪ Sampling cone: | 200 V |
| ▪ Source offset: | 150 V |
| ▪ Source temp.: | 150 °C |
| ▪ Desolvation temp.: | 500 °C |
| ▪ Cone gas flow: | 0 L/Hr |
| ▪ Desolvation gas flow: | 800 L/Hr |
| ▪ Calibration: | AHFP |

### Example 37

A solution of nivolumab in PBS (1.044 mg, 145 µL) (BioXcell InVivoSIM anti-human PD-1 (Nivolumab Biosimilar) SIM0003), a 0.5 M EDTA buffer solution (pH 8.0) (9.5 µL), and a 10 mM TCEP aqueous solution (1.5 µL) were added to a PBS solution (65 µL), and the mixture was rotated at 37°C for 2 hours.

A borate buffer solution (0.1 M, pH 8.7) (857.5 µL) and a solution of the compound of Example 6 in DMSO (11.5 µL, 0.115 µmol) were then added to the reaction mixture, and the mixture was rotated at 20°C for 73 hours.

A PBS solution (1.5 mL) was added to the reaction mixture, and the mixture was subjected to centrifugal concentration (Model 3740, Kubota) using Amicon Ultra 4 mL, 30 kDa (Merck). The concentrated solution (about 0.2 mL) was gel-filtration-purified with an AKTA explorer using a HiPrep 26/10 Desalting (Cytiva) column and PBS as an eluent. The target fraction was subjected to centrifugal concentration using Vivaspin Turbo 15, 50 kDa (Sartorius). After concentration, the sample was adjusted to a solution amount of 530 µL with PBS. The absorbance of the solution was measured at 280 nm (NanoDrop One, Thermo Scientific), and the concentration was calculated from the molar extinction coefficient of the antibody (1.53) and multiplied by the volume of the solution to give a yield of 1.77 mg/mL /1.53 × 530 µL = 0.53/1.54 = 609 µg. In the calculation, the extinction coefficient of Example 6 at 280 nm was not taken into account because it was extremely small compared to that of the antibody.

The concentrated solution was subjected to HIC analysis. Since peaks corresponding to DAR3, 4, and 5 were observed, a weighted average value was calculated from their proportions to obtain an average DAR value of 3.93. In the calculation, the extinction coefficient of Example 6 at 280 nm was not taken into account because it was extremely small compared to that of the antibody. The measurement conditions are as follows.

| | |
|---|---|
| · System: | Acquity UPLC H-Class Bio (Waters) |
| · Column: | Protein-Pak Hi Res HIC, 2.5 um, 2.1 mm x 100 mm |
| · Temperature: | 30 degC |
| · Flow rate: | 0.3 mL/min |
| · Mobile phase A: | 1.5 M (NH₄)₂SO₄, 25 mM Phophate buffer pH 7.0 |
| · Mobile phase B: | 25 mM Phophate buffer pH 7.0 / 2-propanol (8/2) |
| · Gradient: | 20% B to 99% B over 15 min, 99% B for 0.5 min, 99% to 20% B over 0.1 min, then 20% B for 6.4 min |

Each of the compounds of Examples 13 to 23 and 26 was produced as in Example 12, 24, or 25. The structures of Comparative Examples (CEx) 1 and 2 are shown in Table 8. Table 4 show the antibodies and drug-linkers used in the reactions in Examples 12 to 26, 29, 33, and 35 to 37, the reaction time, the amount of NaI added (the number of equivalents per equivalent of antibody), the average drug-to-antibody ratio (DAR), and the DAR4 percentage.

**Table 4**

| | Antibody | Drug-linker | Reaction time | Nal (eq) | DAR Ave | DAR4 percentage (%) |
|---|---|---|---|---|---|---|
| Ex 12 | Trastuzumab | Ex 5 | 1d | 0 | 4.06 | 86.3 |
| Ex 13 | BB4 | Ex 5 | 1d | 0 | 4.04 | 85.2 |
| Ex 14 | VIS832 | Ex 5 | 1d | 0 | 4.01 | 85.5 |
| Ex 15 | Trastuzumab | Ex 6 | 1d | 0 | 4.15 | 78.6 |
| Ex 16 | BB4 | Ex 6 | 2d | 0 | 4.11 | 80.3 |
| Ex 17 | VIS832 | Ex 6 | 2d | 0 | 4.08 | 77.1 |
| Ex 18 | Trastuzumab | Ex 7 | 0.5 d | 0 | 4.17 | 77.2 |
| Ex 19 | BB4 | Ex 7 | 0.5 d | 0 | 4.14 | 77.2 |
| Ex 20 | VIS832 | Ex 7 | 0.5 d | 0 | 4.12 | 77.0 |
| Ex 21 | Trastuzumab | Ex 8 | 1d | 0 | 4.09 | 84.3 |
| Ex 22 | BB4 | Ex 8 | 1d | 0 | 4.06 | 82.2 |
| Ex 23 | VIS832 | Ex 8 | 1d | 0 | 4.03 | 80.9 |
| Ex 24 | Trastuzumab | Ex 9 | 1.5 d | 24000 | 4.05 | 69.0 |
| Ex 25 | Trastuzumab | Ex 10 | 3d | 1200 | 3.87 | 69.8 |
| Ex 26 | Trastuzumab | Ex 11 | 1.5 d | 24000 | 4.12 | 68.8 |
| Ex 29 | Trastuzumab | Ex 28 | 2d | 0 | 3.94 | 76.1 |
| Ex 33 | Trastuzumab | Ex 32 | 2d | 0 | 3.89 | 74.8 |
| Ex 35 | Trastuzumab | Ex 34 | 2d | 0 | 3.89 | 70.6 |
| Ex 36 | Trastuzumab | Ex 34 | 2d | 12000 | 3.98 | 70.0 |
| Ex 37 | Nivolumab | Ex 34 | 2d | 0 | 3.93 | 70.1 |
| CEx 1 | VIS832 | Mal-vcMMAE | 1h | 0 | 4.30 | 33.0 |
| CEx 2 | VIS832 | DBM-vcMMAE* | 1h | 0 | 4.33 | 56.1 |

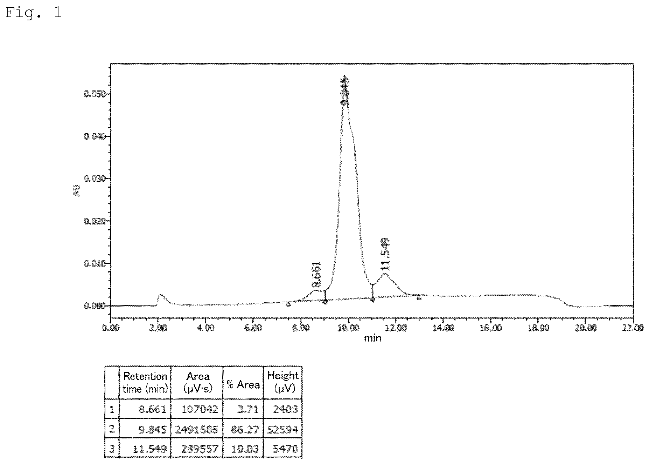

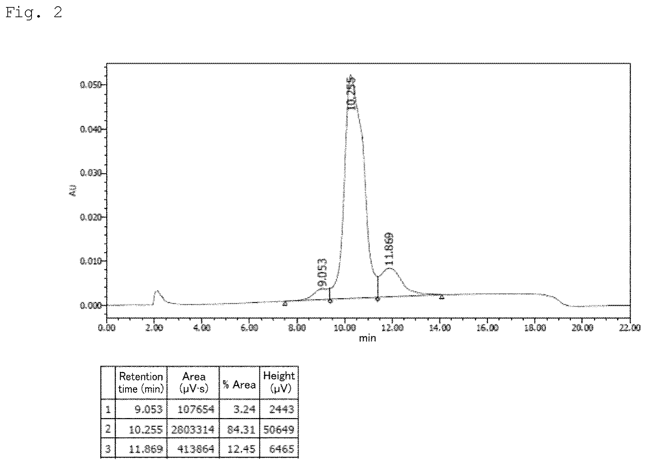

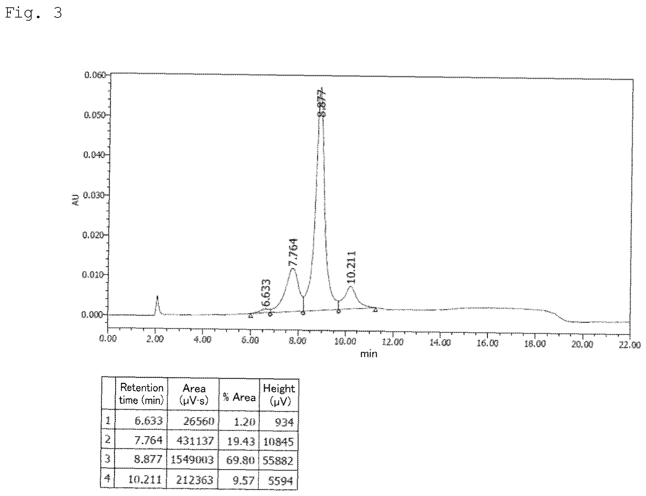

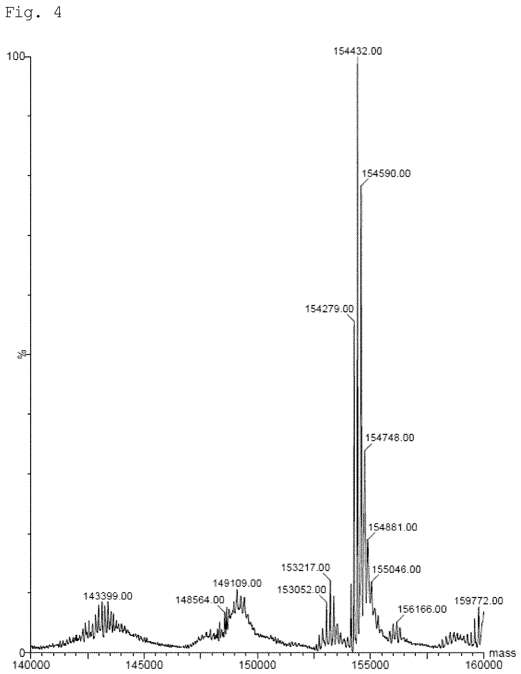

Fig. 1, 2, and 3 show the HIC charts of Examples 12, 21, and 25, respectively. Fig. 4 shows the mass spectrum chart of Example 25.

Table 5 shows the proportions of the drug-to-antibody ratios (DAR) ranging from 2 to 5 when reactions were performed by using the antibody and drug-linker of Example 15, and changing the amount of NaI added (the number of equivalents per equivalent of antibody) and the reaction time.

**Table 5**

| Nal (eq) | Reaction time (day) | DAR2 | DAR3 | DAR4 | DAR5 |
|---|---|---|---|---|---|
| 0 | 1.5 | 0 | 6.14 | 78.1 | 15.8 |
| 12 | 1.5 | 0 | 5.56 | 78.3 | 16.1 |
| 120 | 1.5 | 0 | 5.42 | 78.6 | 16 |
| 12600 | 0.5 | 0 | 5.6 | 77.4 | 17 |
| 40000 | 0.5 | 0 | 3.42 | 70.1 | 26.5 |

Table 6 shows the proportions of the drug-to-antibody ratios ranging from 3 to 5 when reactions were performed by using the antibody and drug-linker of Example 24, and changing the amount of NaI added (the number of equivalents per equivalent of antibody) and the reaction time.

**Table 6**

| Nal (eq) | Time | DAR3 | DAR4 | DAR5 |
|---|---|---|---|---|
| 120 | 2 days | 23.8 | 72.5 | 3.68 |
| | 3 days | 14.3 | 80 | 5.68 |
| | 4 days | 9.42 | 83.4 | 7.21 |
| | 7 days | 5.71 | 84.7 | 9.64 |
| 1200 | 2 days | 22.3 | 71.7 | 5.96 |
| | 3 days | 10.2 | 80.2 | 9.64 |
| | 4 days | 7.29 | 80.5 | 12.2 |
| 12000 | 1 day | 11.2 | 76.8 | 12 |
| | 2 days | 5.26 | 74.1 | 20.7 |
| | 3 days | 5.05 | 72.9 | 22.1 |

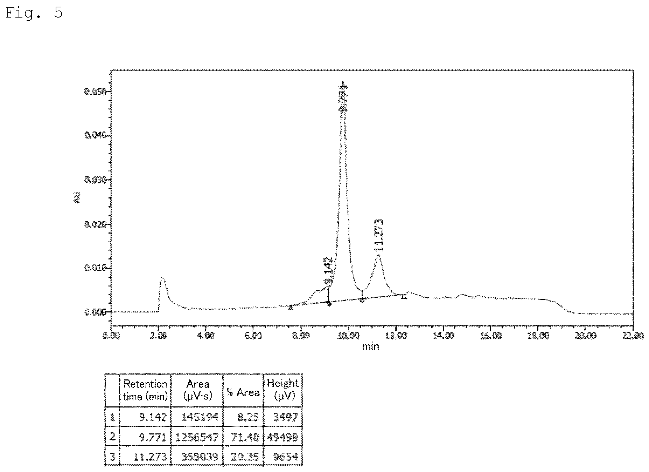

Table 7 shows the proportions of the drug-to-antibody ratios ranging from 3 to 5 when reactions were performed by using the antibody and drug-linker of Example 26, and changing the amount of NaI added (the number of equivalents per equivalent of antibody) and the reaction time. Fig. 5 shows an HIC chart after 2 days when 12000 equivalents of NaI were added.

**Table 7**

| Nal (eq) | Time | DAR3 | DAR4 | DAR5 |
|---|---|---|---|---|
| 120 | 2 days | 29.6 | 61.6 | 6.28 |
| | 3 days | 21.5 | 68.3 | 10.2 |
| | 4 days | 16.1 | 74.6 | 9.29 |
| | 7 days | 11.9 | 77.6 | 10.6 |
| 1200 | 2 days | 28.7 | 62 | 7.37 |
| | 3 days | 14.8 | 74.9 | 10.3 |
| | 4 days | 12.9 | 75.4 | 11.7 |
| 12000 | 1 day | 24.8 | 64.4 | 9.09 |
| | 2 days | 8.25 | 71.4 | 20.4 |
| | 3 days | 7.41 | 69.4 | 23.2 |

### Test Examples

### Test Example 1: Evaluation of Stability in Plasma

### Preparation of Reagents

### - PBS-T (PBS + 0.05% Tween-20)

250 µL of Polysorbate 20 (Cat No. 103168) was added to 500 mL of PBS.

### - Biotinylated Peptide Solution (Biotin-GGGGENTAVVAVEPDRRNQ)

A biotinylated peptide was dissolved in water/acetonitrile (7:3, v/v) to 0.5 mg/mL.

### - 0.1 M Glycine

3.75 mg of glycine was weighed and dissolved in purified water to make 0.5 L.

### - 0.1 M Glycine-HCl (pH 3.0)

1020 µL of 1M HCl was taken and made up to 50 mL with 0.1 M glycine.

### Operation Method

(1) After vortexing magnetic beads (10 mg/mL) in a container for 30 seconds or more, 0.05 mL was collected into a 1.5 mL low protein binding tube.
(2) 1 mL of PBS-T was added, followed by mixing. The following procedure was repeated twice for washing.
   - After a reaction with a magnet for 2 minutes, the supernatant was removed.
   - After removal from the magnet, 1 mL of PBS-T was added, followed by mixing again.
(3) 1 mL of PBS-T and 5 µL of the biotinylated peptide solution (0.5 mg/mL) were added to the beads, followed by mixing. The tube was rotated at room temperature for 1 hour.
(4) The supernatant was removed, and the magnetic beads were washed twice with 1 mL of PBS-T according to procedure of (2).
(5) 10 µg of a test substance (ADC) and 1 mL of PBS-T were added to and mixed with 50 µL of a human or mouse plasma sample.
(6) The mixture obtained in (5) was added to and mixed with the magnetic beads, and the tube was rotated at room temperature for 1 hour.
(7) The supernatant was removed, and the magnetic beads were washed once with PBS-T (1 mL) and twice with PBS (1 mL) according to procedure of (2).
(8) 0.1 mL of 0.1 M Glycine-HCl (pH 3.0) was added, followed by mixing. The tube was shaken with a micro tube mixer (model: MT-400) at room temperature for 5 minutes.
(9) The supernatant was collected from (8) and added to a centrifugal ultrafiltration filter unit.
(10) Centrifugation (14000 g, 25°C, 5 minutes) was performed to remove the flow-through fraction.
(11) 400 µL of purified water was added to the sample obtained in (10).
(12) Centrifugation (14000 g, 25°C, 5 minutes) was performed to remove the flow-through fraction.
(13) 400 µL of purified water was added to the sample obtained in (12).
(14) Centrifugation (14000 g, 25°C, 5 minutes) was performed to remove the flow-through fraction.
(15) The sample obtained in (14) was transferred to a new tube, inverted, and centrifuged (1000 g, 25°C, 2 minutes).
(16) About 25 µL of an ADC extract was obtained from the plasma sample.

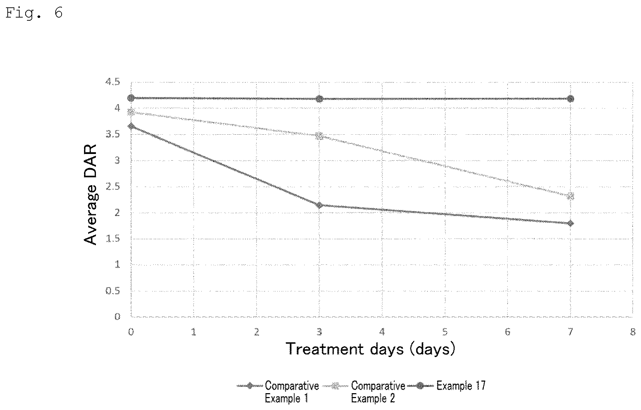

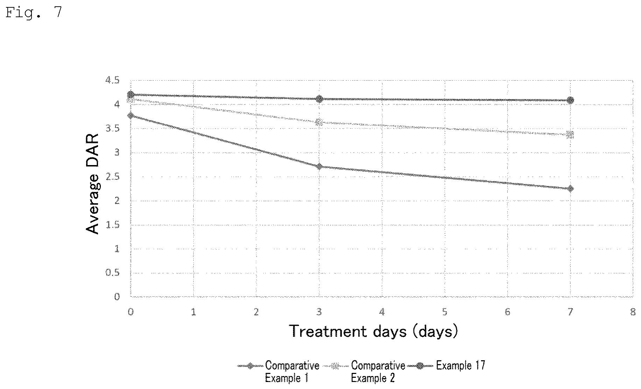

The ADC prepared in Example 17 and the ADCs shown in Comparative Examples 1 and 2 in Table 8 were used as test substances. The antibody used was VIS832. Fig. 6 shows the results for human plasma, and Fig. 7 shows the results for mouse plasma.

**Table 8**

| CEx | Str |
|---|---|
| 1 | 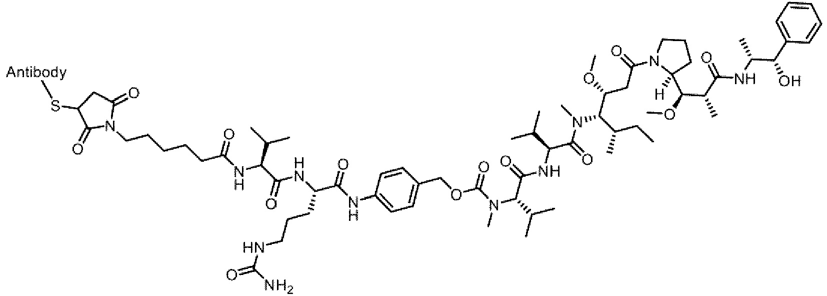 |
| 2 | 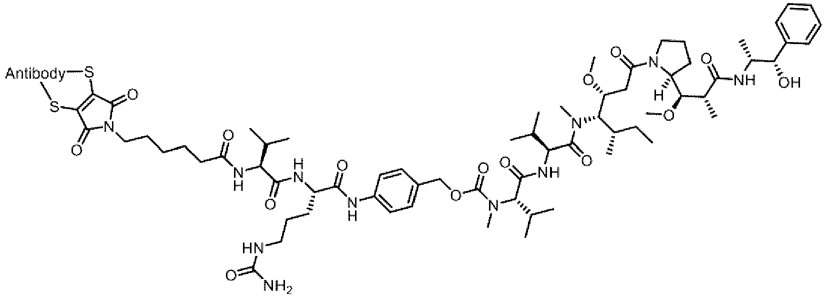 |

### Test Example 2: Structural Stability Evaluation

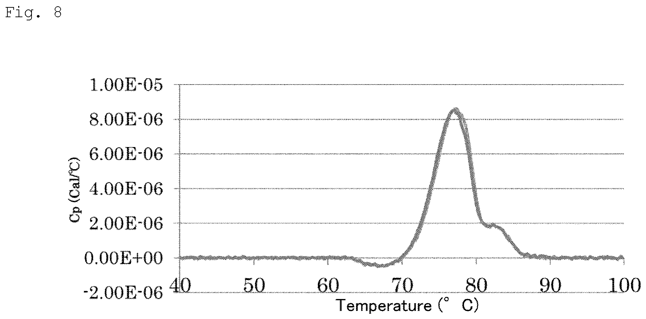

The structural stability of test substances was evaluated using differential scanning calorimetry (DSC) analysis. Table 9 and Fig. 8 show the results.

**Table 9**

| Test substance | Tm (°C) |
|---|---|
| VIS832 | 79.5 |
| Example 17 | 77.1 |
| Comparative Example 1 | 75.5 |
| Comparative Example 2 | 77.5 |

### Test Example 3: Proliferation Test

### ADC Proliferation Test (U266B1 Cells)

RPMI-1640 (10% FBS, 1% penicillin-streptomycin) was used as a medium, and cells were seeded in a microplate (Greiner 655098) at 2 × 10⁴ cells/well, 90 µL/well. 10 µL of each test substance solution was individually added, and serial dilution was performed at a common ratio of 10-fold, followed by culturing at 37°C. After 5 days, CellTiter-Glo 2.0 (Promega, G9242) was added, and luminescence was then measured using Infinite M1000 PRO (Tecan). Table 10 shows the results.

**Table 10**

| Test substance | IC₅₀ (nM) |
|---|---|
| Example 17 | 0.057 |
| Comparative Example 1 | 0.075 |
| Comparative Example 2 | 0.069 |

### Industrial Applicability

The compound of the present invention or a salt thereof is useful for linkers for antibody-drug conjugates and for antibody-drug conjugates.

## Claims

1. A compound represented by formula [ML-I]: 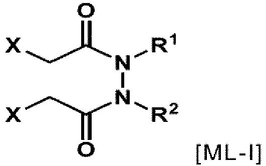 wherein
X is a leaving group;
R¹ and R² are each independently hydrogen, C₁₋₆ alkyl optionally substituted with one or more substituents, or C₁₋₆ alkylene-R¹¹ optionally substituted with one or more substituents,
with the proviso that R¹ and R² are not hydrogen at the same time; or
R¹ and R² together with nitrogen atoms adjacent thereto form a seven-membered ring that further contains a nitrogen atom as a ring-constituting atom, the ring further having, as a substituent, -C(=O)-C₁₋₆ alkylene-R¹¹ optionally substituted with one or more substituents; and
R¹¹ is COOH, SO₃H, PO₃H₂, or an ester group of COOH, SO₃H, or PO₃H₂; or
a salt thereof.

2. The compound or a salt thereof according to claim 1, which is
a compound represented by formula [ML-Ia] or formula [ML-Ib]: 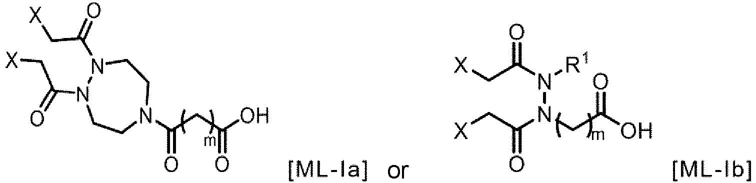 wherein
m is an integer of 1 to 6, and
X and R¹ are as defined above; or
a salt thereof.

3. The compound or a salt thereof according to claim 1, which is selected from the following compounds or salts thereof:

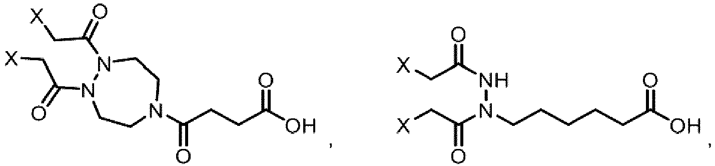

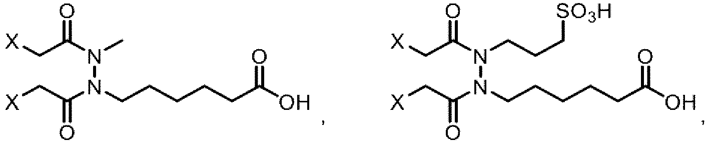

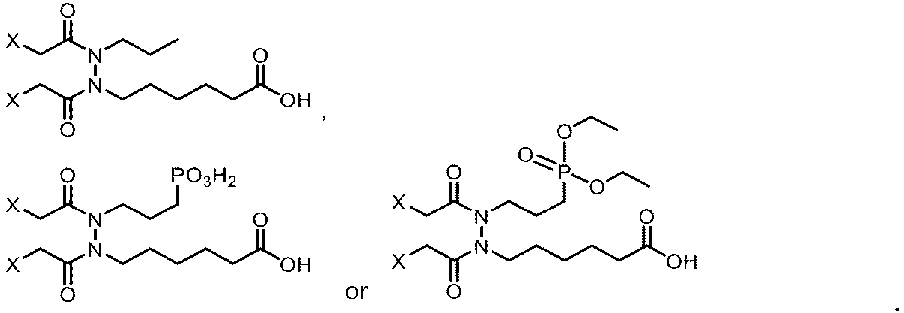

4. The compound or a salt thereof according to any one of claim 1 to 3, wherein X is halogen.

5. A drug-linker intermediate, which is a compound or a salt thereof, the compound being represented by formula [MCL-Iα] or formula [MCL-IIα]: 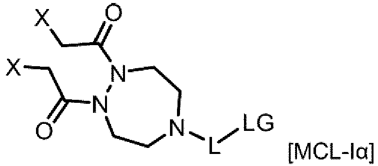 or 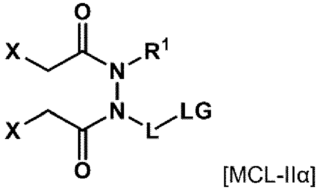 wherein
X is a leaving group;
R¹ is hydrogen, C₁₋₆ alkyl optionally substituted with one or more substituents, or C₁₋₆ alkylene-R¹¹ optionally substituted with one or more substituents;
R¹¹ is SO₃H, PO₃H₂, COOH, or an ester group of SO₃H, PO₃H₂, or COOH; L¹ is 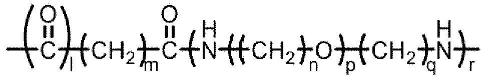 wherein each methylene is optionally substituted with one or more substituents;
L² is a bond or a cleavable linker; and
L³ is a bond, amino(aryl or heteroaryl)methyloxycarbonyl represented by 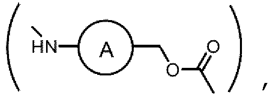
amino(aryl or heteroaryl)methyl represented by 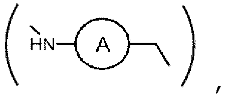
aminomethyl represented by 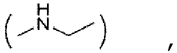
or oxymethyl represented by 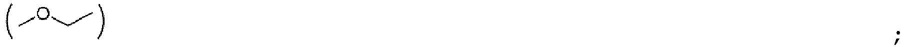 and
A is aryl or heteroaryl each optionally substituted with one or more substituents;
with the proviso that L² and L³ are not a bond at the same time; LG is a halogen or an O-electron-withdrawing group;
l and r are each independently 0 or 1; and
m, n, p, and q are each independently an integer of 1 to 6.

6. A drug-linker, which is a compound or a salt thereof, the compound being represented by formula [ADL-Iα] or formula [ADL-IIα]: 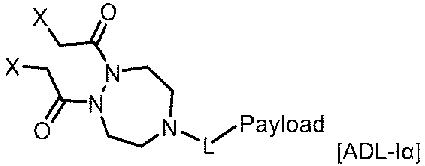 or 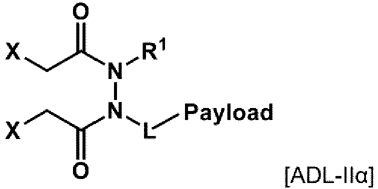 wherein Payload is a drug residue;
X is a leaving group;
R¹ is hydrogen, C₁₋₆ alkyl optionally substituted with one or more substituents, or C₁₋₆ alkylene-R¹¹ optionally substituted with one or more substituents;
R¹¹ is SO₃H, PO₃H₂, COOH, or an ester group of SO₃H, PO₃H₂, or COOH;
L¹ is 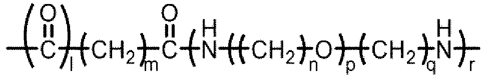 wherein each methylene is optionally substituted with one or more substituents;
L² is a bond or a cleavable linker;
L³ is a bond, amino(aryl or heteroaryl)methyloxycarbonyl represented by 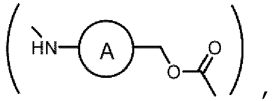
amino(aryl or heteroaryl)methyl represented by 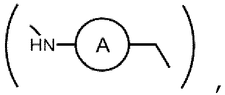
aminomethyl represented by 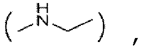
or oxymethyl represented by 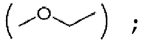 and
A is an aryl or heteroaryl optionally substituted with one or more substituents;
with the proviso that L² and L³ are not a bond at the same time;
l and r are each independently 0 or 1; and
m, n, p, and q are each independently an integer of 1 to 5.

7. An antibody-drug conjugate, which is a compound or a salt thereof, the compound being represented by formula [ADC-Iα] or formula [ADC-IIα] : 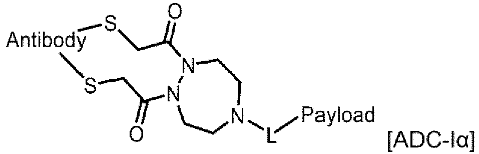 or 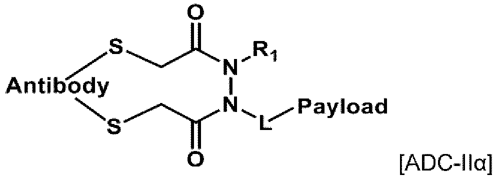 wherein
Payload is a drug residue;
Antibody is an antibody residue;
S is a sulfur atom of a cysteine residue in the antibody moiety;
R¹ is hydrogen, C₁₋₆ alkyl optionally substituted with one or more substituents, or C₁₋₆ alkylene-R¹¹ optionally substituted with one or more substituents;
R¹¹ is SO₃H, PO₃H₂, COOH, or an ester group of SO₃H, PO₃H₂, or COOH;
L¹ is 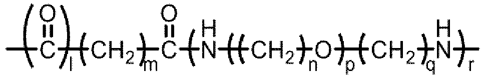 wherein each methylene is optionally substituted with one or more substituents;
L² is a bond or a cleavable linker;
L³ is a bond, amino(aryl or heteroaryl)methyloxycarbonyl represented by 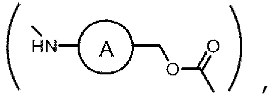
amino(aryl or heteroaryl)methyl represented by 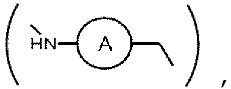
aminomethyl represented by 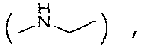 or
oxymethyl represented by 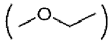 and;
A is aryl or heteroaryl each optionally substituted with one or more substituents;
with the proviso that L² and L³ are not a bond at the same time; l and r are each independently 0 or 1; and
m, n, p, and q are each independently an integer of 1 to 6.
